(19) Europäisches Patentamt
European Patent Office
Office européen des brevets

(11) EP 4 071 764 A2

(12) **EUROPEAN PATENT APPLICATION**

(43) Date of publication:
**12.10.2022 Bulletin 2022/41**

(21) Application number: **21214698.9**

(22) Date of filing: **15.12.2021**

(51) International Patent Classification (IPC):
***G16C 20/30*** *(2019.01)*

(52) Cooperative Patent Classification (CPC):
**G16C 20/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021 JP 2021052505**

(71) Applicant: **FUJITSU LIMITED**
**Kawasaki-shi, Kanagawa 211-8588 (JP)**

(72) Inventors:
- **Jippo, Hideyuki**
  **Kawasaki-shi, Kanagawa, 211-8588 (JP)**
- **Maruo, Akito**
  **Kawasaki-shi, Kanagawa, 211-8588 (JP)**
- **Uemura, Taiki**
  **Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

# (54) INFORMATION PROCESSING PROGRAM, INFORMATION PROCESSING APPARATUS, AND INFORMATION PROCESSING METHOD FOR DETERMINING PROPERTIES OF MOLECULES

(57) An information processing program in which an information processing apparatus analyzes a first molecule different from all of a plurality of molecules based on characteristic data of each of the plurality of molecules performs processing of specifying a structure descriptor that is an index based on each of structures of the plurality of molecules; and generating a model used to analyze the first molecule based on the structure descriptor and a similarity between each of the structures of the plurality of molecules.

FIG. 3

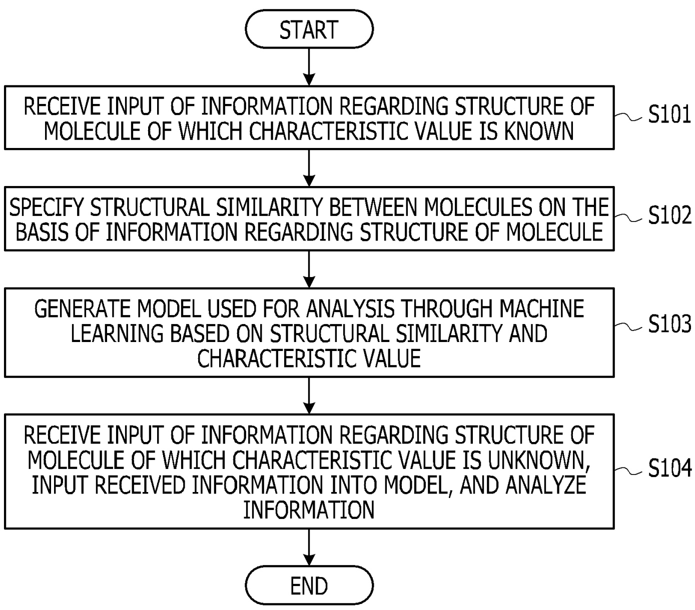


EP 4 071 764 A2

## Description

[Technical Field]

The embodiments discussed herein relate to an information processing program, an information processing apparatus, and an information processing method.

[Background Art]

Generally, compounds (molecules) having similar structures are expected to have similar characteristics (properties). This similar property principle that "similar compounds have similar properties" is widely used, for example, in a case where a compound having a predetermined property is designed by predicting the properties of compounds, or in a case where a compound having a predetermined property is searched for by screening a database of compounds.

When the similar property principle is used, for example, it can be predicted that, by utilizing an existing compound as a query compound, a compound with similarity (a compound having a structure similar to the structure of the query compound) retrieved from the database has the same function (characteristics and physical properties) as the query compound.

Therefore, for example, a technique has been studied for searching for and narrowing a molecule (molecule of which the characteristics are unknown) having a physical property close to a physical property of the molecule on the basis of a molecule of which a target characteristic (biological activity, physical/chemical physical property value or the like) is known. More specifically, for example, a technique has been studied that generates and uses a model that performs regression prediction of a physical property value (multiple regression model), a model that classifies molecules (class classifier), or the like by performing machine learning based on information regarding a molecule of which characteristics are known.

As the related art regarding such a technique, for example, a technique has been proposed that predicts a characteristic value of a material of which characteristics are unknown based on a structural similarity between a material of which characteristics are known and the material of which the characteristics are unknown.

However, with these related art, there has been a case where accuracy of analysis (prediction accuracy, classification accuracy, or the like) about a molecule of which characteristics are unknown is not sufficient (PTL 1).

[Citation List]

[Patent Literature]

[PTL 1] Japanese Laid-open Patent Publication No. 2020-194488.

[Summary]

[Technical Problem]

In one aspect, an object of this case is to provide an information processing program, an information processing apparatus, and an information processing method that can generate a model that can analyze a molecule, of which a characteristic value (characteristic data) of a predetermined characteristic is not specified, with high accuracy.

[Solution to Problem]

According to one embodiment, an information processing program in which an information processing apparatus analyzes a first molecule different from all of a plurality of molecules based on characteristic data of each of the plurality of molecules performs processing of specifying a structure descriptor that is an index based on each of structures of the plurality of molecules; and generating a model used to analyze the first molecule based on the structure descriptor and a similarity between each of the structures of the plurality of molecules.

[Advantageous Effects of Invention]

In one aspect, this case can provide an information processing program, an information processing apparatus, and an information processing method that can generate a model that can analyze a molecule, of which a characteristic value (characteristic data) of a predetermined characteristic is not specified, with high accuracy.

[Brief Description of Drawings]

FIG. 1 is a diagram illustrating an example of an ideal relationship between a target molecule of which a physical property value is known and a candidate molecule of which a physical property value is unknown when a model that performs regression prediction of the physical property value is generated and the physical property value of the molecule of which the physical property value is unknown is predicted through the regression prediction;
FIG. 2 is a diagram illustrating an example of an ideal relationship between the target molecule of which the physical property value is known and the candidate molecule of which the physical property value is unknown when a model that performs classification based on a physical property value is generated and molecules of which physical property values are unknown are classified;
FIG. 3 is a flowchart simply illustrating an example of a flow when a model used to analyze the molecule of which the characteristic value is unknown is generated and analyzed on the basis of a structural similarity between a molecule of which the characteristic value is known and a molecule of which a characteristic value is unknown;
FIG. 4 is a diagram illustrating an example of a relationship between a target molecule of which a physical property value is known and a candidate molecule of which a physical property value is unknown in a case where analysis is performed using the related art that performs analysis based on the structural similarity;
FIG. 5 is a diagram illustrating an example of a relationship between a target molecule of which a physical property value is known and a candidate molecule of which a physical property value is unknown in a case where analysis is performed using an example of the technology disclosed in this case for performing analysis on the basis of the structural similarity and a structure descriptor;
FIG. 6 is a diagram illustrating an example of a state of expressing acetic acid and methyl acetate as graphs;
FIG. 7 is a diagram illustrating an example of combinations in a case of combining the same elements in molecules A and B and making nodes of a conflict graph;
FIG. 8 is a diagram illustrating an example of a rule for creating an edge in the conflict graph;
FIG. 9 is a diagram illustrating an example of the conflict graph of the molecule A and the molecule B;
FIG. 10 is a diagram illustrating an example of a maximum independent set in a graph;
FIG. 11 is a diagram illustrating an example of a flow in a case of obtaining a maximum common substructure between the molecule A and the molecule B by obtaining the maximum independent set of the conflict graph (by solving maximum independent set problem);
FIG. 12 is an explanatory diagram for describing an example of a method for searching for a maximum independent set in a graph of which the number of nodes is six;
FIG. 13 is an explanatory diagram for describing an example of the method for searching for the maximum independent set in the graph of which the number of nodes is six;
FIG. 14 is a diagram illustrating an example of a maximum independent set in a conflict graph;
FIG. 15 is a diagram illustrating an example of expressing acetic acid and methyl acetate as graphs, on the basis of an atom type of general AMBER force field (GAFF);
FIG. 16 is a diagram illustrating an example of creating nodes of a conflict graph from the graphs of acetic acid and methyl acetate based on the GAFF atom type;
FIG. 17 is a diagram illustrating an example of a conflict graph created from the node illustrated in FIG. 16;
FIG. 18 is a diagram illustrating a hardware structure example of an information processing apparatus disclosed in this case;
FIG. 19 is a diagram illustrating another hardware structure example of the information processing apparatus disclosed in this case;
FIG. 20 is a diagram illustrating a functional structure example of the information processing apparatus disclosed in this case;
FIG. 21 is an example of a flowchart when a model used to analyze a non-specific molecule is generated in an example of the technology disclosed in this case;
FIG. 22 is another example of a flowchart when a model used to analyze a non-specific molecule is generated in an example of the technology disclosed in this case;
FIG. 23 is an example of a flowchart when the non-specific molecule is analyzed by using the generated model in an example of the technology disclosed in this case;
FIG. 24 is a diagram illustrating an example of a functional configuration of an annealing machine used for an annealing method;
FIG. 25 is a diagram illustrating an example of an operation flow of a transition control unit;
FIG. 26 is a diagram illustrating an example of a relationship between a type of a classification model generated in a first embodiment and an index of accuracy in each classification model;

FIG. 27 is a diagram illustrating an example of a result of "k-fold cross validation (k = 10)" in the classification model generated in the first embodiment;

FIG. 28 is a diagram illustrating an example of a result of "k-fold cross validation (k = 10)" in a classification model generated on the basis of only the structural similarity as an example corresponding to the first embodiment;

FIG. 29 is a diagram illustrating an example of a result of "k-fold cross validation (k = 10)" in a classification model generated on the basis of only the structure descriptor (nine feature amounts) as an example corresponding to the first embodiment;

FIG. 30 is a diagram illustrating an example of a result of classification regarding seven pieces of test data of which a biological activity is assumed to be unknown, using the classification model generated in the first embodiment;

FIG. 31 is a diagram illustrating an example of a result of classification regarding seven pieces of test data of which a biological activity is assumed to be unknown, using the classification model generated on the basis of only the structural similarity, as an example corresponding to the first embodiment;

FIG. 32 is a diagram illustrating a result of arranging 10 molecules in a descending order of a value of an index "$S_{new}$" by analyzing 25 pieces of training data using the index "$S_{new}$" using an average of relative errors of a feature amount and the structural similarity;

FIG. 33 is a diagram illustrating a result of arranging 10 molecules in a descending order of a value of an index "$S_{DA}$" by analyzing 25 pieces of training data using the index "$S_{DA}$" using only the structural similarity;

FIG. 34 is a diagram illustrating a result of arranging 10 molecules in a descending order of a value of an index "1-$E_{ave}$" by analyzing 25 pieces of training data using the index "1-$E_{ave}$" using only the relative error of the feature amount;

FIG. 35 is a diagram illustrating an example of a result of "k-fold cross validation (k = 10)" in a classification model based on an average of relative errors of six feature amounts and a structural similarity, generated in a second embodiment;

FIG. 36 is a diagram illustrating an example of a result of "k-fold cross validation (k = 10)" in a classification model generated on the basis of the six feature amounts and the structural similarity;

FIG. 37 is a diagram illustrating an example of a result of classification regarding seven pieces of test data of which a biological activity is assumed to be unknown, using the classification model based on the average of the relative errors of the feature amounts and the structural similarity, generated in the second embodiment;

FIG. 38 is a diagram illustrating an example of a relationship between a type of a classification model generated in a third embodiment and an index of accuracy in each classification model;

FIG. 39 is a diagram illustrating an example of a result of "k-fold cross validation (k = 10)" in a prediction model generated in the third embodiment;

FIG. 40 is a diagram illustrating an example of a result of "k-fold cross validation (k = 10)" in a prediction model generated on the basis of only a structural similarity as an example corresponding to the third embodiment;

FIG. 41 is a diagram illustrating an example of a result of "k-fold cross validation (k = 10)" in a prediction model generated on the basis of only a structure descriptor (14 feature amounts) as an example corresponding to the third embodiment;

FIG. 42 is a diagram illustrating an example of a result of predicting a viscosity of test data of which the viscosity is assumed to be unknown using the prediction model generated in the third embodiment;

FIG. 43 is a diagram illustrating a result of predicting the viscosity of the test data of which the viscosity is assumed to be unknown using a prediction model generated on the basis of only the structural similarity as an example corresponding to the third embodiment; and

FIG. 44 is a diagram illustrating a result of predicting the viscosity of the test data of which the viscosity is assumed to be unknown using a prediction model generated on the basis of only the structure descriptor (14 feature amounts) as an example corresponding to the third embodiment.

[Description of Embodiments]

(Information Processing Program)

The technology disclosed in this case is based on findings of the inventors such that there is a case where it is not possible to generate a model that can analyze a molecule, of which a characteristic value (characteristic data) of a predetermined characteristic is not specified, with high accuracy with the related art. Therefore, before describing details of the technology disclosed in this case, problems or the like of the related art will be described.

As described above, when a molecule having a physical property value close to the molecule is searched and narrowed based on a molecule of which a target characteristic value is known, for example, a model generated by performing machine learning based on information regarding the molecule of which the characteristic value is known can be used. More specifically, when the molecule having the characteristic value close to the target molecule characteristic value is narrowed from a large number of molecules, for example, it is possible to use a model that performs regression prediction of a physical property value (multiple regression model), a model that classifies molecules (class classifier), or the like.

Here, FIG. 1 illustrates an example of an ideal relationship between a target molecule of which a physical property value is known and a candidate molecule of which a physical property value is unknown when a model that performs regression prediction of the physical property value is generated and the physical property value of the molecule of which the physical property value is unknown is predicted through the regression prediction. In FIG. 1, the horizontal axis indicates a feature amount representing characteristics of a molecule, and the vertical axis indicates a physical property value to be narrowed (target physical property value).

As illustrated in FIG. 1, for example, it is desirable for the model that performs regression prediction (multiple regression model) to be able to narrow the candidate molecule CM by predicting a target physical property value of each candidate molecule CM and specifying a candidate molecule CM1 of which a target physical property value is close to that of a target molecule QM. Note that, in FIG. 1, a candidate molecule CM2 means a candidate molecule of which a target physical property value is not close to the target molecule QM.

Subsequently, FIG. 2 illustrates an example of an ideal relationship between the target molecule of which the physical property value is known and the candidate molecule of which the physical property value is unknown when a model that performs classification based on a physical property value is generated and molecules of which physical property values are unknown are classified.

As illustrated in FIG. 2, for example, it is desirable for the model that performs classification (classification model, class classifier) to be able to narrow the candidate molecule CM by specifying the candidate molecule CM1 classified into the same class as the target molecule QM. Note that, in FIG. 2, a candidate molecule CM2 means a candidate molecule to be classified into a class different from the target molecule QM.

As described above, in the related art, for example, the model that is used when the molecule having the characteristic value close to the characteristic value of the target molecule is narrowed from among a large number of molecules to be candidates is generated based on the structural similarity between the molecule of which the characteristic value is known and the molecule of which the characteristic value is unknown.

Here, FIG. 3 simply illustrates an example of a flow when a model used to analyze the molecule of which the characteristic value is unknown is generated and analyzed based on the structural similarity between the molecule of which the characteristic value is known and the molecule of which the characteristic value is unknown.

In an example of the related art illustrated in FIG. 3, first, input of information regarding a structure of a molecule of which a characteristic value is known is received (S101).

Next, in the example of the related art illustrated in FIG. 3, a structural similarity between the molecules is specified based on the information regarding the structure of the molecule (S102). More specifically, in S102, the structural similarity between the molecules of which the characteristic values are known is specified.

Subsequently, in the example of the related art illustrated in FIG. 3, a model for analysis is generated through machine learning based on the structural similarity and the characteristic value (S103). More specifically, in S103, a model (multiple regression model) that performs regression prediction of a physical property value, a model (class classifier) that classifies molecules, or the like are generated by learning a relationship between the structural similarity and the characteristic value.

Then, in the example of the related art illustrated in FIG. 3, input of information regarding a structure of a molecule of which a characteristic value is unknown is received, the received information is input to the model, and analysis is performed (S103). More specifically, in S104, the information regarding the structure of the molecule of which the characteristic value is unknown is input to the generated model, and the molecule of which the characteristic value is unknown is analyzed (regression prediction, classification, or the like).

In the example of the related art illustrated in FIG. 3, for example, as described above, the relationship between the characteristic value and the structural similarity of the molecule of which the characteristic value is unknown is specified, and a molecule having a physical property value close to the molecule of which the physical property value is known is searched for and narrowed.

FIG. 4 illustrates an example of a relationship between a target molecule of which a physical property value is known and a candidate molecule of which a physical property value is unknown in a case where analysis is performed using the related art that performs analysis based on a structural similarity. In FIG. 4, the horizontal axis indicates a structural similarity with a target molecule as an example of a feature amount representing characteristics of a molecule, and the vertical axis indicates a physical property value (target physical property value) to be narrowed.

As illustrated in FIG. 4, with the related art that performs analysis based on the structural similarity, because the structural similarity between the target molecule QM of which a target physical property value is a preferable value and each candidate molecule CM is not sufficiently correlated to a target characteristic value, accuracy of the analysis is lowered. That is, for example, with the related art, it is possible to perform only the analysis with low accuracy as illustrated in FIG. 4, and it has been difficult to perform appropriate analysis with high accuracy as illustrated in FIGs. 1 and 2.

As described above, in the related art, for example, because a correlation between the structural similarity between the molecules and the target physical property value decreases, for example, there is a case where accuracy of the model that analyzes the molecule of which the characteristic is unknown is lowered.

In other words, for example, in the related art, there has been a case where it is not possible to generate the model that can analyze the molecule, of which the characteristic value (characteristic data) of the predetermined characteristic is not specified, with high accuracy.

Therefore, the present inventors have repeatedly studied about a program or the like that can generate the model that can analyze the molecule, of which the characteristic value (characteristic data) of the predetermined characteristic is not specified, with high accuracy and have obtained the following findings.

In other words, for example, the present inventors have found that it is possible to generate the model that can analyze the molecule, of which the characteristic value (characteristic data) of the predetermined characteristic is not specified, with high accuracy with the following information processing program or the like.

The information processing program as an example of the technology disclosed in this case is an information processing program that analyzes a first molecule different from a plurality of molecules based on characteristic data of each of the plurality of molecules, and causes a computer to perform a model generation process for generating a model used to analyze the first molecule based on a similarity between respective structures of the plurality of molecules, and a structure descriptor that is an index specified based on the respective structures of the plurality of molecules.

In an example of the technology disclosed in this case, as described above, the first molecule different from all the plurality of molecules is analyzed based on the characteristic data of each of the plurality of molecules. More specifically, for example, a non-specific molecule (molecule of which physical property value is unknown) of which a characteristic value is not specified is analyzed based on data of a specific molecule group including a plurality of specific molecules (molecule of which physical property value is known) of which a characteristic value (characteristic data) of a predetermined characteristic is specified. That is, for example, in an example of the technology disclosed in this case, for example, on the basis of the characteristic data of each of the plurality of molecules (characteristic data of specific molecule), a model that analyzes the first molecule different from the plurality of molecules (for example, molecule of which characteristic value is unknown) is generated, and analysis is performed.

In an example of the technology disclosed in this case, by analyzing the first molecule (non-specific molecule) using the generated model, for example, it is possible to select a first molecule of which a target characteristic has a preferable value from among a large number of first molecules. In this way, in an example of the technology disclosed in this case, for example, it is possible to narrow the first molecule of which the target characteristic has a preferable value (candidate molecule of which characteristics are close to target molecule).

Here, in an example of the technology disclosed in this case, a model used to analyze the first molecule is generated based on a similarity between respective structures of a plurality of molecules and a structure descriptor that is an index specified based on the structure of each of the plurality of molecules. More specifically, for example, a model used to analyze a non-specific molecule is generated based on a structural similarity between specific molecules included in a specific molecule group and a structure descriptor that is an index specified on the basis of the structure in the specific molecule included in the specific molecule group. That is, for example, in an example of the technology disclosed in this case, for example, a model is generated by performing learning using a structure descriptor that is an index specified based on the structure of the specific molecule, in addition to the structural similarity between the plurality of molecules (specific molecule) of which the characteristic data is known.

The structure descriptor is an index that can be calculated by analyzing each molecule based on the information regarding the structure, and a large number of types of structure descriptors have been proposed so far. In an example of the technology disclosed in this case, for example, at least one of the structure descriptors of the plurality of molecules (specific molecule included in specific molecule group) is used to generate a model.

In this way, in an example of the technology disclosed in this case, the model used to analyze the first molecule (non-specific molecule) is generated using both of the similarity between the respective structures of the plurality of molecules and the structure descriptor of each of the plurality of molecules. In other words, for example, in an example of the technology disclosed in this case, for example, a model is generated based on both indexes including the structural similarity that is the index determined according to the structures of the two molecules and the structure descriptor that is the index determined according to the structure of one molecule (each molecule).

Therefore, in an example of the technology disclosed in this case, even in a case where the accuracy of the model is deteriorated with the related art, it is possible to generate a model based on an appropriate index. Therefore, it is possible to generate a model with higher accuracy. Therefore, in an example of the technology disclosed in this case, for example, it is possible to narrow the first molecule of which the target characteristic has a preferable value from among a large number of first molecules (non-specific molecule) with high accuracy.

FIG. 5 illustrates an example of a relationship between a target molecule of which a physical property value is known and a candidate molecule of which a physical property value is unknown in a case where analysis is performed using an example of the technology disclosed in this case for performing analysis based on the structural similarity and the structure descriptor. In FIG. 5, the horizontal axis indicates an index based on the structural similarity and the structure descriptor as an example of feature amounts representing characteristics of a molecule, and the vertical axis indicates a physical property value to be narrowed (target physical property value).

As illustrated in FIG. 5, in an example of the technology disclosed in this case, the index based on the structural similarity between the target molecule QM of which the target physical property value is a preferable value and each candidate molecule CM and the structure descriptor is sufficiently correlated to the target characteristic value, and the accuracy of the analysis can be improved. That is, for example, in an example of the technology disclosed in this case, even in a case where the accuracy of the analysis (regression prediction, classification, or the like) is deteriorated with the related art, the analysis with high accuracy as illustrated in FIG. 5 can be performed.

In this way, in an example of the technology disclosed in this case, the model used to analyze a non-specific molecule is generated based on the similarity between the respective structures of the plurality of molecules and the structure descriptor of each of the plurality of molecules. Therefore, in an example of the technology disclosed in this case, it is possible to generate a model that can analyze the molecule (first molecule, non-specific molecule), of which the characteristic value of the predetermined characteristic is not specified, with high accuracy.

Furthermore, when the first molecule (non-specific molecule) of which the characteristic value is unknown is analyzed, depending on an analysis target and a type of the analysis, what type of model has high accuracy becomes a complicated problem to which various causes contribute. Therefore, it is difficult to predict what type of model has high accuracy. That is, for example, depending on the analysis target and the type of the analysis, there may be a case where accuracy of another model is higher than that of the model based on the structural similarity between the plurality of molecules (specific molecule) and the structure descriptor of the plurality of molecules (specific molecule).

Therefore, in an example of the technology disclosed in this case, analysis using another model may be performed, in addition to the analysis using the model based on the structural similarity and the structure descriptor. For example, analysis using the model based on only the structural similarity and the model based on only the structure descriptor may be further performed. In this way, in an example of the technology disclosed in this case, also in a case where it is difficult to perform appropriate analysis with only the related art, it is possible to perform accurate analysis without exception regardless of the analysis target and the type of the model.

Hereinafter, in an example of an information processing program disclosed in this case, each process to be executed by a computer will be described in detail.

The information processing program disclosed in this case, for example, causes the computer to perform at least a model generation process and further causes the computer to perform other processes as needed.

The information processing program disclosed in this case can be created using various known programming languages according to a configuration of a computer system to be used, a type and version of an operating system, and the like.

The information processing program disclosed in this case may be recorded on a recording medium such as a built-in hard disk or an externally attached hard disk, or may be recorded on a recording medium such as a compact disc read only memory (CD-ROM), a digital versatile disk read only memory (DVD-ROM), a magneto-optical (MO) disk, or a universal serial bus (USB) memory [USB flash drive].

Moreover, in a case of recording the information processing program disclosed in this case on the above-described recording medium, the program can be directly used or can be installed into a hard disk and then used through a recording medium read device included in the computer system, as needed. Furthermore, the information processing program disclosed in this case may be recorded on an external storage region (another computer or the like) accessible from the computer system through an information communication network. In this case, the information processing program disclosed in this case, which is recorded on the external storage region, can be directly used or can be installed in a hard disk and then used through the information communication network from the external storage region, as needed.

Note that the information processing program disclosed in this case may be divided for each of arbitrary pieces of processing and recorded on a plurality of recording media.

Furthermore, processing for executing each process by the information processing program disclosed in this case can be, for example, executed by a central processing unit (CPU), a graphics processing unit (GPU), a processing device of an annealing machine to be described later, a combination of these, or the like.

The information processing program disclosed in this case is a program that analyzes the first molecule different from the plurality of molecules based on the characteristics data of each of the plurality of molecules. More specifically, the information processing program may be a program that analyzes the non-specific molecule of which the characteristic value is not specified based on the data of the specific molecule group including the plurality of specific molecules of which the characteristic value of the predetermined characteristic is specified.

The characteristic value (example of characteristic data) of the predetermined characteristic is not particularly limited as long as the characteristic value is a value representing characteristics (physical property) of a molecule and can be appropriately selected depending on a purpose. The characteristic value of the predetermined characteristic is, for example, a physical characteristic value, a chemical characteristic value, a biological characteristic value, or the like.

The physical or chemical characteristic value is, for example, a mechanical characteristic value (mechanistic characteristic value), a thermal characteristic value, an electrical characteristic value, a magnetic characteristic value, an optical characteristic value, or the like. More specifically, these characteristic values are, for example, a viscosity, density, permittivity, permeability, magnetic susceptibility, electric conductivity, thermal conductivity, specific heat, linear expansion coefficient, boiling point, melting point, elastic modulus, glass-transition point, refractive index, or the like.

Furthermore, the biological characteristic value is, for example, a biological activity used to analyze a quantitative structure-activity relationship (QSAR), quantitative structure-property relationship (QSPR), or the like. Furthermore, the biological activity may be, for example, represented by two values including "Active (active)" or "Inactive (inactive)" or may be continuous values representing an activity strength. As described above, the characteristic value of the predetermined characteristic may be, for example, a discrete value or continuous values.

Furthermore, in an example of the technology disclosed in this case, the specific molecule of which the characteristic value is specified (target molecule, plurality of molecules of which characteristic data is known) is not particularly limited as long as the specific molecule is a molecule of which a characteristic value is specified (characteristic value is known) and can be appropriately selected depending on a purpose.

In an example of the technology disclosed in this case, the data of the specific molecule group including the plurality of specific molecules of which the characteristic value is specified (example of characteristic data) is not particularly limited as long as the data includes data of a plurality of specific molecules and can be appropriately selected depending on a purpose. The data of the specific molecule group can be, for example, data in which information regarding the characteristic value of the specific molecule and information regarding a structure of the specific molecule are associated with each other, for the plurality of specific molecules.

The number of specific molecules (plurality of molecules)included in the specific molecule group is not particularly limited as long as the number is plural and can be appropriately selected depending on a purpose. However, for example, it is preferable to increase the number of specific molecules included in the specific molecule group (plurality of molecules) according to accuracy of a needed model. In an example of the technology disclosed in this case, for example, a model is generated using the data of the specific molecule group as training data (learning data) when the model is generated. Therefore, for example, by training (learning) a model based on data of a specific molecule group including a large number of specific molecules, the accuracy of the model can be further improved.

In an example of the technology disclosed in this case, the first molecule is not particularly limited as long as the first molecule is different from the plurality of molecules, and can be appropriately selected depending on a purpose. More specifically, the first molecule (non-specific molecule of which characteristic value is not specified, target molecule) can be a molecule of which a characteristic value is not specified (characteristic value is unknown). Furthermore, "the characteristic value is not specified (characteristic value is unknown)" means, for example, that "a predetermined characteristic (target characteristic)" to be analyzed using a model is not specified.

In an example of the technology disclosed in this case, as described above, for example, by analyzing the non-specific molecule using the model generated based on the data of the specific molecule group, it is possible to perform regression prediction, classification, or the like regarding the characteristic value of the non-specific molecule.

Furthermore, in an example of the technology disclosed in this case, the number of first molecules (non-specific molecule) to be analyzed is not particularly limited and can be appropriately selected depending on a purpose. That is, for example, in an example of the technology disclosed in this case, it is possible to analyze the plurality of non-specific molecules, and for example, it is possible to select (narrow) a non-specific molecule having a preferable characteristic value from among the plurality of non-specific molecules.

<Model Generation Process>

In a model generation process according to the technology disclosed in this case, a model used to analyze a first molecule is generated based on a similarity between respective structures of a plurality of molecules and a structure descriptor that is an index specified based on the structure of each of the plurality of molecules. More specifically, for example, a model used to analyze a non-specific molecule is generated based on a structural similarity between specific molecules included in a specific molecule group and a structure descriptor that is an index specified based on the structure in the specific molecule included in the specific molecule group.

<<Calculation of Structural Similarity>>

In the model generation process, the similarity between the structures used to generate the model is not particularly limited as long as the similarity is a similarity based on a structure of each molecule between molecules included in a plurality of molecules (specific molecule group), and can be appropriately selected according to a purpose.

A method for calculating the similarity between the respective structures of the plurality of molecules is not particularly limited and can be appropriately selected depending on a purpose. The method for calculating the similarity between the respective structures of the plurality of molecules includes, for example, a method using known software that analyzes a structure of a molecule, a method using a "conflict graph" representing a combination of atoms in the structure of which the similarity is calculated, or the like.

In the method using the known software that analyzes the structure of the molecule in order to calculate the structural similarity, for example, software called "RDKit" can be used. The "RDKit" is an open source Python library used in the chemoinformatics field. For example, "G. Landrum, RDKit: Open-Source Cheminformatics, (http://www.rdkit.org.)" describes details of "RDKit".

In the method using the "conflict graph" representing the combination of the atoms in the structure of which the similarity is calculated in order to calculate the structural similarity, for example, it is possible to obtain the similarity by searching for a maximum independent set (solving maximum independent set problem). In an example of the technology disclosed in this case, in this way, it is preferable to obtain a similarity by specifying a substructure that is common to each structure by searching for the maximum independent set for the conflict graph.

In the following, details of the method using the conflict graph representing the combination of the atoms in the structure of which the similarity is calculated in order to calculate the structural similarity will be described.

Here, when the structural similarity between the molecules is calculated by solving the maximum independent set problem in the conflict graph, the molecules are expressed as graphs to be handled. Here, to express a molecule as a graph means to represent a structure of a molecule by using, for example, information regarding a type of atoms (elements) in the molecule and information regarding a bonding state between the individual atoms.

Furthermore, in this example, the structure of the molecule can be represented using, for example, an expression in a MOL format or a structure data file (SDF) format. Usually, the SDF format means a single file obtained by collecting structural information regarding a plurality of molecules expressed in the MOL format. Furthermore, in addition to the MOL format structural information, the SDF format file is capable of treating additional information (for example, catalog number, chemical abstracts service (CAS) number, molecular weight, or the like) for each molecule. Such structures of these molecules can be expressed as a graph in a comma-separated value (CSV) format in which, for example, "atom 1 (name), atom 2 (name), element information of atom 1, element information of atom 2, bond order between atom 1 and atom 2" are contained in a single row.

In the following, a method for creating the conflict graph will be described first by taking, as an example, a case where a conflict graph of acetic acid ($CH_3COOH$) and methyl acetate ($CH_3COOCH_3$) is created, as an example of obtaining a similarity between molecules.

First, acetic acid (hereinafter, may be referred to as "molecule A") and methyl acetate (hereinafter, may be referred to as "molecule B") expressed as graphs are as illustrated in FIG. 6. In FIG. 6, atoms that form acetic acid are indicated by A1, A2, A3, and A5, and atoms that form methyl acetate are indicated by B1 to B5. Furthermore, in FIG. 6, A1, A2, B1, B2, and B4 indicate carbon, and A3, A5, B3, and B5 indicate oxygen, a single bond is indicated by a thin solid line, and a double bond is indicated by a thick solid line.

Next, vertices (atoms) in the molecules A and B expressed as a graph are combined with each other to create vertices (nodes) of a conflict graph. At this time, for example, as illustrated in FIG. 7, it is preferable to combine the same elements in the molecules A and B with each other to create the nodes of the conflict graph. In the example illustrated in FIG. 7, combinations of A1, A2, B1, B2, and B4 that represent carbon and combinations of A3, A5, B3, and B5 that represent oxygen are employed as nodes of the conflict graph.

Subsequently, edges (branches or sides) in the conflict graph are created. At this time, two nodes are compared, and in a case where the nodes are constituted by atoms in different situations from each other (for example, atomic number, presence or absence of bond, bond order, or the like), an edge is created between these two nodes. Whereas, in a case where two nodes are compared and the nodes are constituted by atoms in the same situation, edge between these two nodes is not created.

Here, a rule for creating the edge in the conflict graph will be described with reference to FIG. 8.

First, in the example illustrated in FIG. 8, whether or not an edge is created between a node [A1B1] and a node [A2B2] will be described. As can be seen from the structure of the molecule A expressed as a graph in FIG. 8, the carbon A1 of the molecule A included in the node [A1B1] and the carbon A2 of the molecule A included in the node [A2B2] are bonded (single bonded) to each other. Likewise, the carbon B1 of the molecule B included in the node [A1B1] and the carbon B2 of the molecule B included in the node [A2B2] are bonded (single bonded) to each other. In other words, for example, the situation of bonding between the carbon A1 and the carbon A2 and the situation of bonding between the carbon B1 and the carbon B2 are identical to each other.

In this manner, in the example in FIG. 8, the situation of the carbon A1 and the carbon A2 in the molecule A and the situation of the carbon B1 and the carbon B2 in the molecule B are identical to each other, and the node [A1B1] and the node [A2B2] are deemed as nodes constituted by atoms in identical situations to each other. Therefore, in the example illustrated in FIG. 8, edge between the node [A1B1] and the node [A2B2] is not created.

Next, in the example illustrated in FIG. 8, whether or not an edge is created between a node [A1B4] and the node [A2B2] will be described. As can be seen from the structure of the molecule A expressed as a graph in FIG. 8, the carbon A1 of the molecule A included in the node [A1B4] and the carbon A2 of the molecule A included in the node [A2B2] are bonded (single bonded) to each other. Whereas, as can be seen from the structure of the molecule B expressed as a graph, the carbon B4 of the molecule B included in the node [A1B4] and the carbon B2 of the molecule B included in the node [A2B2] have the oxygen B3 sandwiched between the carbons B4 and B2, and are not directly bonded. In other words, for example, the situation of bonding between the carbon A1 and the carbon A2 and the situation of bonding between the carbon B4 and the carbon B2 are different from each other.

That is, for example, in the example in FIG. 8, the situation of the carbon A1 and the carbon A2 in the molecule A and the situation of the carbon B4 and the carbon B2 in the molecule B are different from each other, and the node [A1B4] and the node [A2B2] are deemed as nodes constituted by atoms in different situations from each other. Therefore, in the example illustrated in FIG. 8, an edge is created between the node [A1B4] and the node [A2B2].

In this manner, the conflict graph can be created based on the rule that, in a case where nodes are constituted by atoms in different situations, an edge is created between these nodes, and in a case where nodes are constituted by atoms in the same situation, edge between these nodes is not created.

FIG. 9 is a diagram illustrating an example of a conflict graph of the molecule A and the molecule B. As illustrated in FIG. 9, for example, in the node [A2B2] and a node [A5B5], the situation of bonding between the carbon A2 and the oxygen A5 in the molecule A and the situation of bonding between the carbon B2 and the carbon B5 in the molecule B are identical to each other. Therefore, the node [A2B2] and the node [A5B5] are deemed as nodes constituted by atoms in identical situations to each other, and thus edge between the node [A2B2] and the node [A5B5] has not been created.

Next, an example of the method for solving the maximum independent set problem of the created conflict graph will be described.

The maximum independent set (MIS) in the conflict graph means a set that includes the largest number of nodes that do not have edges between the nodes among sets of nodes constituting the conflict graph.

In other words, for example, the maximum independent set in the conflict graph means a set that has the maximum size (number of nodes) among sets formed by nodes that have no edges between the nodes with each other.

FIG. 10 is a diagram illustrating an example of a maximum independent set in a graph. In FIG. 10, nodes included in a set are denoted with a reference sign of "1", and nodes not included in any set are denoted with a reference sign of "0"; for instances where edges exist between nodes, the nodes are connected by solid lines, and for instances where no edge exists, the nodes are connected by dotted lines. Note that, here, as illustrated in FIG. 10, a graph of which the number of nodes is six will be described as an example for simplification of explanation.

In the example illustrated in FIG. 10, among sets constituted by nodes that have no edges between the nodes, there are three sets having the maximum number of nodes, and the number of nodes in each of these sets is three. In other words, for example, in the example illustrated in FIG. 10, three sets surrounded by an alternate long and short dash line are the maximum independent sets in the graph.

Here, as described above, the conflict graph is created based on the rule that, in a case where nodes are constituted by atoms in different situations, an edge is created between these nodes, and in a case where nodes are constituted by atoms in the same situation, edge is between these nodes not created. Therefore, in the conflict graph, to obtain the maximum independent set, which is a set having the maximum number of nodes, among sets constituted by nodes that have no edges between the nodes, is synonymous with to obtain the largest substructure among substructures common to two molecules. In other words, for example, the largest common substructure of two molecules can be specified by obtaining the maximum independent set in the conflict graph.

FIG. 11 illustrates an example of a flow in a case where a maximum common substructure of the molecule A (acetic acid) and the molecule B (methyl acetate) is obtained by obtaining the maximum independent set in the conflict graph (solving maximum independent set problem). As illustrated in FIG. 11, a conflict graph is created in such a manner that the molecule A and the molecule B are each expressed as a graph, the same elements are combined and employed as a node, and an edge is formed according to the situation of atoms constituting the node. Then, by obtaining the maximum independent set in the created conflict graph, the maximum common substructure of the molecule A and the molecule B can be obtained.

Here, an example of a specific method for obtaining (searching for) the maximum independent set in the conflict graph will be described.

The maximum independent set in the conflict graph may be searched for by, for example, using a Hamiltonian in which minimizing means searching for the maximum independent set. More specifically, for example, the search can be performed by using a Hamiltonian (H) indicated by the following equation.

[Expression 1]

$$H = -\alpha \sum_{i=0}^{n-1} b_i x_i + \beta \sum_{i,j=0}^{n-1} w_{ij} x_i x_j$$

Here, in the above equation, n indicates the number of nodes in the conflict graph, and $b_i$ is a numerical value that represents a bias for an i-th node.

Moreover, $w_{ij}$ has a positive non-zero number when there is an edge between the i-th node and a j-th node, and has zero when there is no edge between the i-th node and the j-th node.

Furthermore, $x_i$ represents a binary variable representing that the i-th node has zero or one, and $x_j$ represents a binary variable representing that the j-th node has zero or one.

Note that $\alpha$ and $\beta$ are positive numbers.

A relationship between the Hamiltonian represented by the above equation and the search for the maximum independent set will be described in more detail. The above equation is a Hamiltonian that represents an Ising model equation in the quadratic unconstrained binary optimization (QUBO) format.

In the above equation, in a case where $x_i$ is one, it means that the i-th node is included in a set that is a candidate for the maximum independent set, and in a case where $x_i$ is zero, it means that the i-th node is not included in a set that is a candidate for the maximum independent set. Likewise, in the above equation, in a case where $x_j$ is one, it means that the j-th node is included in a set that is a candidate for the maximum independent set, and in a case where $x_j$ is zero, it means that the j-th node is not included in a set that is a candidate for the maximum independent set.

Therefore, in the above equation, by searching for a combination in which as many nodes as possible have the state of one under the constraint that there is no edge between nodes whose states are designated as one (bits are designated as one), the maximum independent set can be searched.

Here, each term in the above equation will be described.

The first term on the right side of the above equation (term with coefficient of - a) is a term whose value becomes smaller as the number of i whose $x_i$ is one increases (as the number of nodes included in set that is candidate for maximum independent set increases). Note that, the value of the first term on the right side of the above equation becoming smaller means that a larger negative number is given. That is, for example, in the above equation, the value of the Hamiltonian (H) becomes smaller when many nodes have the bit of one, due to an action of the first term on the right side.

The second term on the right side of the above equation (term with coefficient of $\beta$) is a term of a penalty whose value becomes larger in a case where there is an edge between nodes whose bits have one (in a case where $w_{ij}$ has positive non-zero number). In other words, for example, the second term on the right side of the above equation has zero in a case where there is no instance where an edge exists between nodes whose bits have one, and has a positive number in other cases. That is, for example, in the above equation, the value of the Hamiltonian (H) becomes larger when there is an edge between nodes whose bits have one, due to an action of the second term on the right side.

As described above, the above equation has a smaller value when many nodes have the bit of one, and has a larger value when there is an edge between the nodes whose bits have one, and accordingly, it can be said that minimizing the above equation means searching for the maximum independent set.

Here, the relationship between the Hamiltonian represented by the above equation and the search for the maximum independent set will be described using an example with reference to the drawings.

A case where the bit is set in each node as in the example illustrated in FIG. 12 in a graph of which the number nodes is six will be considered. In the example in FIG. 12, as in FIG. 10, for instances where edges exist between nodes, the nodes are connected by solid lines, and for instances where no edges exists, the nodes are connected by dotted lines.

In the example in FIG. 12, when it is assumed, in the above equation, that $b_i$ be one and $w_{ij}$ be one when there is an edge between the i-th node and the j-th node, the above equation is as follows.

[Expression 2]

$$\begin{aligned}H &= -\alpha(x_0 + x_1 + x_2 + x_3 + x_4 + x_5) \\ &\quad + \beta(\lambda_{01}x_0x_1 + \lambda_{02}x_0x_2 + \lambda_{03}x_0x_3 + \lambda_{04}x_0x_4 + \lambda_{05}x_0x_5 + \cdots) \\ &= -\alpha(1 + 0 + 1 + 0 + 1 + 0) \\ &\quad + \beta(1 * 1 * 0 + 0 * 1 * 1 + 0 * 1 * 0 + 0 * 1 * 1 + 0 * 1 * 0 + \cdots) \\ &= -3\alpha\end{aligned}$$

In this manner, in the example in FIG. 12, in a case where there is no instance where an edge exists between nodes whose bits have one (in a case where there is no contradiction as independent set), the second term on the right side has zero, and the value of the first term is the value of the Hamiltonian as it is.

Next, a case where the bit is set in each node as in the example illustrated in FIG. 13 will be considered. As in the example in FIG. 12, when it is assumed, in the above equation, that $b_i$ be one and $w_{ij}$ be one when there is an edge between the i-th node and the j-th node, the above equation is as follows.

[Expression 3]

$$\begin{aligned}H &= -\alpha(x_0 + x_1 + x_2 + x_3 + x_4 + x_5) \\ &\quad + \beta(\lambda_{01}x_0x_1 + \lambda_{02}x_0x_2 + \lambda_{03}x_0x_3 + \lambda_{04}x_0x_4 + \lambda_{05}x_0x_5 + \cdots) \\ &= -\alpha\left(1 + \underline{1} + 1 + 0 + 1 + 0\right) \\ &\quad + \beta\left(1 * 1 * \underline{1} + 0 * 1 * 1 + 0 * 1 * 0 + 0 * 1 * 1 + 0 * 1 * 0 + \cdots\right) \\ &= -4\alpha + 5\beta\end{aligned}$$

In this manner, in the example in FIG. 13, since there is an instance where an edge exists between nodes whose bits have one, the second term on the right side does not have zero, and the value of the Hamiltonian is the sum of the two terms on the right side. Here, in the examples illustrated in FIGs. 12 and 13, for example, when $\alpha > 5\beta$ is assumed, $-3\alpha < -4\alpha + 5\beta$ is satisfied, and accordingly, the value of the Hamiltonian in the example in FIG. 12 is smaller than the value of the Hamiltonian in the example in FIG. 13. In the example in FIG. 12, it can be seen that the maximum independent set can be retrieved by searching for a set of nodes that has no contradiction as the maximum independent set that is a combination of nodes in which the value of the Hamiltonian in the above equation (1) is smaller.

Next, an example of a method for calculating a structural similarity between molecules on the basis of the searched maximum independent set will be described.

The structural similarity between the molecules can be calculated, for example, using the following equation.

[Expression 4]

$$S(G_A, G_B) = \delta \max\left\{\frac{|V_C^A|}{|V_A|}, \frac{|V_C^B|}{|V_B|}\right\} + (1 - \delta)\min\left\{\frac{|V_C^A|}{|V_A|}, \frac{|V_C^B|}{|V_B|}\right\}$$

Here, in the above equation of the similarity, $S(G_A, G_B)$ represents a similarity between a first molecule expressed as a graph (for example, molecule A) and a second molecule expressed as a graph (for example, molecule B), is represented as zero to one, and means that the similarity is higher as the value is closer to one.

Furthermore, $V_A$ represents the total number of node atoms of the first molecule expressed as a graph, and $V_c^A$ represents the number of node atoms included in the maximum independent set of the conflict graph among the node atoms of the first molecule expressed as a graph. Note that, the node atom means an atom at a vertex of a molecule expressed as a graph.

Moreover, $V_B$ represents the total number of node atoms of the second molecule expressed as a graph, and $V_c^B$ represents the number of node atoms included in the maximum independent set of the conflict graph among the node atoms of the second molecule expressed as a graph.

$\delta$ is a number from zero to one.

Furthermore, in the above equation of the similarity, max {A, B} means to select a larger value from among A and B, and min {A, B} means to select a smaller value from among A and B.

Here, as in the examples illustrated in FIGs. 6 to 13, a method for calculating a similarity will be described using acetic acid (molecule A) and methyl acetate (molecule B) as examples.

In a conflict graph illustrated in FIG. 14, the maximum independent set includes four nodes: a node [A1B1], a node [A2B2], a node [A3B3], and a node [A5B5]. That is, for example, in the example in FIG. 14, $|V_A|$ is set as four, $|V_c^A|$ is set as four, $|V_B|$ is set as five, and $|V_c^B|$ is set as four. Furthermore, in this example, when it is assumed that δ be 0.5 and the first molecule and the second molecule are averaged (treated equally), the above equation of the similarity is as follows.

[Expression 5]

$$S(G_A, G_B) = 0.5 * \max\left\{\frac{4}{4}, \frac{4}{5}\right\} + (1 - 0.5) * \min\left\{\frac{4}{4}, \frac{4}{5}\right\}$$

$$= 0.5 * \frac{4}{4} + (1 - 0.5) * \frac{4}{5} = 0.9$$

In this manner, in the example in FIG. 14, the structural similarity between molecules can be calculated as 0.9 based on the above equation of the similarity.

In the above, the method for calculating the similarity between the molecules has been described in detail. However, in an example of the technology disclosed in this case, it is possible to obtain a structural similarity between specific molecules included in a specific molecule group including a plurality of specific molecules of which a characteristic value is specified using the method described above.

In other words, for example, in an example of the technology disclosed in this case, it is preferable to obtain a similarity by searching for a maximum independent set based on molecule structures of a second molecule and a third molecule included in a plurality of molecules using the following equation (1).

[Expression 6]

$$H = -\alpha \sum_{i=0}^{n-1} b_i x_i + \beta \sum_{i,j=0}^{n-1} w_{ij} x_i x_j \quad \text{... EQUATION (1)}$$

Where, in the equation (1), H is a Hamiltonian that means minimizing the H is searching for a maximum independent set, n corresponds to the number of nodes of a conflict graph of a second molecule and a third molecule expressed as graphs, the conflict graph corresponds to a graph created based on a rule in which a combination of each node atom included in the second molecule expressed as a graph and each node atom included in the third molecule expressed as a graph is set as a node, the plurality of nodes is compared and an edge between the nodes that are not identical to each other is created, and the plurality of nodes is compared and an edge is not created between the nodes that are identical to each other,$b_i$ is a numerical value representing a bias with respect to an i-th node,$w_{ij}$ is a positive number that is not zero when an edge exists between the i-th node and a j-th node and is zero when no edge exists between the i-th node and the j-th node,$x_i$ is a binary variable representing that the i-th node is zero or one,$x_j$ is a binary variable representing that the j-th node is zero or one, andα and β are positive numbers.

Here, in an example of the technology disclosed in this case, "a plurality of nodes is compared and are identical to each other" means that, when a plurality of nodes is compared, these nodes are constituted by node atoms in the same situations (bonding situations) from each other. Likewise, in the example of the technology disclosed in this case, "a plurality of nodes is compared and are not identical to each other" means that, when a plurality of nodes are compared, these nodes are constituted by node atoms in different situations (bonding situations) from each other.

In the example of the technology disclosed in this case, in a case where the search for the maximum independent set is performed using above equation (1), it is not highly prioritized to create the conflict graph of the second molecule and the third molecule expressed as graphs, and it is sufficient that at least above equation (1) can be minimized. In other words, for example, in the example of the technology disclosed in this case, the search for the maximum independent set in the conflict graph of the second molecule and the third molecule is replaced with a combination optimization problem in a Hamiltonian in which minimizing means searching for the maximum independent set, and the problem is solved. Here, the minimization of the Hamiltonian represented by the Ising model equation in the QUBO format as in the above equation (1) can be executed in a short time by performing an annealing method (annealing) using an annealing machine or the like.

Therefore, in the technology disclosed in this case, in one aspect, by using the above equation (1), it is possible to search for the maximum independent set with the annealing method using the annealing machine or the like. Therefore, it is possible to analyze a non-specific molecule in a shorter time by searching for a maximum independent set. In other words, for example, in the technology disclosed in this case, in one aspect, it is possible to analyze a non-specific molecule in a shorter time by searching for a maximum independent set by minimizing the Hamiltonian (H) in the above equation (1) with the annealing method.

Examples of the annealing machine used to search for the maximum independent set include a quantum annealing machine, a semiconductor annealing machine using the semiconductor technology, a machine that performs simulated annealing executed by software by using a central processing unit (CPU) or a graphics processing unit (GPU), and the like, for example. Furthermore, for example, a digital annealer (registered trademark) may be used as the annealing machine.

Note that details of the annealing method using the annealing machine will be described below.

Moreover, in an example of the technology disclosed in this case, it is preferable to obtain a structural similarity for the searched maximum independent set using the following equation (2).

[Expression 7]

$$S(G_A, G_B) = \delta \max\left\{\frac{|V_C^A|}{|V_A|}, \frac{|V_C^B|}{|V_B|}\right\} + (1-\delta)\min\left\{\frac{|V_C^A|}{|V_A|}, \frac{|V_C^B|}{|V_B|}\right\} \quad \text{... EQUATION (2)}$$

Where, in the equation (2), $G_A$ represents a second molecule expressed as a graph,$G_B$ represents a third molecule expressed as a graph,S ($G_A$, $G_B$) represents a similarity between the second molecule expressed as a graph and the third molecule expressed as a graph, is represented by zero to one, and means that the similarity is higher as S ($G_A$, $G_B$) is closer to one,$V_A$ represents the total number of node atoms of the second molecule expressed as a graph, $V_c^A$ represents the number of node atoms included in the maximum independent set of the conflict graph of the node atoms of the second molecule expressed as a graph,$V_B$ represents the total number of node atoms of the third molecule expressed as a graph,$V_c^B$ represents the number of node atoms included in the maximum independent set of the conflict graph of the node atoms of the third molecule expressed as a graph, and$\delta$ is a number of zero to one.

In one aspect, the technology disclosed in this case can obtain the similarity regarding the characteristics between the second molecule (first specific molecule) and the third molecule (second specific molecule) based on the maximum independent set searched according to the above equation (1), by obtaining the similarity of the searched maximum independent set using the above equation (2).Furthermore, in order to calculate a structural similarity, for example, content disclosed in the following Non-Patent Document can be appropriately used.

Non-Patent Document: Maritza Hernandez, Arman Zaribafiyan, Maliheh Aramon, Mohammad Naghibi "A Novel Graph-based Approach for Determining Molecular Similarity". arXiv: 1601.06693 (https://arxiv.org/pdf/1601.06693.pdf)

In addition, in an example of the technology disclosed in this case, it is preferable that the node in the conflict graph be a combination of two node atoms that have the same atom type subdivided from the elemental species between the second molecule and the third molecule.

In this way, in an example of the technology disclosed in this case, for example, it is possible to improve the accuracy of the structural similarity and can reduce the number of nodes (reduce the number of bits needed for calculation).

When the node of the conflict graph is configured from the combination of the two atoms that have the same atom type, which is subdivided from the elemental species, between the first specific molecule and the second specific molecule, it is preferable that the atom type include, for example, a hybrid orbital of the outermost shell electron of an atom, a type of aromaticity, a type of chemical environment, or the like.

Furthermore, for example, it can be assumed that the plurality of nodes of the conflict graph can be nodes configured by a combination of two atoms having the same atom type and the same bond type, between the first specific molecule and the second specific molecule. The bond type includes, for example, whether or not the concerned combination is included in an aromatic ring and whether or not the concerned combination has a coordinate bond.

FIG. 15 is a diagram illustrating an example of a state of expressing acetic acid and methyl acetate as graphs.

In FIG. 15, atoms that form acetic acid are indicated by A1, A2, A3, and A5, and atoms that form methyl acetate are indicated by B1 to B5. Furthermore, in FIG. 15, A1, A2, B1, B2, and B4 indicate carbon, and A3, A5, B3, and B5 indicate oxygen, while a single bond is indicated by a thin solid line and a double bond is indicated by a thick solid line. Note that, in the example illustrated in FIG. 15, atoms other than hydrogen are selected and expressed as graphs. However, when a compound is expressed as a graph, all atoms including hydrogen may be selected and expressed as a graph. This graph is the same as the graph illustrated in FIG. 6 up to this point. However, in FIG. 15, carbon and oxygen are further subdivided on the basis of the hybrid orbital, the aromaticity, and the chemical environment. In FIG. 15, the atom type is subdivided on the basis of the atom type of the general AMBER force field (GAFF). The GAFF atom type is introduced, for example, in Table 1 or the like in the following document.

Document: JUNMEI WANG, ROMAIN M. WOLF, JAMES W. CALDWELL, PETER A. KOLLMAN, DAVID A. CASE, "Development and Testing of a General Amber Force Field", Journal of Computational Chemistry, Vol. 25, No. 9

Here, in FIG. 15, "c3" represents $sp^3$ carbon, "c2" represents aliphatic $sp^2$ carbon, "o" represents $sp^2$ oxygen in C=O or $COO^-$, "oh" represents $sp^3$ oxygen in a hydroxyl group, and "os" represents $sp^3$ oxygen in ether or ester.

Furthermore, an atom type and a bond type (bonding situation) can be defined, for example, by using "antechamber" that is a module included in an AMBER Tool.

The graph of acetic acid and the graph of methyl acetate in FIG. 15 have information regarding the atom types of these.

Next, the vertices (atoms) of the molecules A and B expressed as graphs are combined to create vertices (nodes) of the conflict graph. At this time, for example, as illustrated in FIG. 16, the same atom types in the molecules A and B are combined and employed as nodes of the conflict graph. In the example illustrated in FIG. 16, a combination of A1, B1, and B4 that represents the atom type "c3", a combination of A2 and B2 that represents the atom type "c2", and a combination of A5 and B5 that represents the atom type "o" are employed as nodes of the conflict graph. In this manner, by employing, as a node, the combination of not the same elements but the atoms that have the same atom type, which is subdivided from the elemental species, the number of nodes may be suppressed, and the number of bits of a calculator needed to solve the maximum independent set problem may be reduced.

In the example in FIG. 16, the number of nodes of the conflict graph created from the molecules A and B expressed as a graph is four. A conflict graph created based on these four nodes is as illustrated in FIG. 17. In this way, by employing the atoms having the same atom type as nodes, it is possible to improve the accuracy of the structural similarity, and it is possible to reduce the number of nodes (reduce the number of bits needed for calculation).

Furthermore, in an example of the technology disclosed in this case, when a structural similarity between molecules is obtained, a molecule to be a reference of a similarity may be selected and a similarity with the molecule may be calculated for each of other molecules (one-to-many), or the similarities of all patterns of combinations of molecules used for analysis may be calculated (many-to-many).

In a case where the similarity with the molecule to be the reference is calculated when the structural similarity between the plurality of molecules (specific molecule) is obtained, the molecule to be the reference can be appropriately selected, and for example, can be a molecule having a particularly preferable value of characteristics (activity value or the like). Whereas, in a case where similarities of all patterns of combinations of molecules are calculated when the structural similarity between the specific molecules is obtained, it is preferable to specify the similarity that contributes to improve the accuracy of the model from among a large number of the calculated similarities and to use the specified similarity for learning of a model. Note that, the similarity that contributes to improve the accuracy of the model can be specified, for example, with "Boruta" to be described later.

<<Calculation of Structure Descriptor>>

In the model generation process, a structure descriptor used to generate a model is not particularly limited as long as the structure descriptor and is an index specified based on a structure of each of a plurality of molecules and can be appropriately selected depending on a purpose.

A method for calculating the structure descriptor of the plurality of molecules (specific molecule) is not particularly limited and can be appropriately selected depending on a purpose. The method for calculating the structure descriptor of the plurality of molecules (specific molecule) includes, for example, a method using known software that analyzes a structure of a molecule or the like.

In the method using the known software that analyzes the structure of the molecule in order to calculate the structure descriptor, for example, the above-mentioned software called "RDKit" can be used.

Furthermore, as described above, various types of structure descriptors have been proposed so far. For example, in the "RDKit", 208 types of structure descriptors can be calculated for zero-dimensional to two-dimensional structure descriptors. Furthermore, in an example of the technology disclosed in this case, a three-dimensional structure descriptor calculated based on a three-dimensional structure of a molecule (compound) and a four-dimensional structure descriptor determined through interaction with other molecule such as interaction energy can be used.

In an example of the technology disclosed in this case, it is preferable to obtain a plurality of types of structure descriptors for each group of a plurality of molecules (specific molecule). That is, for example, in an example of the technology disclosed in this case, for example, it is preferable to obtain 208 types of zero-dimensional to two-dimensional structure descriptors using the above-described "RDKit" or the like for each specific molecule included in the specific molecule group.

Moreover, in an example of the technology disclosed in this case, all the plurality of types of obtained structure descriptors can be used to generate a model. However, it is preferable to select and use a structure descriptor that is considered to contribute to improve the accuracy of the model from among the plurality of types of structure descriptors. In other words, for example, in an example of the technology disclosed in this case, in the model generation process, it is preferable to specify the structure descriptor that contributes to improve the accuracy of the model from among the plurality of structure descriptors as a feature amount and generate a model based on the similarity and the feature amount.

The feature amount can be, for example, a structure descriptor that contributes to the accuracy of the model among the plurality of types of structure descriptors. In an example of the technology disclosed in this case, it is possible to further improve the accuracy of the generated model by generating the model based on of the structural similarity and the feature amount.

As a method for specifying (selecting) the feature amount that contributes to improve the accuracy of the model from the plurality of types of structure descriptors, for example, a method called "Boruta" can be used.

"Boruta" assumes a "false feature amount" that is considered not to contribute to improve the accuracy of the model using a machine learning method called random forest and verifies significance with respect to the "false feature amount" for each structure descriptor. Then, in "Boruta", a structure descriptor of which significance with respect to the "false feature amount" is specified as high, that is, a (significant) structure descriptor that contributes the accuracy of the model is specified.

Furthermore, for example, "Kursa MB, Rudnicki WR (2010). "Feature Selection with the Boruta Package." Journal of Statistical Software, 36 (11), 1-13. (http://www.jstatsoft.org/v36/ill/.)" describes details of "Boruta".

Furthermore, when "Boruta" selects the feature amount from the structure descriptor, for example, a threshold for the significance with respect to the "false feature amount" described above can be set, and a structure descriptor of which significance is higher than the threshold can be selected as a feature amount. For example, when the threshold is set to be lower, a large number of types of structure descriptors are selected as feature amounts, and when the threshold is set to be higher, a small number of structure descriptors that are particularly considered to largely affect the model are selected as feature amounts.

It is preferable to appropriately set the threshold (the number of feature amounts) for the significance to an appropriate value by performing verification or the like using training data (learning data) according to the type of the characteristic to be analyzed, the type of the model to be generated, or the like.

Furthermore, as the method for specifying (selecting) the feature amount that contributes to improve the accuracy of the model from among the plurality of types of structure descriptors, for example, a method called "Lasso regression" can be used.

For example, "Tibshirani, R., "Regression shrinkage and selection via the lasso", J. Roy. Statist. Soc. Ser. B, 58, pp. 267 to 288, 1996" describes details of "Lasso regression".

Moreover, in an example of the technology disclosed in this case, correlation analysis may be performed on the feature amount specified using "Boruta" or the like, and a model may be generated by excluding feature amounts having a strong correlation (similar to each other). In other words, in an example of the technology disclosed in this case, in the model generation process, it is preferable to specify the feature amounts correlated to each other by performing the correlation analysis on the plurality of feature amounts and not to use at least one of the feature amounts correlated to each other in order to generate a model.

In this way, in an example of the technology disclosed in this case, because the number of feature amounts (similar feature amounts) having the similar meaning can be reduced, over-training when the model is learned can be prevented. In other words, for example, in an example of the technology disclosed in this case, by reducing the number of explanatory variables when the model is generated by excluding the feature amounts having the strong correlation (similar to each other), it is possible to prevent over-training when the model is learned.

Furthermore, the correlation analysis of the feature amount can be performed using known software, a program created as needed, or the like.

In addition, in an example of the technology disclosed in this case, a relative error of a feature amount of another molecule included in the plurality of molecules with respect to a feature amount of one molecule included in the plurality of molecules may be specified, and analysis may be performed using an index using the similarity and the relative error. That is, for example, regarding the feature amount selected from the structure descriptor, a relative error of the feature amount of the non-specific molecule to be analyzed with respect to the feature amount of the specific molecule to be the reference may be obtained, and analysis may be performed using an index using the structural similarity and the relative error.

In other words, for example, in the model generation process, it is possible to specify the relative error of the feature amount of another molecule included in the plurality of molecules with respect to the feature amount of the one molecule included in the plurality of molecules and generate a model on the basis of the similarity and the relative error.

That is, for example, in an example of the technology disclosed in this case, the analysis can be performed using the index using the relative error of the feature amount of the non-specific molecule (Source molecule, candidate molecule) with respect to the feature amount of the specific molecule to be the reference (Query molecule). Furthermore, when the relative error is obtained, for example, it is preferable to use an average of the relative errors of the respective feature amounts (structure descriptor).

The average of the relative errors for the respective feature amounts can be calculated, for example, using the following equation.

[Expression 8]

$$E_{ave} = \frac{1}{n}\sum_{i=1}^{n}\frac{\left|x_i^s - x_i^q\right|}{\left|x_i^q\right|}$$

Here, in the equation described above, "$E_{ave}$" means an average of relative errors, "$x_i^s$" means a value of an i-th structure descriptor in a non-specific molecule (Source molecule), and "$x_i^q$" means a value of an i-th structure descriptor in a specific molecule (Query molecule) to be a reference. Furthermore, in the equation described above, "n" means the total number of the feature amounts (selected structure descriptor).

In the equation described above, for example, in a case where the value of the structure descriptor in the specific molecule (Query molecule) is "0", the structure descriptor is excluded from "$x_i^q$".

Furthermore, when the relative error of the feature amount is obtained, for example, it is preferable to consider importance of each feature amount by weighting each feature amount (setting weighting coefficient). In other words, for example, in the model generation process, it is preferable to set a weight for each of the plurality of feature amounts according to the degree of the contribution to improve the accuracy of the model and specify the relative error.

For example, the weighting coefficient of each feature amount can be set by appropriately performing adjustment (tuning) so as to improve the accuracy of the model.

Moreover, in an example of the technology disclosed in this case, analysis may be performed using an index using the average of the relative errors of the feature amounts described above and the structural similarity. As the index using the average of the relative errors of the feature amounts and the structural similarity, for example, an index indicated in the following equation can be used.

[Expression 9]

$$S_{new} = \alpha S_{DA} + (1-\alpha)(1-E_{ave})$$

Here, in the equation described above, "$S_{new}$" means an index using an average of relative errors of feature amounts and a structural similarity, "$S_{DA}$" means a structural similarity, "$E_{ave}$" means an average of relative errors, and "a" means a coefficient.

Furthermore, for example, the coefficient $\alpha$ can be set by appropriately adjusting (tuning) so as to improve the accuracy of the model and, for example, can be set to 1/2.

The relative error for each feature amount may be calculated, for example, using the following equation.

[Expression 10]

$$e_i = \min\left\{\left(\frac{\left|x_i^s - x_i^q\right|}{2\left|x_i^q\right|} + \frac{\left|x_i^q - x_i^s\right|}{2\left|x_i^s\right|}\right), 1\right\}$$

Here, in the equation described above, "$e_i$" means a relative error, "$x_i^s$" means a value of an i-th structure descriptor in a non-specific molecule (Source molecule), and "$x_i^q$" means a value of an i-th structure descriptor in a specific molecule (Query molecule) to be a reference. Furthermore, in the equation described above, min {A, B} means that a smaller one of A and B is selected.

In addition, in an example of the technology disclosed in this case, the analysis may be performed using the relative error calculated using the equation described above and the structural similarity. As the index using the relative error calculated using the equation described above and the structural similarity, for example, an index indicated in the following equation can be used.

[Expression 11]

$$S_{new} = \max\left\{\left(S_{DA} - \sum_{i=1}^{n} w_i e_i\right), 0\right\}$$

Here, "$S_{new}$" means an index using a relative error of a feature amount and a structural similarity, "$S_{DA}$" means a structural similarity, "$e_i$" means a relative error, "$w_i$" means a weight, and max {A, B} means that a larger one of A and B is selected.

Furthermore, in the equation described above, for example, in a case where "$S_{new}$" is equal to or less than zero, the value of "$S_{new}$" is set to zero.

<<Model Generation>>

In an example of the technology disclosed in this case, as described above, a model used to analyze a first molecule is generated on the basis of a similarity between respective structures of a plurality of molecules and a structure descriptor that is an index specified based on the structure of each of the plurality of molecules. More specifically, for example, a model used to analyze a non-specific molecule is generated based on a structural similarity between specific molecules included in a specific molecule group and a structure descriptor in the specific molecule included in the specific molecule group.

In an example of the technology disclosed in this case, the generated model is not particularly limited as long as the model can analyze the first molecule, and can be appropriately selected depending on a purpose. The generated model includes, for example, a model (learned model) that can be generated through machine learning, a model (index) represented by a mathematical formula, or the like.

As the model that can be generated through machine learning, for example, a model (multiple regression model) that performs regression prediction on a physical property value, a model (class classifier) that classifies molecules into classes, or the like can be preferably used. In other words, for example, in an example of the technology disclosed in this case, it is preferable that the model be a prediction model that predicts a characteristic value of the first molecule or a classification model that classifies the first molecule based on the characteristic value.

In this way, in an example of the technology disclosed in this case, based on the molecule (specific molecule) of which a target characteristic value is known, using the prediction model or the classification model, it is possible to search for and accurately narrow a molecule having a physical property value close to that of the above molecule.

Here, in an example of the technology disclosed in this case, when the model is generated based on the structural similarity and the structure descriptor (feature amount), for example, "PyCaret" that is a Python library regarding automatic machine learning (AutoML) can be used.

In "PyCaret", for example, by inputting learning data and setting the characteristics to be a target of prediction or the like as an objective variable and the structural similarity and the structure descriptor (feature amount) as explanatory variables, it is possible to collectively generate a plurality of type of models. Furthermore, in a case where a model is generated based on the structural similarity and the relative error, for example, by performing calculation using "PyCaret" by setting the structural similarity and the relative error as explanatory variables and the characteristic as an objective variable, it is possible to generate the model.

Note that, for example, "PyCaret.org. PyCaret, July 2020. URL(https://pycaret.org/about). PyCaret version 2.3." describes details of "PyCaret".

In an example of the technology disclosed in this case, when accuracy of the generated model is verified, for example, a method called "k-fold cross validation" can be used. In "k-fold cross validation", training data (learning data) is divided into k groups, and a model learned by using "k - 1" groups of the k groups is verified according to data of the remaining one group. Then, in "k-fold cross validation", this verification is repeated k times as changing a group used for learning and verification so as to obtain the average of the accuracy of the model or the like.

"k-fold cross validation" can be performed, for example, by "PyCaret" described above, and in a case where the classification model (class classifier) is evaluated, it is possible to obtain an index regarding the accuracy of the model such as "Accuracy", "AUC", or "Recall". In addition, for example, in a case where the prediction model (multiple regression model) is evaluated, it is possible to obtain an index such as "MAE", "MSE", "RMSE", or "R2 (determination coefficient)".

Furthermore, in an example of the technology disclosed in this case, for example, when the classification model is evaluated, the evaluation can be performed as paying attention to the index such as "Accuracy" or "AUC", and in particular, it is preferable to pay attention to "AUC" for a class classifier that performs binary classification. Furthermore, when the prediction model is evaluated, for example, it is preferable to perform evaluation while paying attention to "R2 (determination coefficient)".

In an example of the technology disclosed in this case, when a model is generated, it is preferable to verify the accuracy of the model and update the model until the accuracy becomes equal to or higher than a predetermined value. In other words, for example, in an example of the technology disclosed in this case, in the model generation process, it is preferable to specify, by the model, analysis accuracy when the analysis for verification using the specific molecule is performed, and to update the model until the analysis accuracy becomes equal to or higher than a predetermined value by changing at least one of the model generation method and a parameter.

The analysis accuracy can be specified by the model, for example, using "k-fold cross validation" described above. More specifically, for example, by performing "k-fold cross validation" using the data of the specific molecule group as training data, it is possible to specify the analysis accuracy when the analysis is performed to perform the verification using the specific molecule.

Furthermore, the model generation method can be changed, for example, by changing the type of the model to be generated using "PyCaret" described above. In this way, with "PyCaret" described above, it is possible to collectively generate the plurality of types of models. Therefore, by selecting the model with high accuracy from among the generated models, it is possible to improve the accuracy of the model.

On the other hand, for example, the parameter of the model may be changed by appropriately changing and adjusting a value of the parameter by a user, or in a case where a value of the parameter is randomly changed and the accuracy of the model is improved, the parameter of the model may be changed by adopting the value of the parameter.

<Analysis of First Molecule (Non-Specific Molecule)>

In an example of the technology disclosed in this case, as described above, by analyzing the first molecule (non-specific molecule) using the generated model, for example, it is possible to select a first molecule of which a target characteristic has a preferable value from among a large number of first molecules.

When the first molecule is analyzed using the generated model, for example, by inputting data of the first molecule into the generated model, it is possible to perform analysis such as prediction of a characteristic value of the first molecule or classification of a non-specific molecule. In other words, for example, in an example of the technology disclosed in this case, it is preferable to analyze the first molecule by inputting the data of the first molecule into the model generated in the model generation process.

Furthermore, in an example of the technology disclosed in this case, analysis using another model, in addition to the analysis according to the model based on the structural similarity and the structure descriptor (feature amount), may be performed. More specifically, for example, in addition to the model based on the structural similarity and the structure descriptor, a model based on only the structural similarity and a model based on only the structure descriptor (feature amount) are further generated, and a model with high accuracy may be selected from among these models and used.

In this way, for example, even in a case where accuracy of another model is higher, accurate analysis can be performed without exception regardless of the analysis target and the type of the model.

Note that, in an example of the technology disclosed in this case, a process for analyzing a non-specific molecule using a generated model may be referred to as an "analysis process".

<Other Processes>

Other processes are not particularly limited and can be appropriately selected depending on a purpose.

(Information Processing Method)

An information processing method disclosed in this case that is an information processing method for analyzing a first molecule different from a plurality of molecules based on characteristic data of each of the plurality of molecules with a computer, includes a model generation process for generating a model used to analyze the first molecule based ona similarity between respective structures of the plurality of molecules, anda structure descriptor that is an index specified based on the structure of each of the plurality of molecules.

For example, the information processing method disclosed in this case can be performed similarly to the model generation process in the information processing program disclosed in this case, for example. Furthermore, a preferred mode of the information processing method disclosed in this case can be, for example, similar to a preferred mode of the model generation process in the information processing program disclosed in this case.

The information processing method disclosed in this case can be, for example, a method for performing the model generation process using a computer.

(Information Processing Apparatus)

An information processing apparatus disclosed in this case that is an information processing apparatus that analyzes a first molecule different from a plurality of molecules based on characteristic data of each of the plurality of molecules, includes a model generation unit that generates a model used to analyze the first molecule based on a similarity between respective structures of the plurality of molecules, and a structure descriptor that is an index specified on the basis of the structure of each of the plurality of molecules.

The information processing apparatus disclosed in this case includes the model generation unit and further includes other units (unit) as needed.

The information processing apparatus includes, for example, a memory and a processor, and further includes other units as needed. As the processor, a processor that is coupled to the memory can be preferably used so as to perform the model generation process.

The processor can be, for example, a central processing unit (CPU), a graphics processing unit (GPU), or a combination thereof.

As described above, the information processing apparatus disclosed in this case can be, for example, a device (computer) that executes the information processing program disclosed in this case. Therefore, a preferred mode of the information processing apparatus disclosed in this case can be similar to a preferred mode of the information processing program disclosed in this case.

(Computer-Readable Recording Medium)

A computer-readable recording medium disclosed in this case records the information processing program disclosed in this case.

The computer-readable recording medium disclosed in this case is not particularly limited and can be appropriately selected according to a purpose. Examples of the computer-readable recording medium include a built-in hard disk, an externally attached hard disk, a CD-ROM, a DVD-ROM, an MO disk, a USB memory, and the like, for example.

Furthermore, the computer-readable recording medium disclosed in this case may be a plurality of recording media in which the information processing program disclosed in this case is divided and recorded for each of arbitrary pieces of processing.

Hereinafter, an example of the technology disclosed in this case will be described in more detail using configuration examples of the device, flowcharts, and the like.

FIG. 18 illustrates a hardware structure example of an information processing apparatus disclosed in this case.

In an information processing apparatus 100, for example, a control unit 101, a main storage device 102, an auxiliary storage device 103, an input output (I/O) interface 104, a communication interface 105, an input device 106, an output device 107, and a display device 108 are connected to one another via a system bus 109.

The control unit 101 performs arithmetic operations (four arithmetic operations, comparison operations, arithmetic operations for annealing method, or the like), hardware and software operation control, and the like. The control unit 101 may be, for example, a central processing unit (CPU), a part of the annealing machine used for the annealing method, or a combination thereof.

The control unit 101 realizes various functions, for example, by executing a program (for example, information processing program disclosed in this case or the like) read in the main storage device 102 or the like.

Processing executed by the model generation unit in the information processing apparatus disclosed in this case can be executed, for example, by the control unit 101.

The main storage device 102 stores various programs and data or the like needed for executing various programs. As the main storage device 102, for example, a device having at least one of a read only memory (ROM) and a random access memory (RAM) can be used.

For example, the ROM stores various programs such as a basic input/output system (BIOS) or the like. Furthermore, the ROM is not particularly limited and can be appropriately selected according to a purpose. For example, a mask ROM, a programmable ROM (PROM), or the like can be exemplified.

The RAM functions, for example, as a work range expanded when various programs stored in the ROM, the auxiliary storage device 103, or the like are executed by the control unit 101. The RAM is not particularly limited and can be appropriately selected according to a purpose. For example, a dynamic random access memory (DRAM), a static random access memory (SRAM), or the like can be exemplified.

The auxiliary storage device 103 is not particularly limited as long as the device can store various types of information and can be appropriately selected according to a purpose. For example, a solid state drive (SSD), a hard disk drive (HDD), or the like can be exemplified. Furthermore, the auxiliary storage device 103 may be a portable storage device such as a CD drive, a DVD drive, or a Blu-ray (registered trademark) disc (BD) drive.

Furthermore, the information processing program disclosed in this case is, for example, stored in the auxiliary storage device 103, loaded into the RAM (main memory) of the main storage device 102, and executed by the control unit 101.

The I/O interface 104 is an interface used to connect various external devices. The I/O interface 104 can input/output data to/from, for example, a compact disc ROM (CD-ROM), a digital versatile disk ROM (DVD-ROM), a magneto-optical disk (MO disk), a universal serial bus (USB) memory (USB flash drive), or the like.

The communication interface 105 is not particularly limited, and a known communication interface can be appropriately used. For example, a communication device using wireless or wired communication or the like can be exemplified.

The input device 106 is not particularly limited as long as the device can receive input of various requests and information to the information processing apparatus 100, and a known device can be appropriately used. For example, a keyboard, a mouse, a touch panel, a microphone, or the like can be exemplified. Furthermore, in a case where the input device 106 is a touch panel (touch display), the input device 106 can also serve as the display device 108.

The output device 107 is not particularly limited, and a known device can be appropriately used. For example, a printer or the like can be exemplified.

The display device 108 is not particularly limited, and a known device can be appropriately used. For example, a liquid crystal display, an organic EL display, or the like can be exemplified.

FIG. 19 is another hardware structure example of the information processing apparatus disclosed in this case.

In the example illustrated in FIG. 19, the information processing apparatus 100 is divided into a computer 200 that executes processing for calculating a structure descriptor or the like and an annealing machine 300 that executes processing for obtaining a maximum independent set of a conflict graph or the like in order to calculate a structural similarity. Furthermore, in the example illustrated in FIG. 19, the computer 200 and the annealing machine 300 of the information processing apparatus 100 are connected via a network 400.

In the example illustrated in FIG. 19, for example, as a control unit 101a of the computer 200, a CPU or the like can be used, and as a control unit 101b of the annealing machine 300, a device specialized in the annealing method (annealing) can be used.

FIG. 20 illustrates a functional structure example of the information processing apparatus disclosed in this case.

As illustrated in FIG. 20, the information processing apparatus 100 includes a communication function unit 120, an input function unit 130, an output function unit 140, a display function unit 150, a storage function unit 160, and a control function unit 170.

The communication function unit 120 transmits and receives, for example, various types of data to and from an external device. The communication function unit 120 may receive, for example, characteristic data of each of the plurality of molecules, data of the first molecule, or the like from an external device.

The input function unit 130 receives, for example, various instructions to the information processing apparatus 100. Furthermore, the input function unit 130 may receive, for example, inputs of the characteristic data of each of the plurality of molecules, the data of the first molecule, or the like.

The output function unit 140 prints and outputs, for example, data of an analysis result or the like.

The display function unit 150 displays, for example, the data of the analysis result or the like on a display.

The storage function unit 160 stores, for example, various programs, the characteristic data of each of the plurality of molecules, the data of the first molecule, the data of the analysis result, or the like.

The control function unit 170 includes a model generation unit 171 and an analysis unit 174.

For example, the model generation unit 171 executes processing for generating a model used to analyze the first molecule on the basis of a similarity between respective structures of the plurality of molecules and a structure descriptor that is an index specified on the basis of the structure of each of the plurality of molecules.

The analysis unit 174 executes, for example, processing for analyzing the first molecule (non-specific molecule) according to the model generated by the model generation unit 171.

Furthermore, the model generation unit 171 includes a similarity specification unit 172 and a structure descriptor specification unit 173.

The similarity specification unit 172 executes, for example, processing for specifying (calculating) the similarity between the respective structures of the plurality of molecules. The structure descriptor specification unit 173 executes, for example, processing for specifying the structure descriptor that is the index specified based on the structure of each of the plurality of molecules, selecting the feature amount from the structure descriptor, or the like.

FIG. 21 illustrates an example of a flowchart when a model used to analyze a non-specific molecule is generated, in an example of the technology disclosed in this case.

First, the model generation unit 171 receives input of information regarding a structure and information regarding a characteristic value of a specific molecule (S201). In other words, for example, in S201, the model generation unit 171 acquires, for example, the information regarding the structure and the information regarding the characteristic value of each specific molecule from the characteristic data of each of the plurality of molecules (data of specific molecule group).

Next, the model generation unit 171 obtains a structural similarity between the specific molecules based on the information regarding the structure of the specific molecule (S202). More specifically, in S202, for example, the model generation unit 171 specifies the similarity between the respective structures of the plurality of molecules by searching for the maximum independent set of the conflict graph or performing analysis with "RDKit".

Subsequently, the model generation unit 171 obtains a structure descriptor of the specific molecule based on the information regarding the structure of the specific molecule (S203). More specifically, in S203, for example, the model generation unit 171 specifies the structure descriptor that is the index specified based on the structure of each of the plurality of molecules by performing the analysis with "RDKit".

Next, the model generation unit 171 specifies a structure descriptor that contributes to improve the accuracy of the model from the plurality of structure descriptors as a feature amount (S204). More specifically, in S204, for example, the model generation unit 171 specifies a feature amount as assuming that a structure descriptor that is specified to have high significance with respect to the "false feature amount" using "Boruta" is a (significant) structure descriptor that contributes the accuracy of the model.

Then, the model generation unit 171 generates a model used for analysis through machine learning based on the structural similarity, the feature amount, and the characteristic value (S205). More specifically, in S205, for example, the model generation unit 171 generates a prediction model or a classification model as setting the structural similarity and the feature amount as explanatory variables and the characteristic value as an objective variable using "PyCaret".

Next, the model generation unit 171 performs analysis for verification using a specific molecule and specifies analysis accuracy (S206). More specifically, in S206, for example, the model generation unit 171 performs "k-fold cross validation" regarding the characteristic data of each of the plurality of molecules for the generated model so as to verify the accuracy of the model.

Subsequently, the model generation unit 171 determines whether or not the analysis accuracy is equal to or higher than a predetermined value (S207). More specifically, in S207, in a case where the analysis accuracy specified in S206 is lower than the predetermined value, the model generation unit 171 proceeds the processing to S208, and in a case where the analysis accuracy specified in S206 is equal to or higher than the predetermined value, the model generation unit 171 ends the processing.

Next, the model generation unit 171 changes at least one of the model generation method and the parameter (S208). More specifically, in S208, for example, the model generation unit 171 selects a model with high accuracy from among the generated models, changes a value of the parameter of the model, and returns the processing to S205.

In an example illustrated in FIG. 21, the model used for analysis is generated based on the similarity between the respective structures of the plurality of molecules and the feature amount selected from the structure descriptor that is the index specified based on the structure of each of the plurality of molecules, and the model is updated until the analysis accuracy of the model reaches a value equal to or higher than the predetermined value. Therefore, in the example illustrated in FIG. 21, the model with higher accuracy can be generated.

FIG. 22 illustrates another example of the flowchart when a model used to analyze a non-specific molecule is generated, in an example of the technology disclosed in this case. Note that, because S301 and S302 in FIG. 22 respectively correspond to S201 and S202 in FIG. 21, S304 and S305 in FIG. 22 respectively correspond to S203 and S204 in FIG. 21, and S308 to S310 in FIG. 22 respectively correspond to S206 to S208 in FIG. 21, description thereof will be omitted.

In the example illustrated in FIG. 22, in S303, the model generation unit 171 specifies a structural similarity that contributes to improve the accuracy of the model from the plurality of structural similarities. More specifically, in S303, for example, the model generation unit 171 specifies the (significant) structural similarity that contributes to the accuracy of the model using "Boruta" for the structural similarities obtained by calculating all the patterns of combinations of specific molecules.

Furthermore, in S306, the model generation unit 171 obtains a relative error of a feature amount of another molecule with respect to the feature amount of the molecule to be a reference. More specifically, in S306, for example, the model generation unit 171 obtains an average of relative errors of a feature amount of a non-specific molecule (Source molecule, candidate molecule) with respect to a feature amount of a specific molecule (Query molecule) to be the reference.

Then, in S307, the model generation unit 171 generates a model for analysis through machine learning based on the structural similarity, the relative error of the feature amount, and the characteristic value. More specifically, in S307, for example, the model generation unit 171 generates a prediction model or a classification model as setting the structural similarity and the average of the relative errors of the feature amount as explanatory variables and the characteristic value as an objective variable using "PyCaret".

In this way, in the example illustrated in FIG. 22, the structural similarity that contributes to improve the accuracy of the model is obtained, and the model is generated using the relative error of the similarity, so that the model with higher accuracy can be generated.

FIG. 23 illustrates an example of a flowchart when a non-specific molecule is analyzed using a generated model in an example of the technology disclosed in this case.

First, the analysis unit 174 receives input of information regarding a structure of a non-specific molecule (S401). In other words, for example, in S401, the analysis unit 174 acquires information regarding a structure of each first molecule from data including the plurality of first molecules (non-specific molecule).

Next, the analysis unit 174 obtains a structural similarity based on the information regarding the structure of the non-specific molecule (S402). More specifically, in S402, for example, the analysis unit 174 specifies a structural similarity between the specific molecule and the non-specific molecule (first molecule) and the structural similarity between the non-specific molecules (first molecule) by searching for the maximum independent set of the conflict graph or performing analysis with "RDKit".

Subsequently, the analysis unit 174 obtains a structure descriptor of the non-specific molecule corresponding to the feature amount based on the information regarding the structure of the non-specific molecule (S403). More specifically, in S403, for example, the analysis unit 174 specifies the value of the feature amount of the non-specific molecule by analyzing the structure descriptor of the non-specific molecule (first molecule) that is the same type as the feature amount specified when the model is generated, with "RDKit".

Then, the analysis unit 174 inputs the information regarding the structural similarity and the feature amount of the non-specific molecule into the generated model and analyzes the non-specific molecule (S404). More specifically, in S404, for example, the analysis unit 174 analyzes the characteristic value of the non-specific molecule (first molecule) by inputting the information regarding the structural similarity and the feature amount of the non-specific molecule (first molecule) into the prediction model or the classification model generated with "PyCaret". Furthermore, the analysis unit 174 may output an analysis result to a display or the like.

Then, when the analysis of the non-specific molecule (first molecule) is completed, the analysis unit 174 ends the processing.

In this way, in the example illustrated in FIG. 23, because the non-specific molecule (first molecule) is analyzed using the model based on both of the structural similarity and the structure descriptor, it is possible to perform the analysis with higher accuracy.

Furthermore, in FIGs. 21 to 23, the flow of the processing in an example of the technology disclosed in this case has been described according to a specific order. However, in the technology disclosed in this case, it is possible to appropriately switch an order of individual steps in a technically possible range.

Furthermore, in the technology disclosed in this case, a plurality of steps may be collectively performed in a technically possible range. For example, in the example illustrated in FIG. 21, because S202 (calculation of structural similarity) is processing independent from S203 and S204 (calculation of structure descriptor and specification of feature amount), both processing may be executed in parallel or S203 and S204 may be executed prior to S202.

Examples of the annealing method and the annealing machine will be described below.

The annealing method is a method for probabilistically obtaining a solution using superposition of random number values and quantum bits. The following describes a problem of minimizing a value of an evaluation function to be optimized as an example. The value of the evaluation function is referred to as energy. Furthermore, in a case where the value of the evaluation function is maximized, the sign of the evaluation function only needs to be changed.

First, a process is started from an initial state in which one of discrete values is assigned to each variable. With respect to a current state (combination of variable values), a state close to the current state (for example, a state in which only one variable is changed) is selected, and a state transition therebetween is considered. An energy change with respect to the state transition is calculated. Depending on the value, it is probabilistically determined whether to adopt the state transition to change the state or not to adopt the state transition to keep the original state. In a case where an adoption probability in a case where the energy decreases is selected to be larger than that in a case where the energy increases, it can be expected that a state change will occur in a direction that the energy decreases on average, and that a state transition will occur to a more appropriate state over time. Therefore, there is a possibility that an optimum solution or an approximate solution that gives energy close to the optimum value can be obtained finally.

If this is adopted in a case where the energy decreases deterministically and is not adopted in a case where the energy increases, the energy change decreases monotonically in a broad sense with respect to time, but no further change occurs when reaching a local solution. As described above, since there are a very large number of local solutions in the discrete optimization problem, a state is almost certainly caught in a local solution that is not so close to an optimum value. Therefore, when the discrete optimization problem is solved, it is important to determine probabilistically whether or not to adopt the state.

In the annealing method, it has been proved that, by determining an adoption (permissible) probability of a state transition as follows, a state reaches an optimum solution in the limit of infinite time (iteration count).

Hereinafter, a method for obtaining an optimum solution using the annealing method will be described step by step.

(1) For an energy change (energy reduction) value (- ΔE) due to a state transition, a permissible probability p of the state transition is determined by any one of the following functions f ().

[Expression 12]

$$p(\Delta E,T)=f(-\Delta E/T) \quad \text{(EQUATION 1-1)}$$

[Expression 13]

$$f_{metro}(x)=\min(1,e^{x}) \quad \text{(METROPOLIS METHOD) (EQUATION 1-2)}$$

[Expression 14]

$$f_{Gibbs}(x)=\frac{1}{1+e^{-x}} \quad \text{(GIBBS METHOD)(EQUATION 1-3)}$$

Here, T represents a parameter called a temperature value and can be changed as follows, for example.

(2) The temperature value T is logarithmically reduced with respect to an iteration count t as represented by the following equation.

[Expression 15]

$$T=\frac{T_0\log(c)}{\log(t+c)} \quad \text{(EQUATION 2)}$$

Here, $T_0$ is an initial temperature value, and is desirably a sufficiently large value depending on a problem.

In a case where the permissible probability represented by the equation in (1) is used, if a state reaches a steady state after sufficient iterations, an occupation probability of each state follows a Boltzmann distribution for a thermal equilibrium state in thermodynamics.

Then, when the temperature is gradually lowered from a high temperature, an occupation probability of a low energy state increases. Therefore, it is considered that the low energy state is obtained when the temperature is sufficiently lowered. Since this state is very similar to a state change caused when a material is annealed, this method is referred to as the annealing method (or pseudo-annealing method). Note that probabilistic occurrence of a state transition that increases energy corresponds to thermal excitation in the physics.

FIG. 24 illustrates an example of a functional configuration of an annealing machine that performs the annealing method. However, in the following description, a case of generating a plurality of state transition candidates is also described. However, a basic annealing method generates one transition candidate at a time.

The annealing machine 300 includes a state holding unit 111 that holds a current state S (plurality of state variable values). Furthermore, the annealing machine 300 includes an energy calculation unit 112 that calculates an energy change value {- ΔEi} of each state transition in a case where a state transition from the current state S occurs due to a change in any one of the plurality of state variable values. Moreover, the annealing machine 300 includes a temperature control unit 113 that controls the temperature value T, and a transition control unit 114 that controls a state change. Note that, the annealing machine 300 can be a part of the information processing apparatus 100 described above.

The transition control unit 114 probabilistically determines whether or not to accept any one of a plurality of state transitions according to a relative relationship between the energy change value {- ΔEi} and thermal excitation energy, based on the temperature value T, the energy change value {- ΔEi}, and a random number value.

Here, the transition control unit 114 includes a candidate generation unit 114a that generates a state transition candidate, and an availability determination unit 114b to probabilistically determine whether or not to permit a state transition for each candidate based on the energy change value {- ΔEi} and the temperature value T. Moreover, the transition control unit 114 includes a transition determination unit 114c that determines a candidate to be adopted from the candidates that have been permitted, and a random number generation unit 114d that generates a random variable.

An operation of the annealing machine 300 in one iteration is as follows.

First, the candidate generation unit 114a generates one or a plurality of state transition candidates (candidate number {Ni}) from the current state S held in the state holding unit 111 to a next state. Next, the energy calculation unit 112 calculates the energy change value {- ΔEi} for each state transition listed as a candidate by using the current state S and the state transition candidates. The availability determination unit 114b permits a state transition with a permissible probability of the above equation (1) according to the energy change value {- ΔEi} of each state transition using the temperature value T generated by the temperature control unit 113 and the random variable (random number value) generated by the random number generation unit 114d.

Then, the availability determination unit 114b outputs availability {fi} of each state transition. In a case where there is a plurality of permitted state transitions, the transition determination unit 114c randomly selects one of the permitted state transitions using a random number value. Then, the transition determination unit 114c outputs a transition number N and transition availability f of the selected state transition. In a case where there is a permitted state transition, a state variable value stored in the state holding unit 111 is updated according to the adopted state transition.

Starting from an initial state, the above-described iteration is repeated while the temperature value is lowered by the temperature control unit 113. When a completion determination condition such as reaching a certain iteration count or energy falling below a certain value is satisfied, the operation is completed. An answer output by the annealing machine 300 is a state when the operation is completed.

The annealing machine 300 illustrated in FIG. 24 may be implemented by using, for example, a semiconductor integrated circuit. For example, the transition control unit 114 may include a random number generation circuit that functions as the random number generation unit 114d, a comparison circuit that functions as at least a part of the availability determination unit 114b, a noise table to be described later, or the like.

Regarding the transition control unit 114 illustrated in FIG. 24, details of a mechanism that permits a state transition at a permissible probability represented in the equation (1) will be further described.

A circuit that outputs one at the permissible probability p and outputs zero at a permissible probability (1 - p) can be achieved by inputting the permissible probability p for input A and a uniform random number that takes a value of a section [0, 1) for input B in a comparator that has the two inputs A and B, and outputs one when A > B is satisfied and outputs zero when A < B is satisfied. Therefore, if the value of the permissible probability p calculated on the basis of the energy change value and the temperature value T using the equation (1) is input to the input A of this comparator, the above-described function can be achieved.

In other words, for example, with a circuit that outputs one when f (ΔE/T) is larger than u, in which f is a function used in the equation (1), and u is a uniform random number that takes a value of the section [0, 1), the above-described function can be achieved.

Furthermore, the same function as the above-described function can also be achieved by making the following modification.

Applying the same monotonically increasing function to two numbers does not change a magnitude relationship. Therefore, an output is not changed even if the same monotonically increasing function is applied to two inputs of the comparator. If an inverse function $f^{-1}$ of f is adopted as this monotonically increasing function, it can be seen that a circuit that outputs one when - ΔE/T is larger than $f^{-1}$ (u) can be adopted. Moreover, since the temperature value T is positive, it can be seen that a circuit that outputs one when - ΔE is larger than $Tf^{-1}$ (u) may be adopted.

The transition control unit 114 in FIG. 24 may include a noise table that is a conversion table that realizes the inverse function $f^{-1}$ (u), and outputs a value of a next function with respect to an input that is a discretized section [0, 1).

[Expression 16]

$$f_{metro}^{-1}(u) = \log(u) \qquad \text{(EQUATION 3-1)}$$

[Expression 17]

$$f_{Gibbs}^{-1}(u) = \log\left(\frac{u}{1-u}\right) \qquad \text{(EQUATION 3-2)}$$

FIG. 25 is a diagram illustrating an example of an operation flow of the transition control unit 114. The operation flow illustrated in FIG. 25 includes a step of selecting one state transition as a candidate (S0001), a step of determining availability of the state transition by comparing an energy change value for the state transition with a product of a temperature value and a random number value (S0002), and a step of adopting the state transition when the state transition is available, and not adopting the state transition when the state transition is not available (S0003).

[Embodiment]

Hereinafter, specific embodiment of the present invention and comparative examples with respect to the present invention will be described. Note that the present invention is not limited to these embodiments.

(First Embodiment)

As a first embodiment, using an example of the information processing apparatus disclosed in this case, a model is generated, and accuracy of the generated model is verified. In the first embodiment, an information processing apparatus that has a hardware structure as illustrated in FIG. 19 and a functional configuration as illustrated in FIG. 20 is used. Then, in the first embodiment, according to a flow illustrated in the flowcharts in FIGs. 21 and 23, the model is generated, and the accuracy of the model is verified.

Specifically, for example, in the first embodiment, for 32 molecules of which biological activities are known (16 Actives and 16 Inactives), 25 pieces of data are used as training data (learning data), and seven pieces of data are used as test data. Furthermore, as the 32 molecules of which the biological activities are known, 32 molecules randomly extracted from "AID 1006 (https://pubchem.ncbi.nlm.nih.gov/bioassay/1006)" are used.

In the first embodiment, in order to verify the accuracy of the model, 25 molecules of the 32 molecules of which the biological activities are known are treated as specific molecules (characteristic data of each of plurality of molecules), seven molecules are assumed as non-specific molecules (first molecule) and analyzed, and an analysis result of the seven molecules is compared with actual biological activities of the seven molecules. That is, for example, in the first embodiment, a binary classification model (class classifier) that performs classification depending on whether the biological activity is "Active" or "Inactive" is generated, and its accuracy is verified.

First, in the first embodiment, a molecule having the best biological activity value of the 25 pieces of training data is set as a reference molecule of the structural similarity, and a structural similarity of another molecule with respect to the reference molecule (similarity in one-to-many relationship) is obtained. Specifically, for example, as the reference molecule of the structural similarity, "PubChem CID603597 (https://pubchem.ncbi.nlm.nih.gov/compound/603597)" is selected.

Furthermore, the structural similarity is calculated by searching for the maximum independent set of the conflict graph using the digital annealer (registered trademark). Furthermore, when the maximum independent set of the conflict graph is searched, a node of the conflict graph is set as a combination of two atoms having the same atom type subdivided from the elemental species based on an atom type of a GAFF.

Moreover, in the first embodiment, 208 types of structure descriptors (from zero-dimensional to two-dimensional) for each of the 32 molecules are calculated using "RDKit".

Subsequently, in the first embodiment, nine structure descriptors that contribute to accuracy of classification are specified from the 208 types of structure descriptors as feature amounts, using "Boruta".

In the first embodiment, the nine structure descriptors selected as the feature amounts are as follows.

- MolWt
- HeavyAtomMolWt

- ExactMolWt
- BCUT2D_MWLOW
- BCUT2D_MRLOW
- Kappa2
- SlogP_VSA3
- SlogP_VSA5
- NumHeteroatoms

Furthermore, structure descriptors, of which meanings are clear, of the nine structure descriptors selected as the feature amounts described above are as follows.

- MolWt: Average molecular weight
- HeavyAtomMolWt: Molecular weight excluding hydrogen atoms
- ExactMolWt: Exact molecular weight
- SlogP_VSA3 and SlogP_VSA5: Means the sum of a surface area of an atom having an atom component of LogP that falls within a predetermined range in a molecule (partial surface area of molecule) and represents SlogP_VSA1 (sum of surface area of hydrophilic atom) to SlogP_VSA12 (sum of surface area of hydrophobic atom).
- NumHeteroatoms: Number of heteroatoms

Subsequently, in the first embodiment, a classification model (class classifier) is generated based on the structural similarity and the nine feature amounts using "PyCaret". Furthermore, in the first embodiment, the plurality of types of classification models is collectively generated with "PyCaret", and the classification model with high accuracy is selected from among the generated models and used.

FIG. 26 illustrates an example of a relationship between the type of the classification model generated in the first embodiment and an index of accuracy of each classification model. As illustrated in FIG. 26, when the indexes of the accuracy of the respective classification models (Model) are compared, "Extra Trees Classifier" has high values of "Accuracy" and "AUC" that are important when evaluating the classification model. Therefore, in the first embodiment, "Extra Trees Classifier" is used as a classification model.

Note that, for example, "P. Geurts, D. Ernst., and L. Wehenkel, "Extremely randomized trees", Machine Learning, 63(1), 3 to 42, 2006." discloses details of "Extra Trees Classifier".

Then, in the first embodiment, regarding the generated classification model, the accuracy of the classification model is verified by performing "k-fold cross validation (k = 10)" using the 25 pieces of training data.

Furthermore, in order to compare the accuracy of the classification model, in the method according to the first embodiment described above, the classification model is generated on the basis of only the structural similarity (without executing S203 and S204 in FIG. 21). Then, regarding the classification model based on only the structural similarity, accuracy is verified and compared by performing "k-fold cross validation (k = 10)" using the training data.

Moreover, in order to compare the accuracy of the classification model in the method according to the first embodiment described above, the classification model is generated based on only the structure descriptor (nine feature amounts) (without executing S202 in FIG. 21). Then, regarding the classification model based on only the structure descriptor (nine feature amounts), accuracy is verified and compared by performing "k-fold cross validation (k = 10)" using the training data.

FIG. 27 illustrates an example of a result of "k-fold cross validation (k = 10)" in the classification model generated in the first embodiment.

FIG. 28 illustrates an example of a result of "k-fold cross validation (k = 10)" in the classification model generated based on only the structural similarity as an example corresponding to the first embodiment.

FIG. 29 illustrates an example of a result of "k-fold cross validation (k = 10)" in the classification model generated based on only the structure descriptor (nine feature amounts) as an example corresponding to the first embodiment.

As illustrated in FIG. 27, for the classification model generated in the first embodiment, "Accuracy" representing accuracy of classification is "0.85 (85%)". On the other hand, as illustrated in FIGs. 28 and 29, "Accuracy" of the classification model generated based on only the structural similarity is "0.50 (50%)", and "Accuracy" of the classification model generated based on only the structure descriptor (nine feature amounts) is "0.80 (80%)".

In this way, in the first embodiment, it can be verified that the accuracy of the prediction model based on the structural similarity and the structure descriptor (nine feature amounts) is higher than accuracy of other classification models.

Moreover, in the first embodiment, the biological activity of the seven pieces of test data is assumed to be unknown, and classification is performed using the classification model generated based on the structural similarity and the structure descriptor (nine feature amounts).

FIG. 30 illustrates an example of a result of classification regarding seven pieces of test data of which biological activity is assumed to be unknown, using the classification model generated in the first embodiment. Furthermore, FIG. 31 illustrates an example of a result of the classification regarding the seven pieces of test data of which the biological activity is assumed to be unknown, using the classification model generated based on only the structural similarity, as an example corresponding to the first embodiment.

In FIGs. 30 and 31, in "Extra Trees Classifier Confusion Matrix", the vertical axis means a correct biological activity of test data, and the horizontal axis means a biological activity classified with the classification model in the test data. Furthermore, in FIGs. 30 and 31, "1" means "Active (with biological activity)", and "0" means "Inactive (no biological activity)".

Therefore, in FIGs. 30 and 31, in a case where it is possible to correctly perform classification with the classification model, the test data is classified into the upper left or lower right of "Extra Trees Classifier Confusion Matrix". On the other hand, in FIGs. 30 and 31, in a case where wrong classification is performed with the classification model, the test data is classified into the lower left or upper right of "Extra Trees Classifier Confusion Matrix".

As illustrated in FIG. 30, with the classification model in the first embodiment generated based on the structural similarity and the structure descriptor (nine feature amounts), all of the seven pieces of test data can be correctly classified (accuracy 100%). On the other hand, as illustrated in FIG. 31, with the classification model generated based on only the structural similarity, four of seven pieces of test data can be correctly classified, and the accuracy of the classification is 57%.

In this way, in the first embodiment, it can be confirmed that a molecule of which a biological activity is unknown can be classified (classified) with high accuracy with the prediction model based on the structural similarity and the structure descriptor (nine feature amounts).

(Second Embodiment)

In a second embodiment, analysis is performed as in the first embodiment, except that, in the first embodiment described above, the number of feature amounts is reduced from nine to seven through correlation analysis, the average of the relative errors regarding the feature amount is obtained, and the classification model is generated based on the average of the relative errors and the structural similarity.

Specifically, for example, in the second embodiment, a classification model is generated by specifying feature amounts having a strong correlation (similar to each other) by performing the correlation analysis regarding the nine feature amounts and without using some of the feature amounts having the strong correlation to generate the classification model.

When the correlation analysis is performed on the nine feature amounts in the second embodiment, three structure descriptors below are specified as feature amounts having a strong correlation (similar to each other).

- MolWt: Average molecular weight
- HeavyAtomMolWt: Molecular weight excluding hydrogen atoms
- ExactMolWt: Exact molecular weight

Therefore, in the second embodiment, of the three structure descriptors described above, "HeavyAtomMolWt" and "ExactMolWt" are excluded not to be used to generate a classification model, and a classification model is generated.

Moreover, in the second embodiment, an average of relative errors regarding the feature amount is obtained using the following equation.

[Expression 18]

$$E_{ave} = \frac{1}{n}\sum_{i=1}^{n}\frac{\left|x_i^s - x_i^q\right|}{\left|x_i^q\right|}$$

Here, in the equation described above, "$E_{ave}$" means an average of relative errors. Furthermore, "$x_i^s$" means a value of an i-th structure descriptor in a molecule included in test data, and "$x_i^q$" means a value of an i-th structure descriptor in a molecule to be a reference (in second embodiment, PubChem CID603597). Furthermore, in the above equation, "n" means the total number of the feature amounts.

In the above equation, for example, calculation is performed as excluding "SlogP_VSA3" of which the value of the structure descriptor of the reference molecule (PubChem CID603597) is "0" from "$x_i^q$".

Then, in the second embodiment, an index represented by the following equation is obtained.

[Expression 19]

$$S_{new} = \alpha S_{DA} + (1 - \alpha)(1 - E_{ave})$$

Here, in the equation described above, "$S_{new}$" means an index using an average of relative errors of feature amounts and a structural similarity, "$S_{DA}$" means a structural similarity, "$E_{ave}$" means an average of relative errors, and "a" means a coefficient (1/2 in second embodiment).

Furthermore, in order to verify accuracy of the above index "$S_{new}$", an index based on only the structural similarity ($S_{DA}$) (corresponding to case of $\alpha$ = 1 in above equation) is obtained in a similar manner to the method described above.

Moreover, in order to verify the accuracy of the above index "$S_{new}$", similarly to the method described above, an index (corresponding to case of $\alpha$ = 0 in above equation) based on only the average of the relative errors of the feature amounts ($E_{ave}$).

FIG. 32 illustrates a result of arranging 10 molecules in a descending order of a value of the index "$S_{new}$" by analyzing 25 pieces of training data using the index "$S_{new}$" using the average of the relative errors of the feature amount and the structural similarity.

FIG. 33 illustrates a result of arranging 10 molecules in a descending order of a value of the index "$S_{DA}$" by analyzing 25 pieces of training data using the index "$S_{DA}$" using only the structural similarity.

FIG. 34 illustrates a result of arranging 10 molecules in a descending order of a value of an index "1 - $E_{ave}$" by analyzing 25 pieces of training data using the index "1 - $E_{ave}$" using only the relative error of the feature amount.

As illustrated in FIG. 32, with the index "$S_{new}$" using the average of the relative errors of the feature amount and the structural similarity, of 10 molecules having a large value of the index "$S_{new}$", nine molecules have the same biological activity "Active" as the molecule to be the reference (CID603597). On the other hand, as illustrated in FIGs. 33 and 34, with the index "$S_{DA}$" using only the structural similarity, five of ten molecules have the biological activity "Active", and with the index "1 - $E_{ave}$" using only the relative error of the feature amount, ten of ten molecules have the biological activity "Active".

As described above, in the second embodiment, an evaluation result of the index "$E_{ave}$" using only the relative error of the feature amount is the highest. In an example of the technology disclosed in this case, as in the second embodiment, for example, in addition to the analysis result according to the index based on the structural similarity and the feature amount (structure descriptor), an analysis result of an index using only the structural similarity and an analysis result of an index using only the feature amount (structure descriptor) may be presented.

In this way, correct analysis can be performed without exception regardless of the analysis target or the type of the model.

Moreover, in the second embodiment, a classification model is generated with "Extra Trees Classifier" using "PyCaret" as in the first embodiment, on the basis of an average of relative errors of feature amounts (six) calculated as described above and the structural similarity. Then, in the second embodiment, regarding the generated classification model, the accuracy of the classification model is verified by performing "k-fold cross validation (k = 10)" using the 25 pieces of training data.

In order to compare accuracy of the classification model, similarly to the method described above, the classification model is generated based on the six feature amounts and the structural similarity. Then, "k-fold cross validation (k = 10)" using the training data is performed on the classification model, and accuracy is verified and compared.

FIG. 35 illustrates an example of a result of "k-fold cross validation (k = 10)" in the classification model based on the average of the relative errors of the six feature amounts and the structural similarity, generated in the second embodiment.

FIG. 36 illustrates an example of a result of "k-fold cross validation (k=10)" in the classification model generated based on the six feature amounts and the structural similarity.

As illustrated in FIG. 35, in the classification model based on the average of the relative errors of the six feature amounts and the structural similarity, "AUC" that is particularly important in binary classification is "0.85". On the other hand, as illustrated in FIG. 36, "AUC" of the classification model generated based on the six feature amounts and the structural similarity is "0.80".

In this way, in the second embodiment, using the classification model based on the average of the relative errors of the feature amounts and the structural similarity, it can be verified that the accuracy of the classification model can be further improved.

Furthermore, in an example of the technology disclosed in this case, as in the second embodiment, for example, when the accuracy of the model is verified, the accuracy may be verified as paying attention to a specific index ("AUC" in second embodiment) that is particularly important, according to the analysis target, the type of the model, and the like.

Moreover, in the second embodiment, the biological activity of the seven pieces of test data is assumed to be unknown, and classification is performed using the classification model based on the average of the relative errors of the feature amounts and the structural similarity.

FIG. 37 illustrates an example of a result of classification regarding seven pieces of test data of which the biological activity is assumed to be unknown, using the classification model based on the average of the relative errors of the feature amounts and the structural similarity, generated in the second embodiment. Furthermore, the format of FIG. 37 is similar to those of FIGs. 30 and 31.

As illustrated in FIG. 37, with the classification model in the second embodiment based on the average of the relative errors of the feature amounts and the structural similarity, it is possible to correctly classify all the seven pieces of test data (accuracy 100%).

In this way, in the second embodiment, it can be confirmed that a molecule of which a biological activity is unknown can be classified (classified) with high accuracy with the prediction model based on the average of the relative errors of the feature amounts and the structural similarity.

(Third Embodiment)

In a third embodiment, 80% of 83 molecules, of which viscosity used in solvent is known, written in the chemistry handbook is used as training data (characteristic data of specific molecule and each of plurality of molecules), and 20% of the 83 molecules is used as test data (non-specific molecule, first molecule). Then, in the third embodiment, a prediction model that predicts viscosity in the test data (multiple regression model) is generated, and accuracy of the prediction model is verified. Note that, in the third embodiment, content other than the procedure or the like described below is similarly performed to that in the first embodiment. Furthermore, in the third embodiment, the viscosity of each molecule is set to a logarithmic value (value obtained by taking log).

First, in the third embodiment, unlike the first embodiment, when a structural similarity between molecules is obtained, similarities of all patterns of combinations of the 83 molecules (83 * 83) are obtained. Then, in the third embodiment, five similarities that contribute to improve the accuracy of the multiple regression model are specified using "Boruta" described above, and the similarities are used to generate the multiple regression model.

In third embodiment, similarities specified as similarities that contribute to improve the accuracy of the multiple regression model are as follows.

- Similarity to PUBCHEM_CID 103
- Similarity to PUBCHEM_CID 174
- Similarity to PUBCHEM_CID 284
- Similarity to PUBCHEM_CID 753
- Similarity to PUBCHEM_CID 887

Subsequently, in the third embodiment, 208 types of structure descriptors are calculated for each of the 83 molecules using "RDKit", and 14 structure descriptors that contribute to improve accuracy of classification are specified from the 208 types of structure descriptors using "Boruta" and used as feature amounts.

In the third embodiment, the 14 structure descriptors selected as feature amounts are as follows.

- MinAbsEStateIndex
- BertzCT
- Chi1v
- Chi3v
- Ipc
- PEOE_VSA1
- TPSA
- EState_VSA2
- VSA_EState3
- NHOHCount
- NumHDonors
- MolLogP
- fr_Al_OH
- fr_Al_OH_noTert

Furthermore, structure descriptors, of which meanings are clear, of the 14 structure descriptors selected as the feature amounts described above are as follows.

- BertzCT: A topological index aimed at quantifying molecular complexity
- Ipc: Information regarding coefficients of characteristic polynomials of an adjacency matrix of a molecular graph
- TPSA: Information regarding coefficients of characteristic polynomials of an adjacency matrix of a molecular graph
- NHOHCount: Information regarding coefficients of characteristic polynomials of an adjacency matrix of a molecular graph
- NumHDonors: Information regarding coefficients of characteristic polynomials of an adjacency matrix of a molecular graph
- MolLogP: Information regarding coefficients of characteristic polynomials of an adjacency matrix of a molecular graph
- fr_Al_OH: Information regarding coefficients of characteristic polynomials of an adjacency matrix of a molecular graph
- fr_Al_OH_noTert: Information regarding coefficients of characteristic polynomials of an adjacency matrix of a molecular graph

Subsequently, in the third embodiment, a prediction model (multiple regression model) is generated on the basis of five structural similarities and 14 feature amounts using "PyCaret". Furthermore, in the third embodiment, a plurality of types of prediction models is collectively generated with "PyCaret", and a prediction model with high accuracy is selected from the generated prediction models and is used.

FIG. 38 illustrates an example of a relationship between a type of a classification model generated in the third embodiment and an index of accuracy of each classification model. As illustrated in FIG. 38, when the indexes of the accuracy of the respective prediction models (Model) are compared, for "CatBoost Regressor", it is found that a value of "R2 (determination coefficient)" that is important when the prediction model is evaluated is high. Therefore, in the third embodiment, "CatBoost Regressor" is used as a prediction model.

Note that, for example, "Liudmila Prokhorenkova, Gleb Gusev, Aleksandr Vorobev, Anna Veronika Dorogush, Andrey Gulin, Bulat Ibragimov, arXiv: 1706.09516" discloses details of "CatBoost Regressor".

Then, in the third embodiment, regarding the generated prediction model, the accuracy of the prediction model is verified by performing "k-fold cross validation (k = 10)" using training data. Note that, in the third embodiment, a parameter of the prediction model is optimized by performing "k-fold cross validation (k = 10)" (100 times of grid search).

Furthermore, in order to compare the accuracy of the prediction model, in the method according to the third embodiment described above, the prediction model is generated on the basis of only the structural similarity (without executing S203 and S204 in FIG. 21). Then, regarding the prediction model based on only the structural similarity, accuracy is verified and compared by performing "k-fold cross validation (k = 10)" using the training data.

Moreover, in order to compare the accuracy of the prediction model in the method according to the third embodiment described above, the prediction model is generated on the basis of only the structure descriptor (14 feature amounts) (without executing S202 in FIG. 21). Then, regarding the prediction model based on only the structure descriptor (14 feature amounts), accuracy is verified and compared by performing "k-fold cross validation (k = 10)" using the training data.

FIG. 39 illustrates an example of a result of "k-fold cross validation (k = 10)" in the prediction model generated in the third embodiment.

FIG. 40 illustrates an example of a result of "k-fold cross validation (k = 10)" in the prediction model generated based on only a structural similarity as an example corresponding to the third embodiment.

FIG. 41 illustrates an example of a result of "k-fold cross validation (k = 10)" in a prediction model generated based on only a structure descriptor (14 feature amounts) as an example corresponding to the third embodiment.

As illustrated in FIG. 39, "R2 (determination coefficient)" that is important when the prediction model is evaluated of the prediction model generated in the third embodiment is "0.4644". On the other hand, as illustrated in FIGs. 40 and 41, "R2 (determination coefficient)" of a prediction model generated based on only a structural similarity is "0.0993", and "R2" of a prediction model generated on the basis of only a structure descriptor (14 feature amounts) is "0.4751".

Moreover, in the third embodiment, it is assumed that viscosity of test data be unknown, and the viscosity is predicted using a prediction model generated based on the structural similarity and the structure descriptor (14 feature amounts).

FIG. 42 illustrates an example of a result of predicting viscosity of test data of which the viscosity is assumed to be unknown using the prediction model generated in the third embodiment.

FIG. 43 illustrates a result of predicting the viscosity of the test data of which the viscosity is assumed to be unknown using a prediction model generated based on only the structural similarity as an example corresponding to the third embodiment.

FIG. 44 illustrates a result of predicting the viscosity of the test data of which the viscosity is assumed to be unknown using a prediction model generated based on only the structure descriptor (14 feature amounts) as an example corresponding to the third embodiment.

In FIGs. 42 to 44, in a graph (Prediction Error for CatBoost Regressor), the vertical axis means correct viscosity of test data, and the horizontal axis means viscosity predicted by the prediction model in the test data.

As illustrated in FIG. 42, for the prediction model generated based on the structural similarity and the structure descriptor (14 feature amounts), "R2" of the test data of which the viscosity is assumed to be unknown is "0.7165". On the other hand, as illustrated in FIGs. 43 and 44, "R2" of the test data of the prediction model generated based on only the structural similarity is "- 0.1039", and "R2" of the prediction model generated based on only the structure descriptor (14 feature amounts) is "0.7368".

In this way, in the third embodiment, evaluation results of the prediction model generated based on the five structural similarities and the 14 feature amounts and the prediction model generated based on only the 14 feature amounts are higher than the evaluation result of the prediction model generated based on only the five structural similarities. In an example of the technology disclosed in this case, in the third embodiment, for example, in addition to an analysis result by the prediction model based on the structural similarity and the feature amount, an analysis result by the prediction model using only the structural similarity and an analysis result by the prediction model using only the feature amount (structure descriptor) may be presented.

In this way, correct analysis can be performed without exception regardless of the analysis target or the type of the model even in a case where regression prediction is performed.

## Claims

1. An information processing program in which an information processing apparatus analyzes a first molecule different from all of a plurality of molecules based on characteristic data of each of the plurality of molecules performs processing of:

   specifying a structure descriptor that is an index based on each of structures of the plurality of molecules; and
   generating a model used to analyze the first molecule based on the structure descriptor and a similarity between each of the structures of the plurality of molecules.

2. The information processing program according to claim 1, wherein

   the specifying includes specifying the structure descriptor contributing to improve accuracy of the model from among a plurality of structure descriptors as a feature amount, and
   the generating includes generating the model based on the similarity and the feature amount.

3. The information processing program according to claim 1, wherein
   the specifying includes specifying, by performing correlation analysis regarding a plurality of feature amounts, structure descriptors correlating to each other from among a plurality of structure descriptors as a feature amounts, at least one of the feature amounts bring not used to generate the model.

4. The information processing program according to claim 2, wherein the processing further includes:

   specifying a relative error of a feature amount of another molecule included in the plurality of molecules with respect to the feature amount of one molecule included in the plurality of molecules, wherein
   the generating includes generating the model based on the similarity and the relative error.

5. The information processing program according to claim 2, wherein the processing further includes:

   setting a weight to each of the plurality of feature amounts according to a degree of contribution to an improvement of accuracy of the model, wherein
   the relative error is specified based on the weight.

6. The information processing program according to according to any one of claims 1 to 5, the process further comprising:

   specifying analysis accuracy when analysis for verification using the plurality of molecules is performed, by the model, wherein
   updating the model by changing at least one of a model generation method and a parameter until the analysis accuracy becomes equal to or higher than a predetermined value.

7. The information processing program according to according to any one of claims 1 to 6, wherein the model is a prediction model that predicts a characteristic value of the first molecule or a classification model that classifies the first molecule based on the characteristic value.

8. The information processing program according to according to any one of claims 1 to 7, wherein

the similarity is obtained by searching for a maximum independent set based on molecule structures of a second molecule and a third molecule included in the plurality of molecules using the following equation (1),
[Expression 1]

$$H = -\alpha \sum_{i=0}^{n-1} b_i x_i + \beta \sum_{i,j=0}^{n-1} w_{ij} x_i x_j \quad \text{... EQUATION (1)}$$

where, in the equation (1),

the H is Hamiltonian that means that minimizing the H is searching for the maximum independent set,
the n corresponds to the number of nodes of a conflict graph of the second molecule and the third molecule expressed as graphs,
the conflict graph corresponds to a graph created on the basis of a rule in which a combination of each node atom included in the second molecule expressed as a graph and each node atom included in the third molecule expressed as a graph is set as the node, the plurality of nodes is compared and an edge between the nodes that are not identical to each other is created, and the plurality of nodes is compared and an edge is not created between the nodes that are identical to each other,
the $b_i$ is a numerical value that represents a bias with respect to the i-th node,
the $w_{ij}$ is
a positive number that is not zero when an edge exists between the i-th node and the j-th node and is zero when no edge exists between the i-th node and the j-th node,
the $x_i$ is a binary variable that represents that the i-th node is zero or one,
the $x_j$ is a binary variable that represents that the j-th node is zero or one, and
the $\alpha$ and the $\beta$ are positive numbers.

9. The information processing program according to according to claim 8, wherein

the similarity for a searched maximum independent set is obtained using the following equation (2),
[Expression 2]

$$S(G_A, G_B) = \delta \max\left\{\frac{|V_C^A|}{|V_A|}, \frac{|V_C^B|}{|V_B|}\right\} + (1-\delta) \min\left\{\frac{|V_C^A|}{|V_A|}, \frac{|V_C^B|}{|V_B|}\right\}$$

... EQUATION (2)

where, in the equation (2),

the $G_A$ represents the second molecule expressed as a graph,
the $G_B$ represents the third molecule expressed as a graph,
the S ($G_A$, $G_B$) represents the similarity between the second molecule expressed as a graph and the third molecule expressed as a graph, is represented by zero to one, and means that the similarity is higher as S ($G_A$, $G_B$) is closer to one,
the $V_A$ represents the total number of the node atoms of the second molecule expressed as a graph,
the $V_c^A$ represents the number of the node atoms included in a maximum independent set of the conflict graph of the node atoms of the second molecule expressed as a graph,
the $V_B$ represents the total number of the node atoms of the third molecule expressed as a graph,
the $V_c^B$ represents the number of the node atoms included in a maximum independent set of the conflict graph of the node atoms of the third molecule expressed as a graph, and
the δ is a number of zero to one.

**10.** The information processing program according to according to claim 8 or 9, wherein a node in the conflict graph is a combination of two node atoms that have the same atom type subdivided from elemental species between the second molecule and the third molecule.

**11.** The information processing program according to according to any one of claims 8 to 10, wherein
the maximum independent set is searched by minimizing the Hamiltonian in the equation (1) with an annealing method.

**12.** The information processing program according to claim 1, wherein the first molecule is analyzed by inputting data of the first molecule into the model generated in the model generation process.

**13.** An information processing apparatus that analyzes a first molecule different from all of a plurality of molecules based on characteristic data of each of the plurality of molecules, the information processing apparatus comprising:

a memory; and
a processor coupled to the memory and configured to:

specify a structure descriptor that is an index based on each of structures of the plurality of molecules; and
generating a model used to analyze the first molecule based on the structure descriptor and a similarity between each of the structures of the plurality of molecules.

**14.** An information processing method performing by an information processing apparatus that analyzes a first molecule different from all of a plurality of molecules based on characteristic data of each of the plurality of molecules to execute a process, the information processing method comprising:

specifying a structure descriptor that is an index based on each of structures of the plurality of molecules; and
generating a model used to analyze the first molecule based on the structure descriptor and a similarity between each of the structures of the plurality of molecules.

FIG. 1

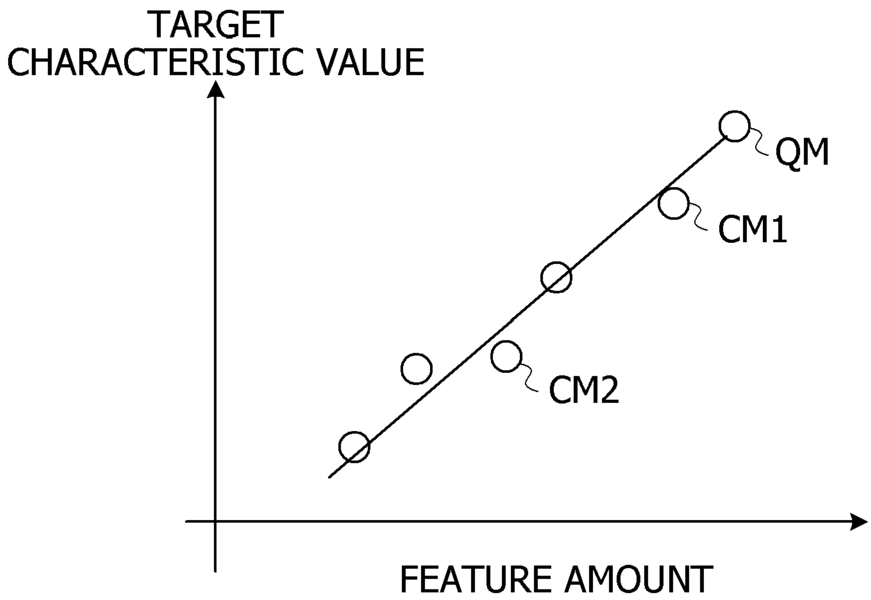

FIG. 2

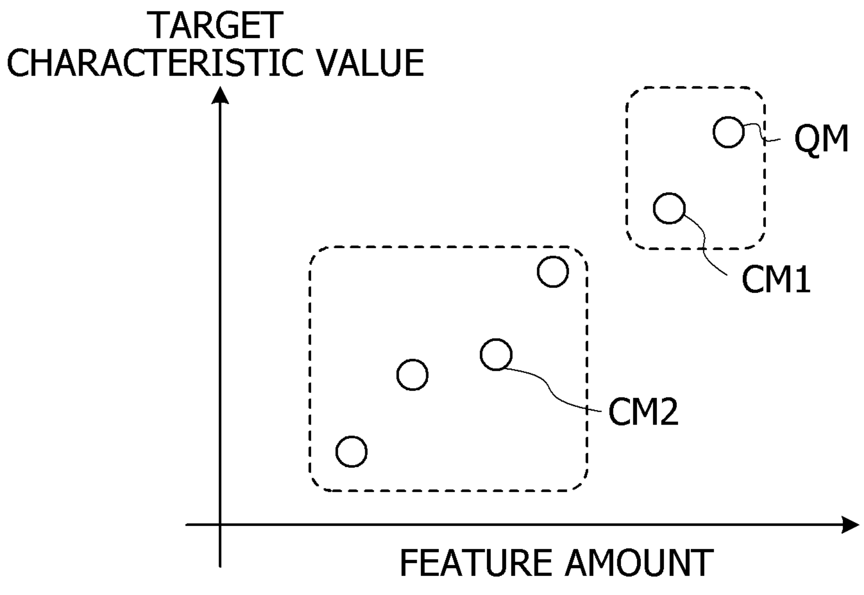

FIG. 3

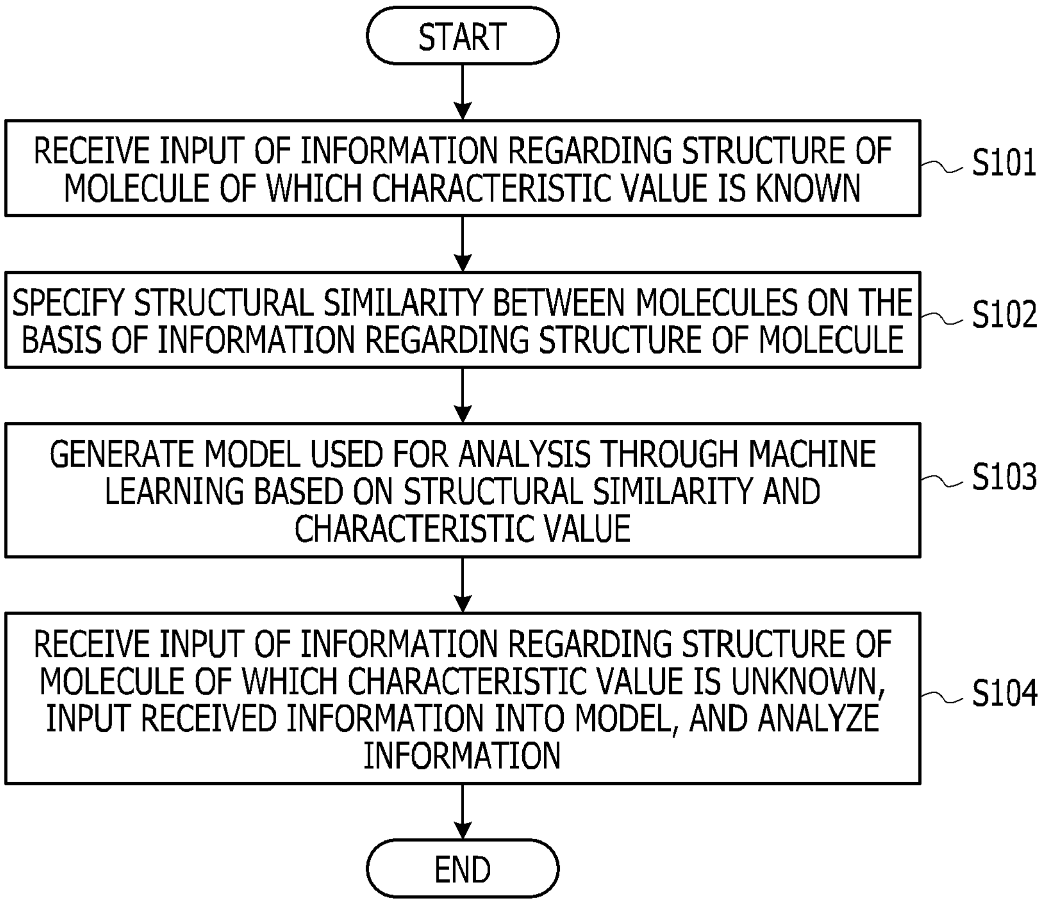
START
RECEIVE INPUT OF INFORMATION REGARDING STRUCTURE OF MOLECULE OF WHICH CHARACTERISTIC VALUE IS KNOWN
S101
SPECIFY STRUCTURAL SIMILARITY BETWEEN MOLECULES ON THE BASIS OF INFORMATION REGARDING STRUCTURE OF MOLECULE
S102
GENERATE MODEL USED FOR ANALYSIS THROUGH MACHINE LEARNING BASED ON STRUCTURAL SIMILARITY AND CHARACTERISTIC VALUE
S103
RECEIVE INPUT OF INFORMATION REGARDING STRUCTURE OF MOLECULE OF WHICH CHARACTERISTIC VALUE IS UNKNOWN, INPUT RECEIVED INFORMATION INTO MODEL, AND ANALYZE INFORMATION
S104
END

FIG. 4

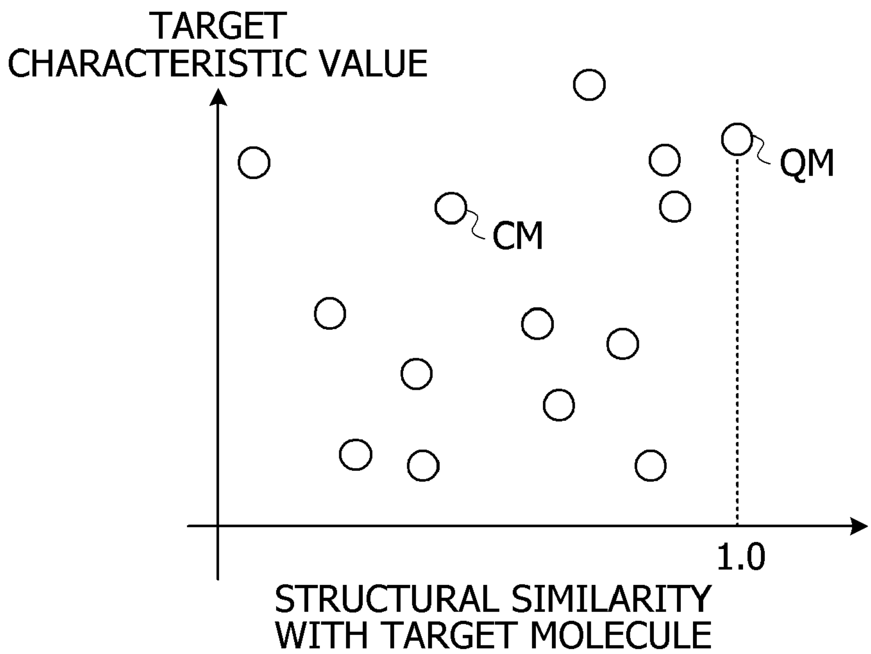
TARGET
CHARACTERISTIC VALUE
QM
CM
1.0
STRUCTURAL SIMILARITY
WITH TARGET MOLECULE

FIG. 5

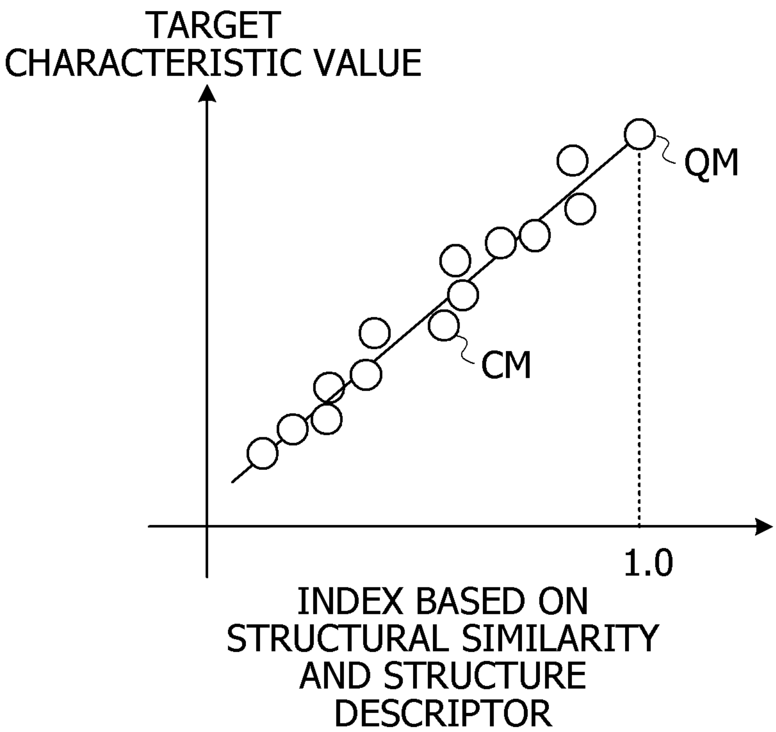
TARGET
CHARACTERISTIC VALUE
QM
CM
1.0
INDEX BASED ON
STRUCTURAL SIMILARITY
AND STRUCTURE
DESCRIPTOR

FIG. 6

MOLECULE A:
ACETIC ACID

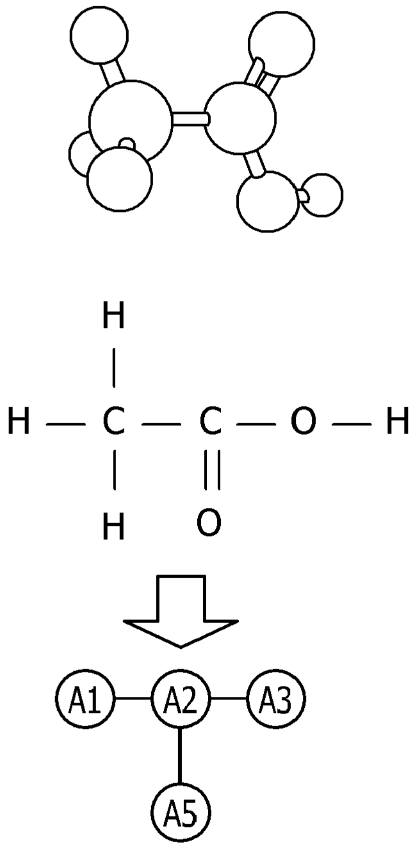


MOLECULE B:
METHYL ACETATE

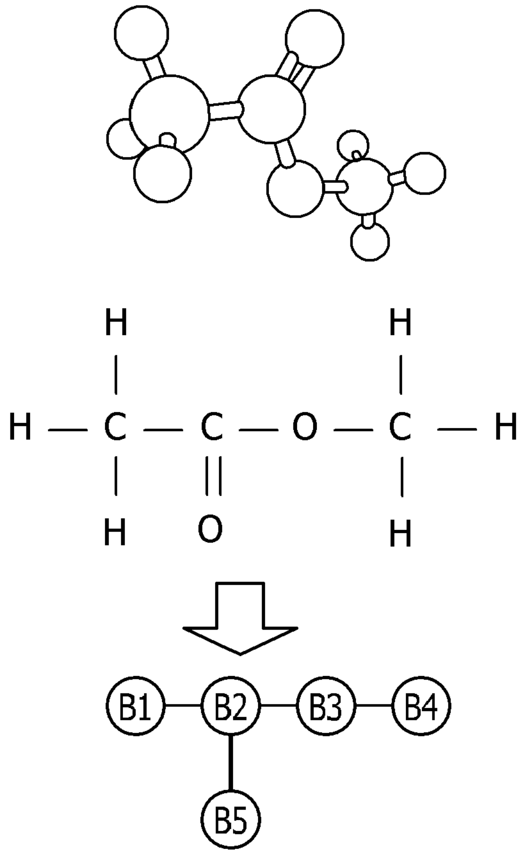

FIG. 7

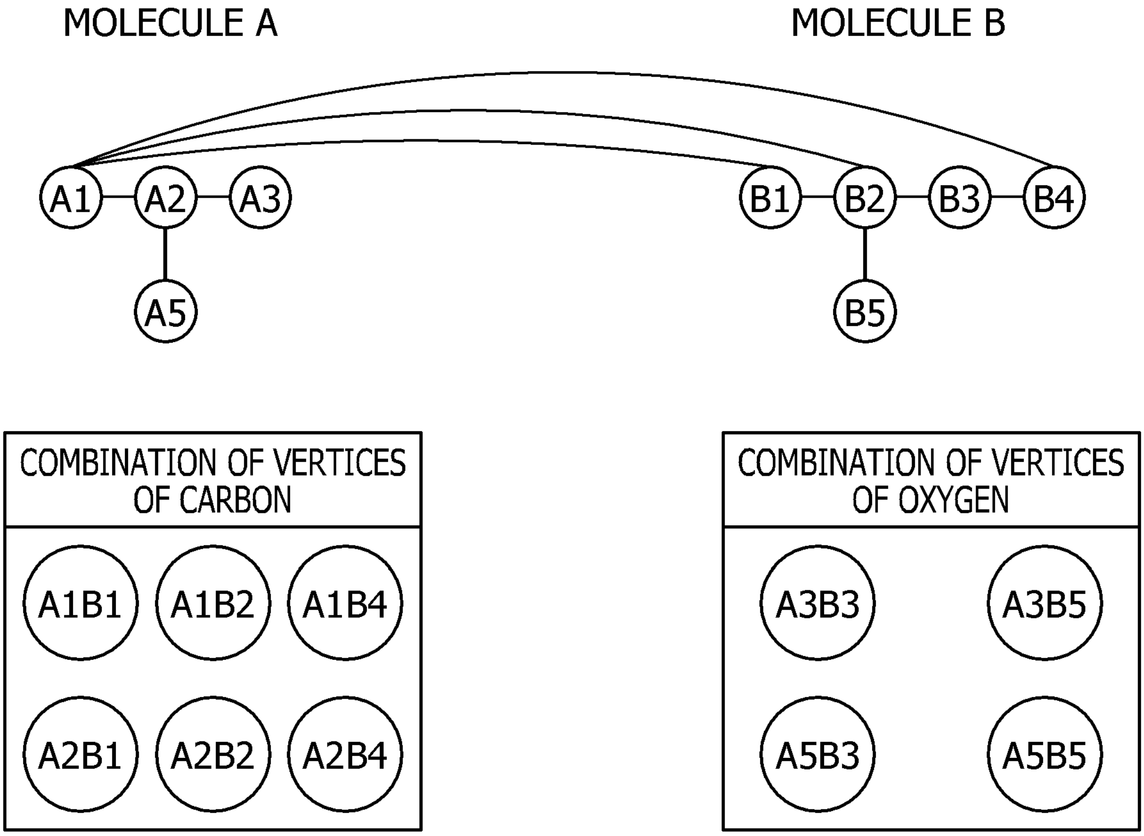
MOLECULE A
MOLECULE B
A1
A2
A3
A5
B1
B2
B3
B4
B5
COMBINATION OF VERTICES OF CARBON
A1B1
A1B2
A1B4
A2B1
A2B2
A2B4
COMBINATION OF VERTICES OF OXYGEN
A3B3
A3B5
A5B3
A5B5

FIG. 8

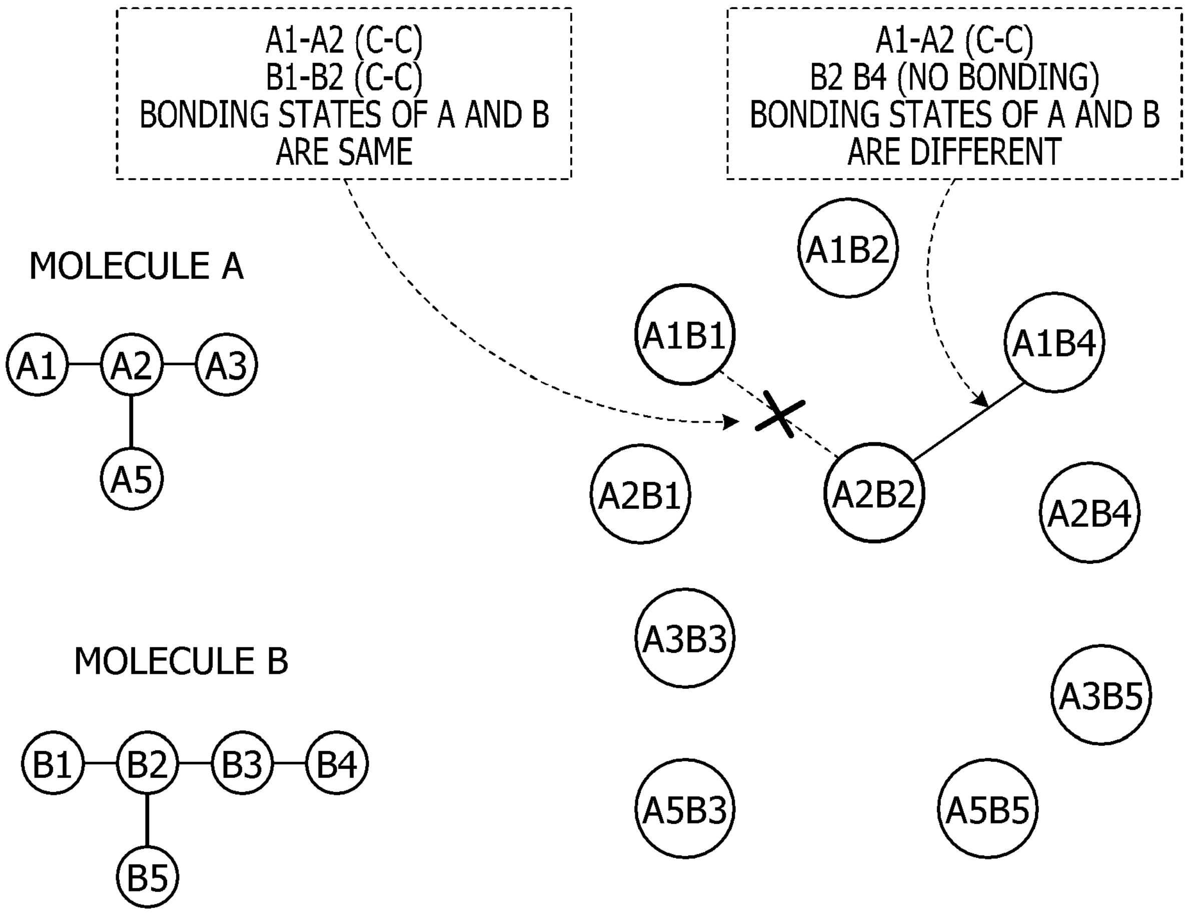
A1-A2 (C-C)
B1-B2 (C-C)
BONDING STATES OF A AND B
ARE SAME
A1-A2 (C-C)
B2 B4 (NO BONDING)
BONDING STATES OF A AND B
ARE DIFFERENT
MOLECULE A
A1
A2
A3
A5
MOLECULE B
B1
B2
B3
B4
B5
A1B2
A1B1
A1B4
A2B1
A2B2
A2B4
A3B3
A3B5
A5B3
A5B5

FIG. 9

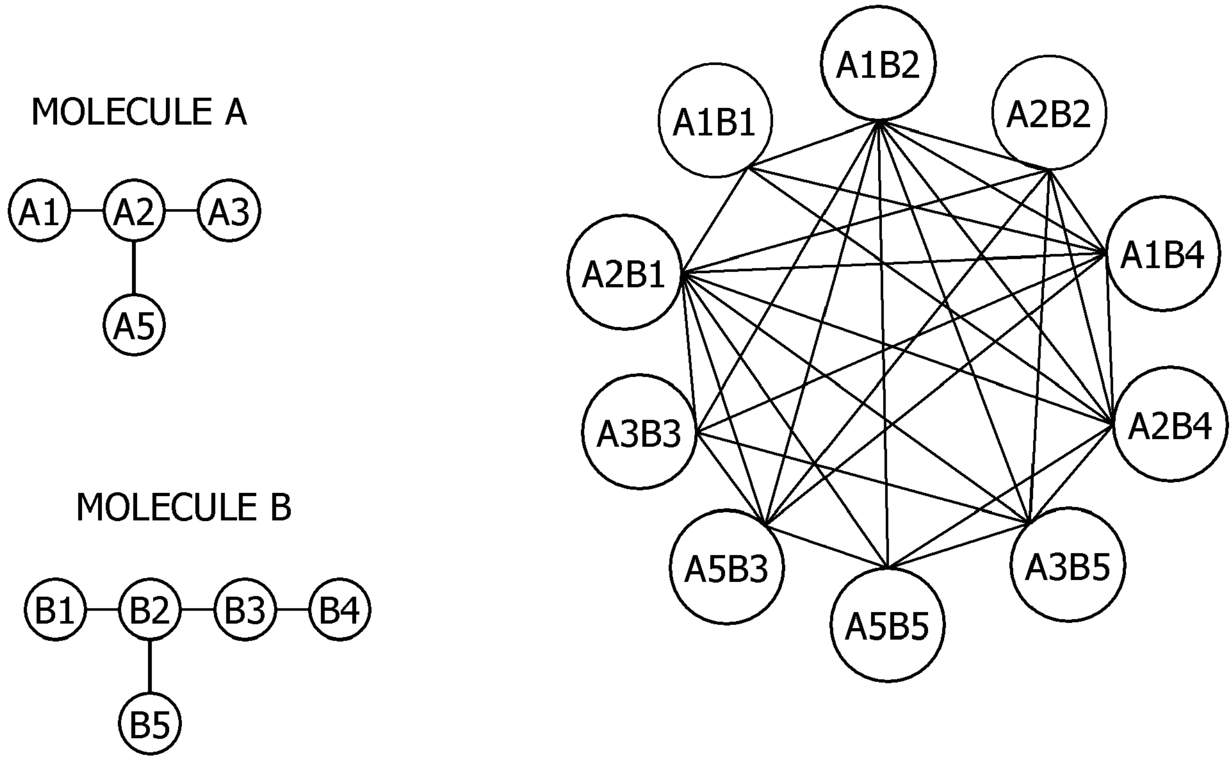
MOLECULE A
A1
A2
A3
A5
MOLECULE B
B1
B2
B3
B4
B5
A1B2
A1B1
A2B2
A1B4
A2B1
A3B3
A2B4
A5B3
A3B5
A5B5

FIG. 10

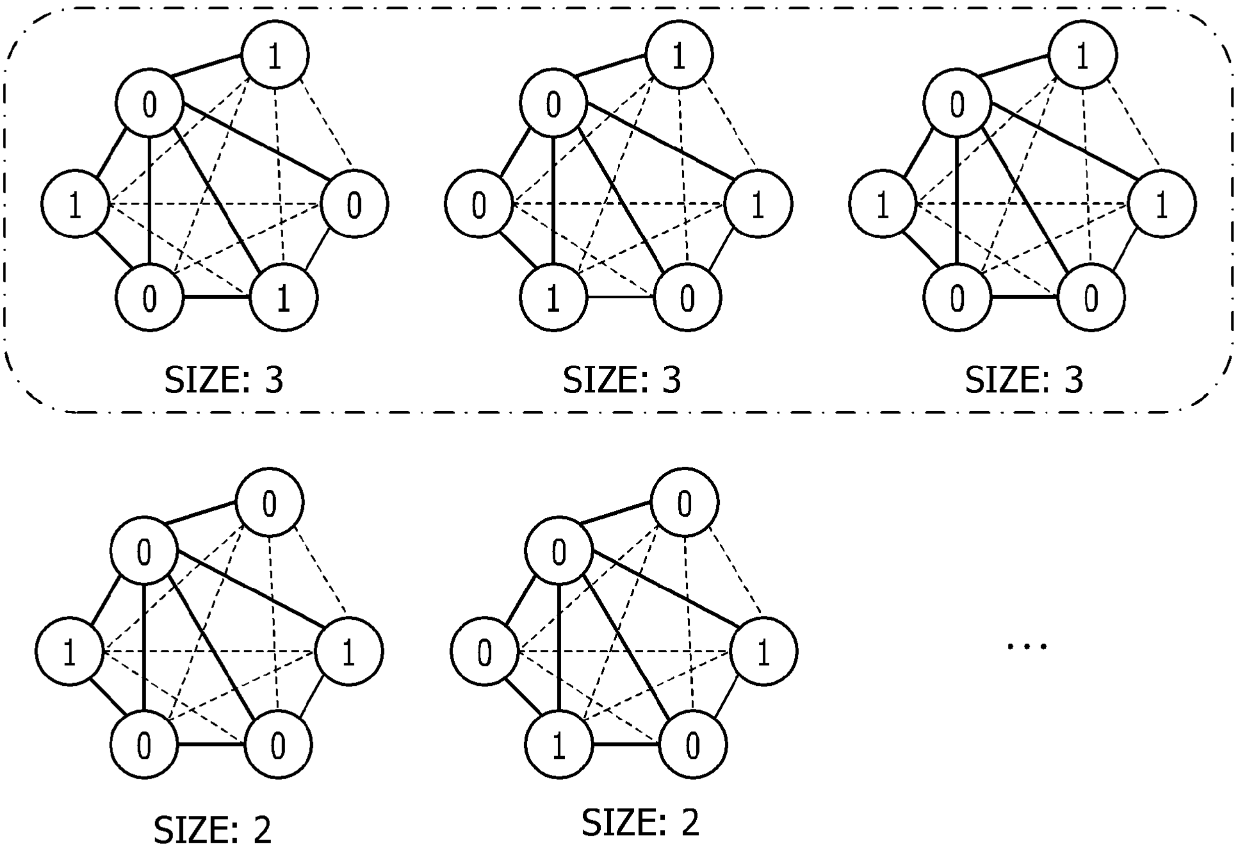

11/44

FIG. 11

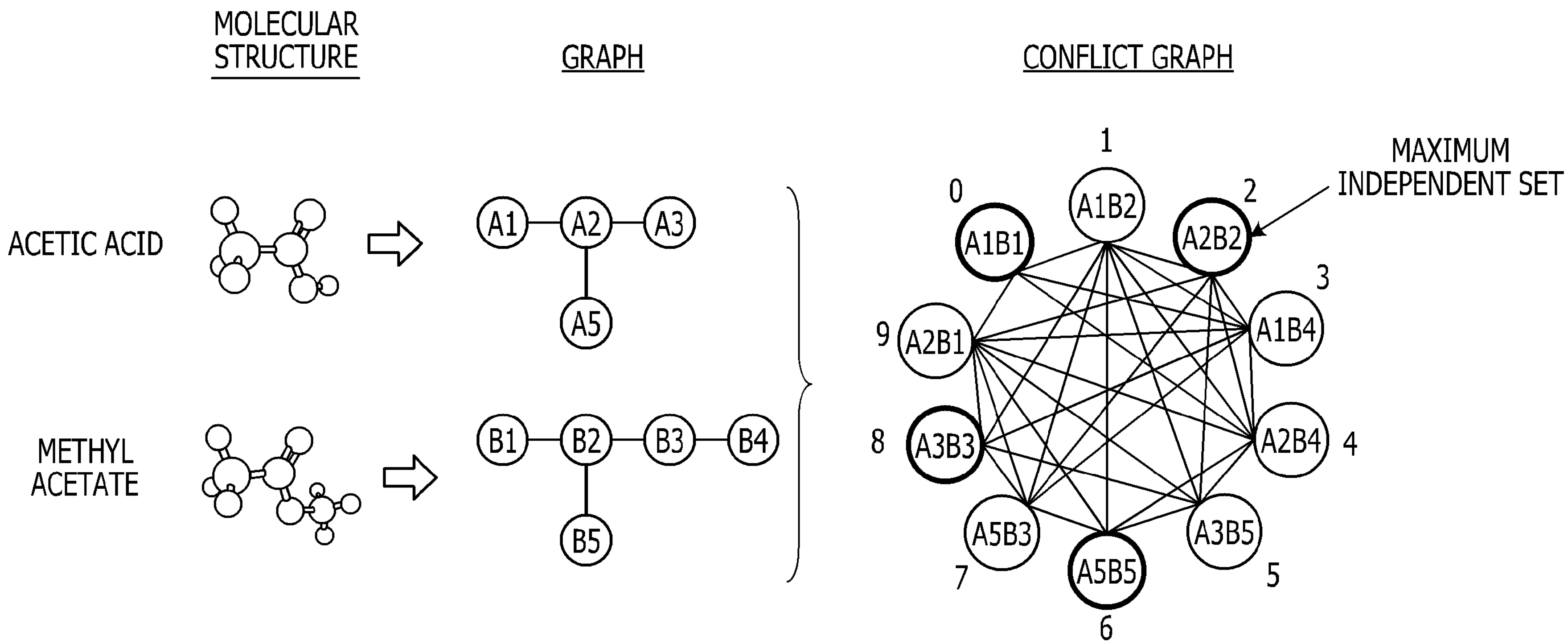
MOLECULAR STRUCTURE
GRAPH
CONFLICT GRAPH
ACETIC ACID
METHYL ACETATE
A1
A2
A3
A5
B1
B2
B3
B4
B5
A1B1
A1B2
A2B2
A1B4
A2B4
A3B5
A5B5
A5B3
A3B3
A2B1
0
1
2
3
4
5
6
7
8
9
MAXIMUM INDEPENDENT SET

FIG. 12

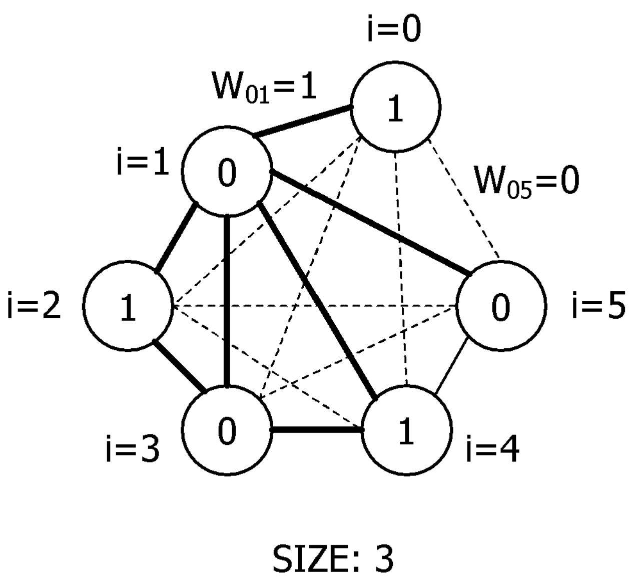

FIG. 13

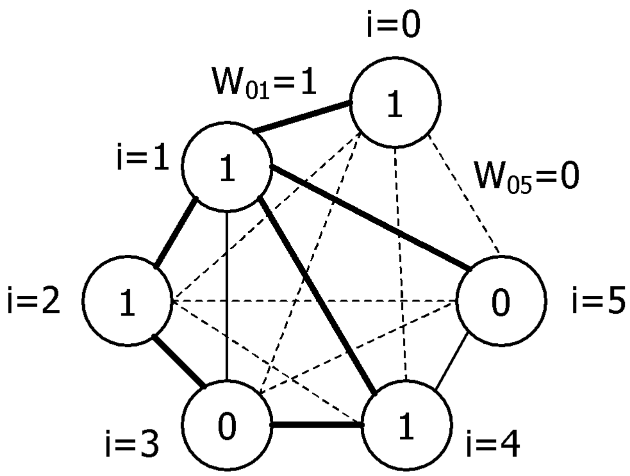


SIZE: 3

FIG. 14

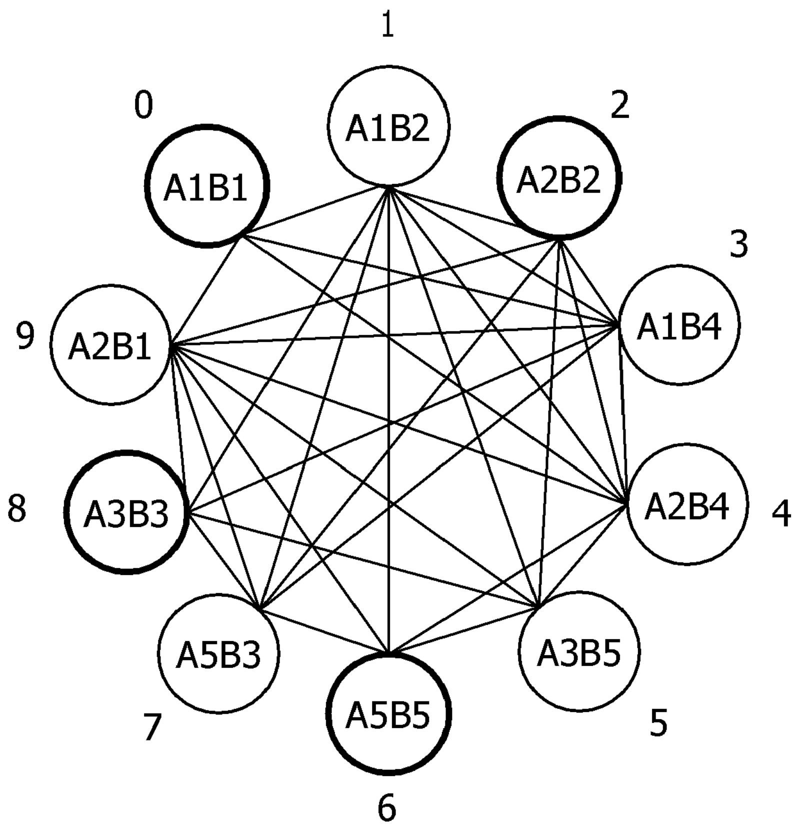
1
0
2
3
9
8
4
7
5
6
A1B2
A1B1
A2B2
A1B4
A2B1
A3B3
A2B4
A5B3
A5B5
A3B5

# FIG. 15

MOLECULE A:
ACETIC ACID

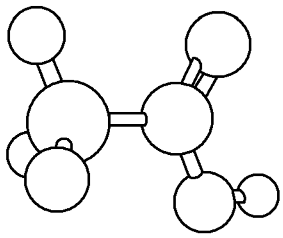

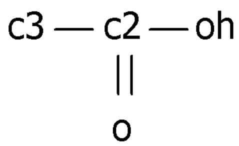

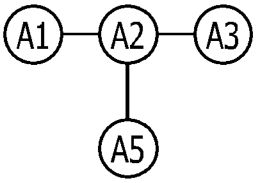


MOLECULE B:
METHYL ACETATE

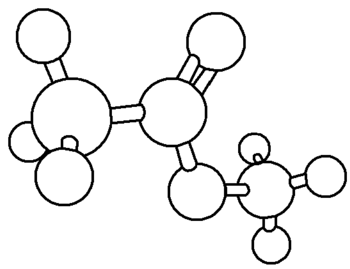

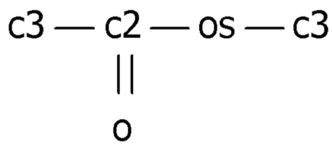

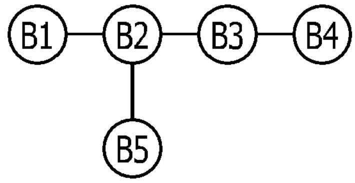

# FIG. 16

MOLECULE A:
ACETIC ACID

MOLECULE B:
METHYL ACETATE

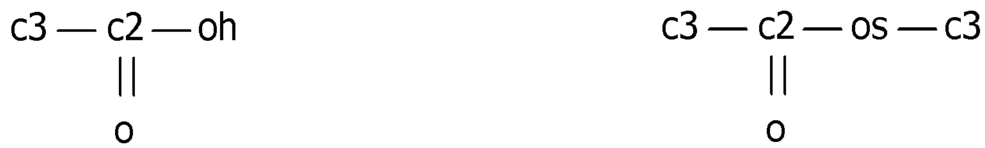

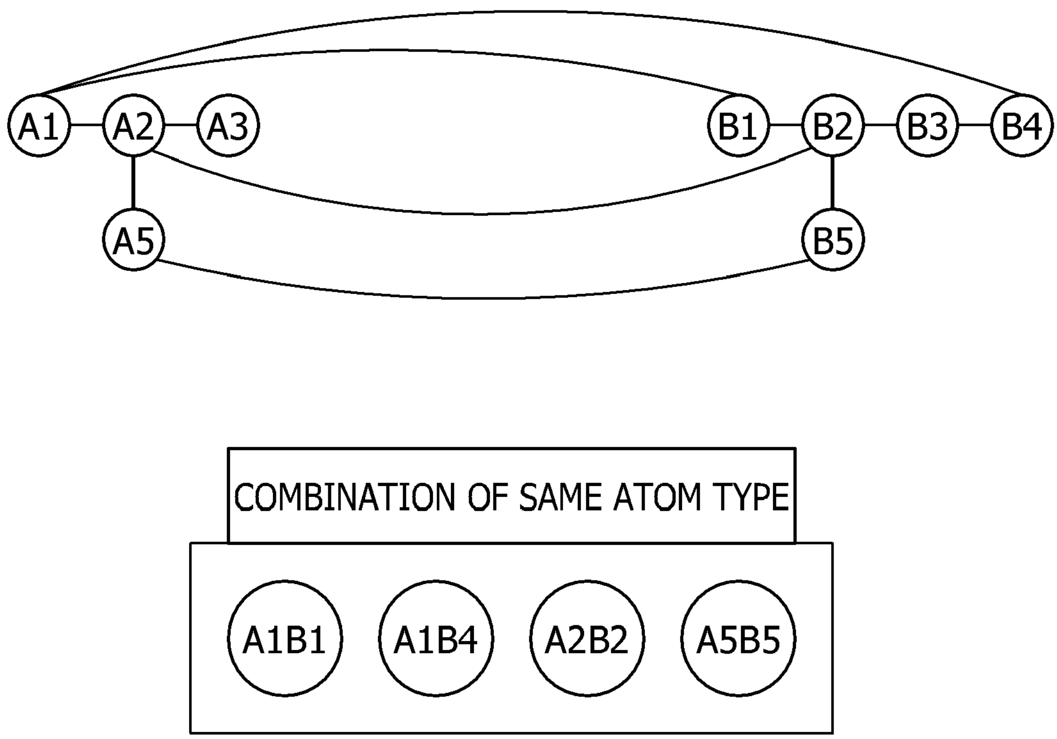

FIG. 17

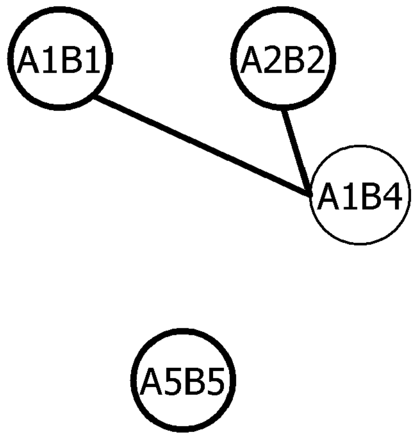

FIG. 18

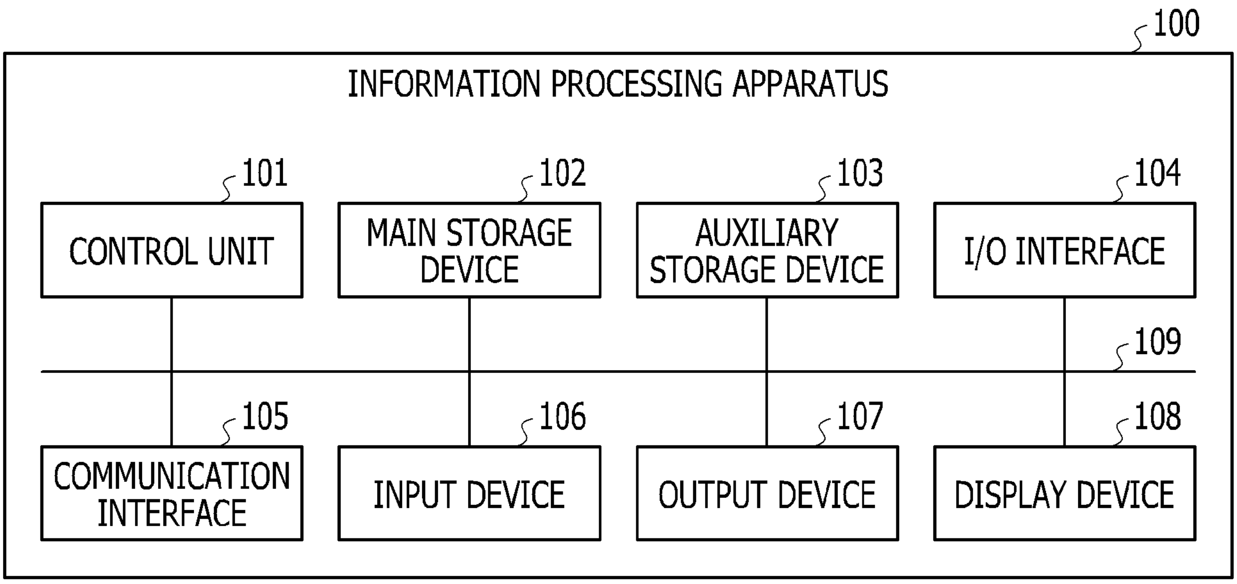
100
INFORMATION PROCESSING APPARATUS
101
CONTROL UNIT
102
MAIN STORAGE DEVICE
103
AUXILIARY STORAGE DEVICE
104
I/O INTERFACE
109
105
COMMUNICATION INTERFACE
106
INPUT DEVICE
107
OUTPUT DEVICE
108
DISPLAY DEVICE

FIG. 19

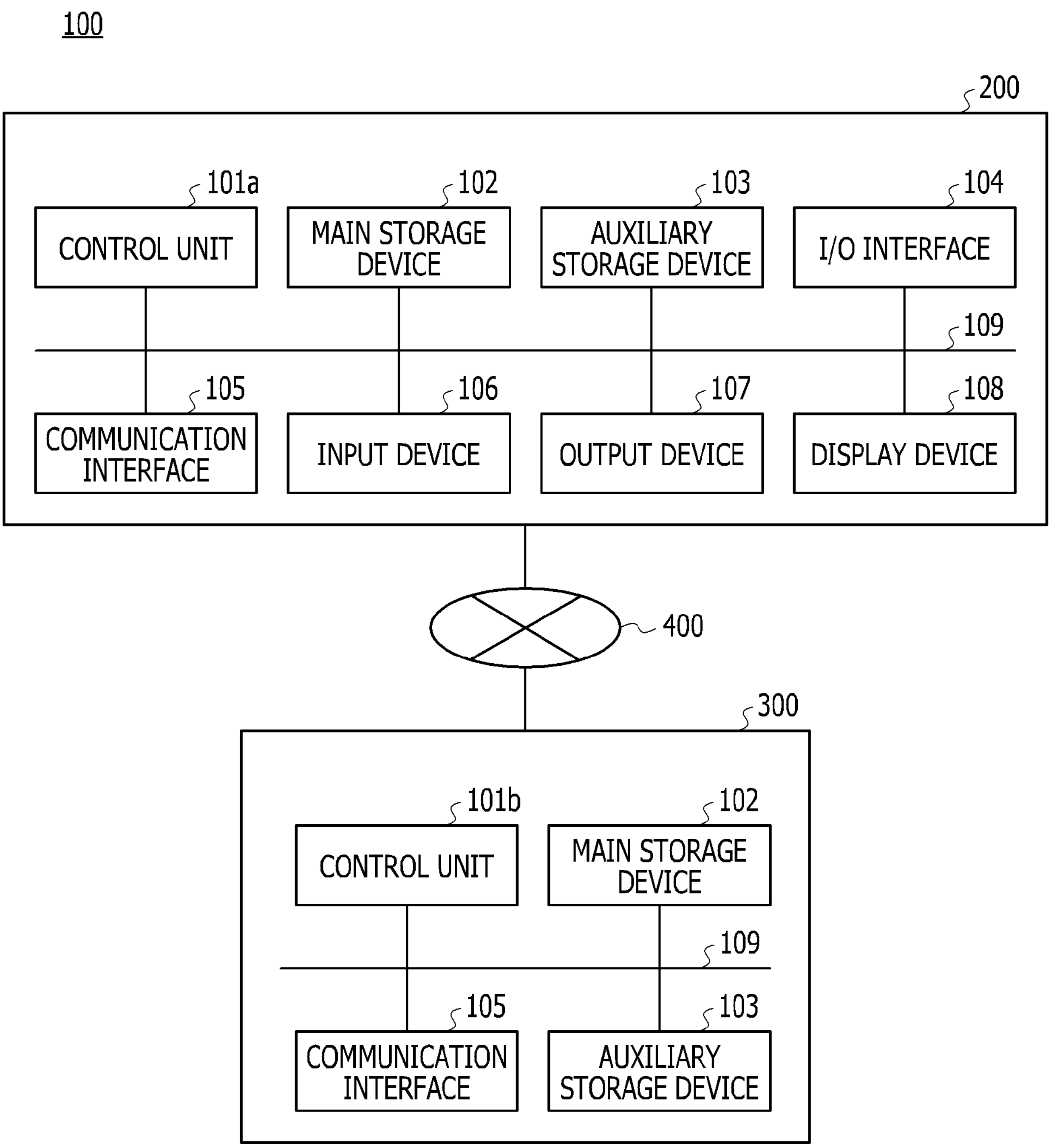
100
200
101a
CONTROL UNIT
102
MAIN STORAGE DEVICE
103
AUXILIARY STORAGE DEVICE
104
I/O INTERFACE
109
105
COMMUNICATION INTERFACE
106
INPUT DEVICE
107
OUTPUT DEVICE
108
DISPLAY DEVICE
400
300
101b
CONTROL UNIT
102
MAIN STORAGE DEVICE
109
105
COMMUNICATION INTERFACE
103
AUXILIARY STORAGE DEVICE

FIG. 20

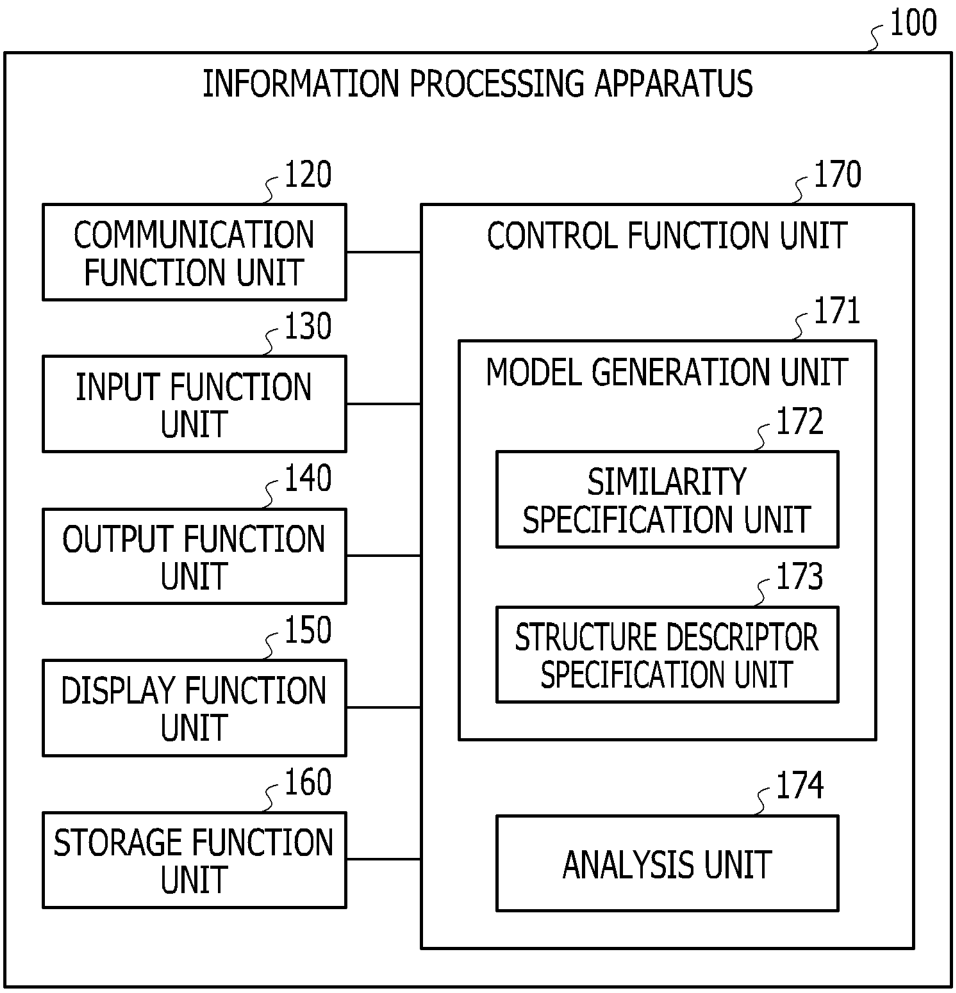
100
INFORMATION PROCESSING APPARATUS
120
COMMUNICATION FUNCTION UNIT
130
INPUT FUNCTION UNIT
140
OUTPUT FUNCTION UNIT
150
DISPLAY FUNCTION UNIT
160
STORAGE FUNCTION UNIT
170
CONTROL FUNCTION UNIT
171
MODEL GENERATION UNIT
172
SIMILARITY SPECIFICATION UNIT
173
STRUCTURE DESCRIPTOR SPECIFICATION UNIT
174
ANALYSIS UNIT

FIG. 21

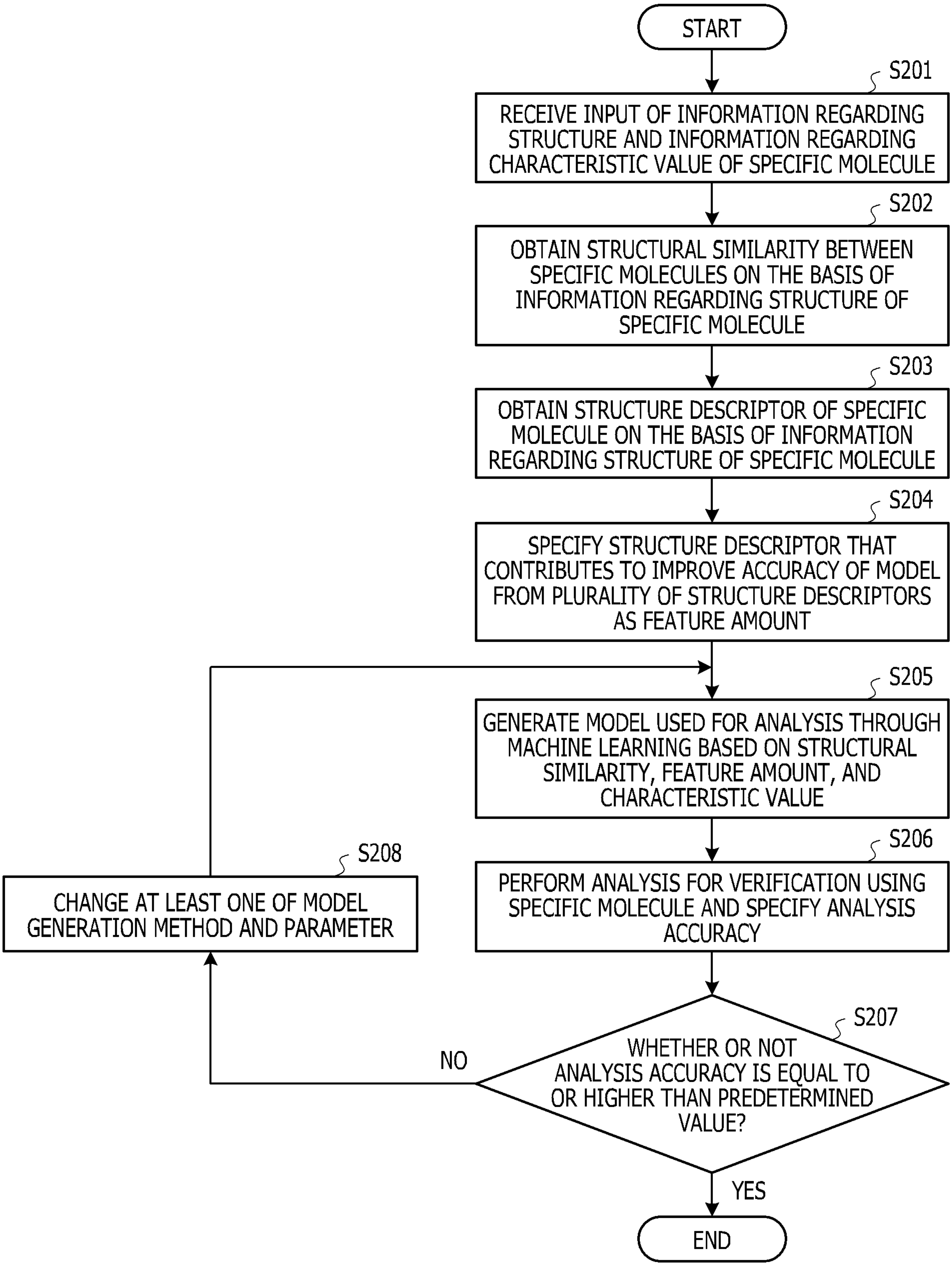
START
S201
RECEIVE INPUT OF INFORMATION REGARDING STRUCTURE AND INFORMATION REGARDING CHARACTERISTIC VALUE OF SPECIFIC MOLECULE
S202
OBTAIN STRUCTURAL SIMILARITY BETWEEN SPECIFIC MOLECULES ON THE BASIS OF INFORMATION REGARDING STRUCTURE OF SPECIFIC MOLECULE
S203
OBTAIN STRUCTURE DESCRIPTOR OF SPECIFIC MOLECULE ON THE BASIS OF INFORMATION REGARDING STRUCTURE OF SPECIFIC MOLECULE
S204
SPECIFY STRUCTURE DESCRIPTOR THAT CONTRIBUTES TO IMPROVE ACCURACY OF MODEL FROM PLURALITY OF STRUCTURE DESCRIPTORS AS FEATURE AMOUNT
S205
GENERATE MODEL USED FOR ANALYSIS THROUGH MACHINE LEARNING BASED ON STRUCTURAL SIMILARITY, FEATURE AMOUNT, AND CHARACTERISTIC VALUE
S206
PERFORM ANALYSIS FOR VERIFICATION USING SPECIFIC MOLECULE AND SPECIFY ANALYSIS ACCURACY
S207
WHETHER OR NOT ANALYSIS ACCURACY IS EQUAL TO OR HIGHER THAN PREDETERMINED VALUE?
NO
S208
CHANGE AT LEAST ONE OF MODEL GENERATION METHOD AND PARAMETER
YES
END

FIG. 22

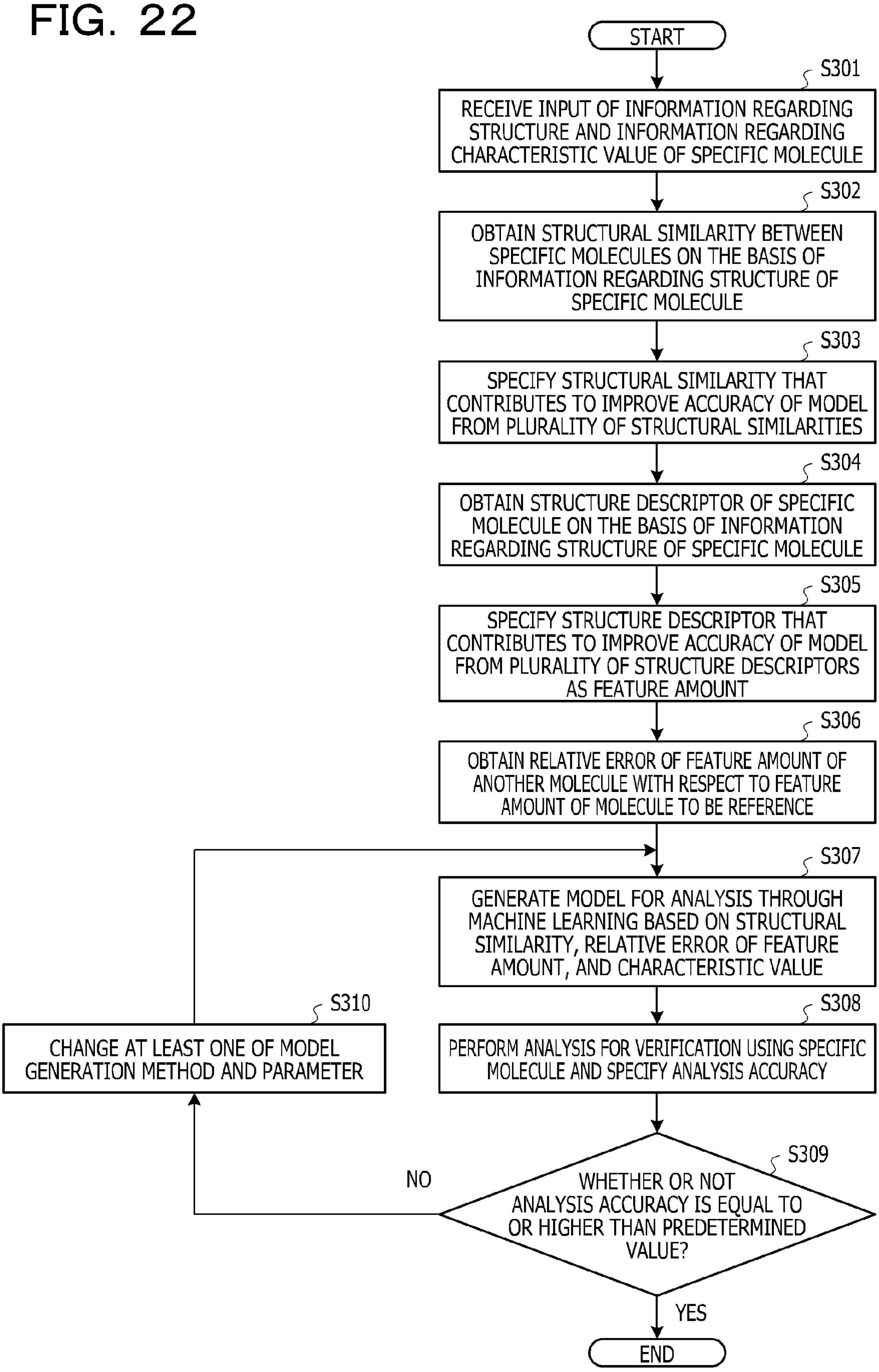
START
S301
RECEIVE INPUT OF INFORMATION REGARDING STRUCTURE AND INFORMATION REGARDING CHARACTERISTIC VALUE OF SPECIFIC MOLECULE
S302
OBTAIN STRUCTURAL SIMILARITY BETWEEN SPECIFIC MOLECULES ON THE BASIS OF INFORMATION REGARDING STRUCTURE OF SPECIFIC MOLECULE
S303
SPECIFY STRUCTURAL SIMILARITY THAT CONTRIBUTES TO IMPROVE ACCURACY OF MODEL FROM PLURALITY OF STRUCTURAL SIMILARITIES
S304
OBTAIN STRUCTURE DESCRIPTOR OF SPECIFIC MOLECULE ON THE BASIS OF INFORMATION REGARDING STRUCTURE OF SPECIFIC MOLECULE
S305
SPECIFY STRUCTURE DESCRIPTOR THAT CONTRIBUTES TO IMPROVE ACCURACY OF MODEL FROM PLURALITY OF STRUCTURE DESCRIPTORS AS FEATURE AMOUNT
S306
OBTAIN RELATIVE ERROR OF FEATURE AMOUNT OF ANOTHER MOLECULE WITH RESPECT TO FEATURE AMOUNT OF MOLECULE TO BE REFERENCE
S307
GENERATE MODEL FOR ANALYSIS THROUGH MACHINE LEARNING BASED ON STRUCTURAL SIMILARITY, RELATIVE ERROR OF FEATURE AMOUNT, AND CHARACTERISTIC VALUE
S308
PERFORM ANALYSIS FOR VERIFICATION USING SPECIFIC MOLECULE AND SPECIFY ANALYSIS ACCURACY
S309
WHETHER OR NOT ANALYSIS ACCURACY IS EQUAL TO OR HIGHER THAN PREDETERMINED VALUE?
NO
S310
CHANGE AT LEAST ONE OF MODEL GENERATION METHOD AND PARAMETER
YES
END

FIG. 23

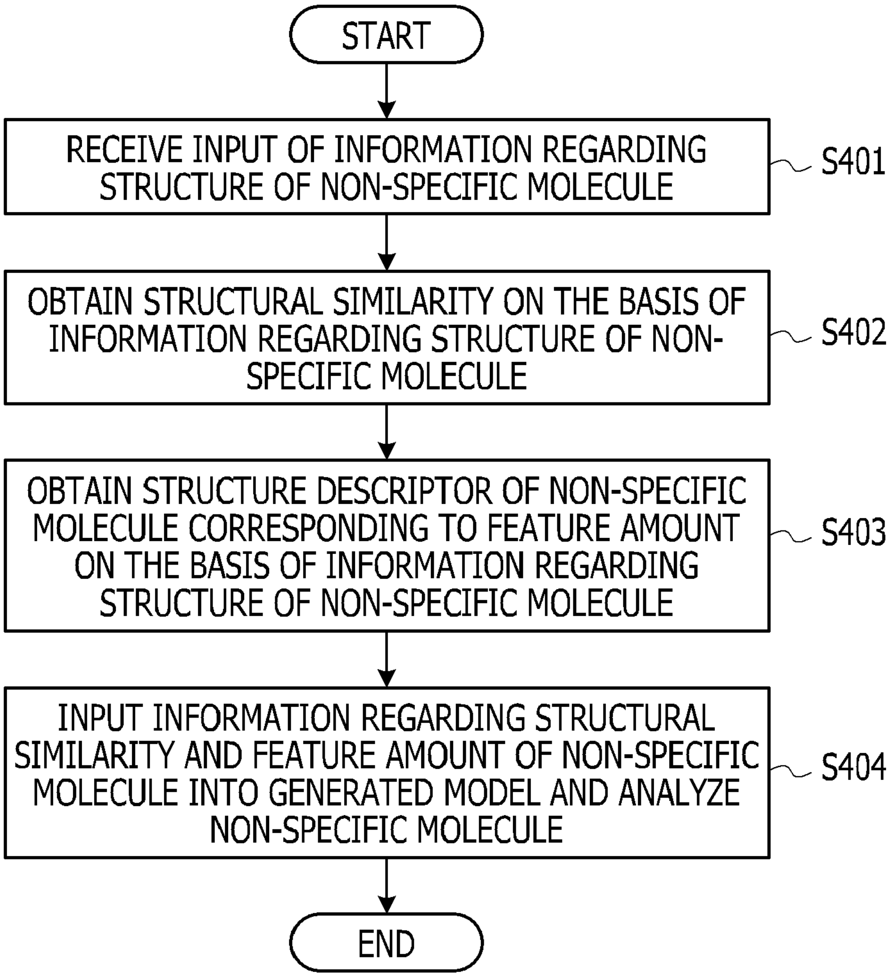
START
RECEIVE INPUT OF INFORMATION REGARDING STRUCTURE OF NON-SPECIFIC MOLECULE
S401
OBTAIN STRUCTURAL SIMILARITY ON THE BASIS OF INFORMATION REGARDING STRUCTURE OF NON-SPECIFIC MOLECULE
S402
OBTAIN STRUCTURE DESCRIPTOR OF NON-SPECIFIC MOLECULE CORRESPONDING TO FEATURE AMOUNT ON THE BASIS OF INFORMATION REGARDING STRUCTURE OF NON-SPECIFIC MOLECULE
S403
INPUT INFORMATION REGARDING STRUCTURAL SIMILARITY AND FEATURE AMOUNT OF NON-SPECIFIC MOLECULE INTO GENERATED MODEL AND ANALYZE NON-SPECIFIC MOLECULE
S404
END

FIG. 24

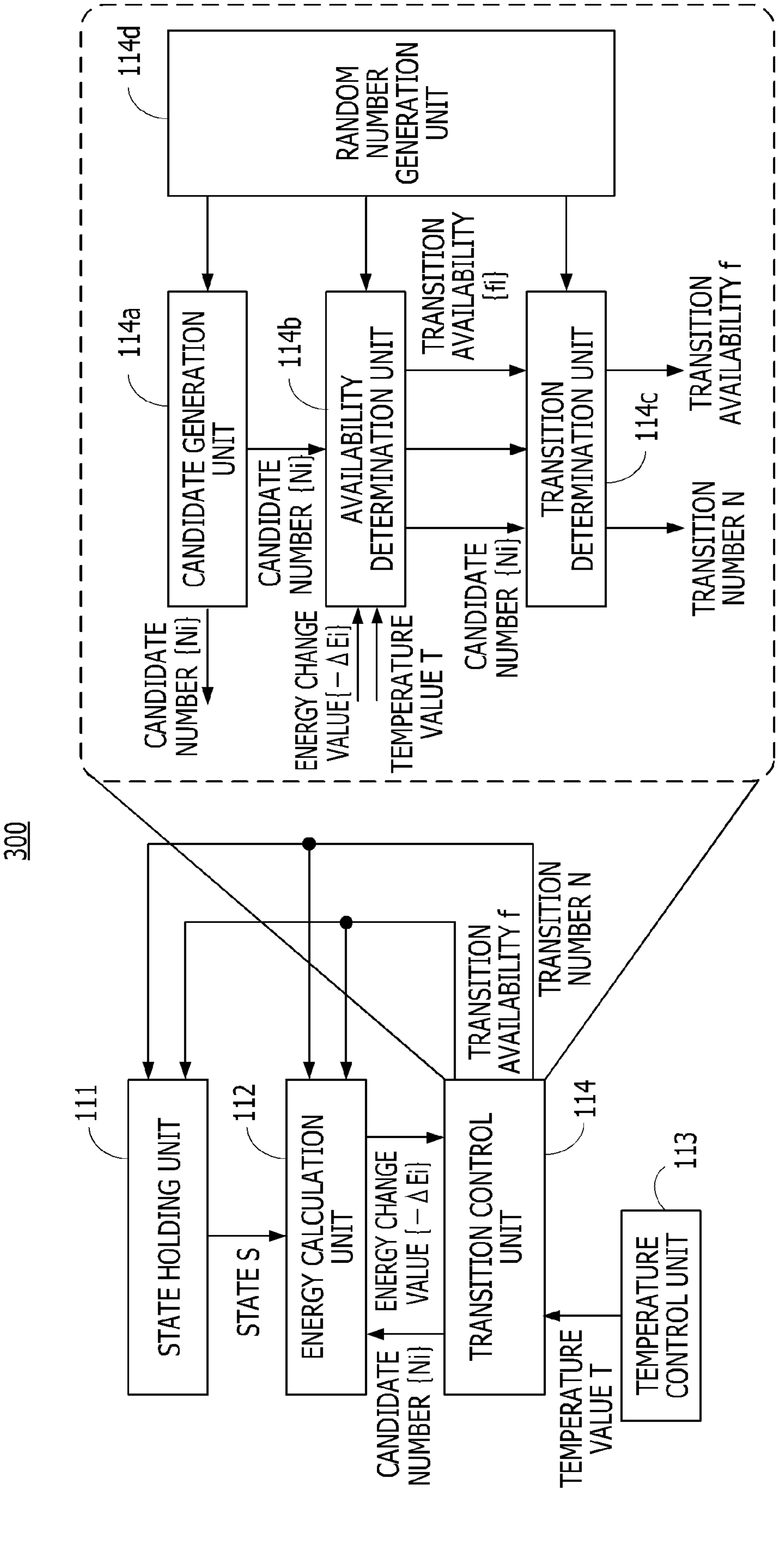
300
111
STATE HOLDING UNIT
STATE S
112
ENERGY CALCULATION UNIT
ENERGY CHANGE VALUE {−ΔEi}
CANDIDATE NUMBER {Ni}
114
TRANSITION CONTROL UNIT
TRANSITION AVAILABILITY f
TRANSITION NUMBER N
TEMPERATURE VALUE T
113
TEMPERATURE CONTROL UNIT
114d
RANDOM NUMBER GENERATION UNIT
114a
CANDIDATE GENERATION UNIT
CANDIDATE NUMBER {Ni}
114b
AVAILABILITY DETERMINATION UNIT
ENERGY CHANGE VALUE{−ΔEi}
TEMPERATURE VALUE T
TRANSITION AVAILABILITY {fi}
CANDIDATE NUMBER {Ni}
114c
TRANSITION DETERMINATION UNIT
TRANSITION AVAILABILITY f
TRANSITION NUMBER N

FIG. 25

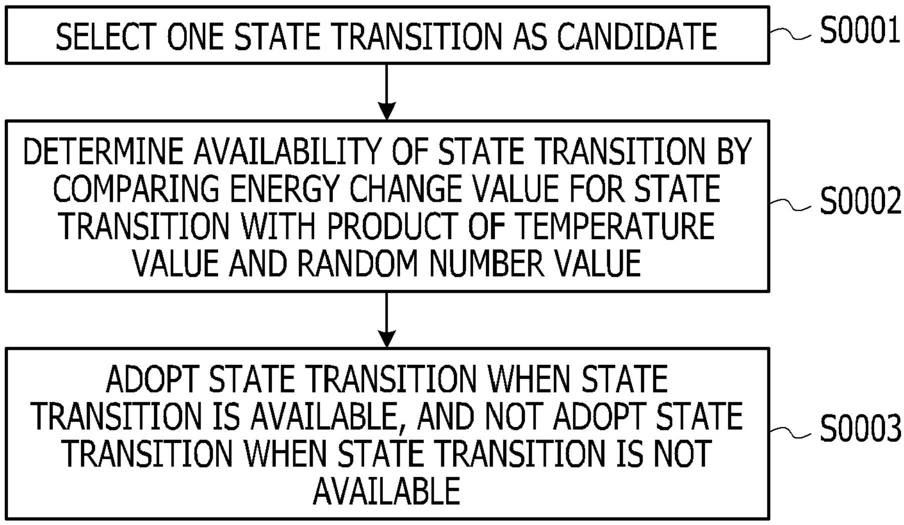
SELECT ONE STATE TRANSITION AS CANDIDATE
S0001
DETERMINE AVAILABILITY OF STATE TRANSITION BY COMPARING ENERGY CHANGE VALUE FOR STATE TRANSITION WITH PRODUCT OF TEMPERATURE VALUE AND RANDOM NUMBER VALUE
S0002
ADOPT STATE TRANSITION WHEN STATE TRANSITION IS AVAILABLE, AND NOT ADOPT STATE TRANSITION WHEN STATE TRANSITION IS NOT AVAILABLE
S0003

FIG. 26

| | MODEL | ACCURACY | AUC | RECALL | PREC. | F1 | KAPPA | MCC | TT (Sec) |
|---|---|---|---|---|---|---|---|---|---|
| nb | Naive Bayes | 0.8833 | 0.9000 | 0.9000 | 0.8167 | 0.8467 | 0.7000 | 0.7000 | 0.1720 |
| ada | Ada Boost Classifier | 0.8500 | 0.8750 | 0.8500 | 0.8167 | 0.8133 | 0.6400 | 0.6500 | 0.0210 |
| et | Extra Trees Classifier | 0.8500 | 0.9500 | 0.8500 | 0.8167 | 0.8133 | 0.6400 | 0.6500 | 0.1200 |
| catboost | CatBoost Classifier | 0.8500 | 0.9000 | 0.8500 | 0.8167 | 0.8133 | 0.6400 | 0.6500 | 1.3680 |
| rf | Random Forest Classifier | 0.8167 | 0.9000 | 0.8000 | 0.8000 | 0.7833 | 0.5900 | 0.6000 | 0.1310 |
| ridge | Ridge Classifier | 0.7833 | 0.0000 | 0.7000 | 0.7500 | 0.7000 | 0.5800 | 0.6000 | 0.0130 |
| ida | Linear Discriminant Analysis | 0.7833 | 0.8500 | 0.7000 | 0.7500 | 0.7000 | 0.5800 | 0.6000 | 0.0140 |
| lr | Logistic Regression | 0.7667 | 0.9500 | 0.7500 | 0.8000 | 0.7333 | 0.5200 | 0.5500 | 0.1790 |
| dt | Decision Tree Classifier | 0.7667 | 0.7750 | 0.9000 | 0.8000 | 0.8167 | 0.5300 | 0.5500 | 0.1720 |
| knn | K Neighbors Classifier | 0.7500 | 0.7250 | 0.6500 | 0.6333 | 0.6267 | 0.3900 | 0.4000 | 0.1950 |
| qda | Quadratic Discriminant Analysis | 0.7000 | 0.8000 | 0.8000 | 0.6333 | 0.6933 | 0.3000 | 0.3000 | 0.0140 |
| gbc | Gradient Boosting Classifier | 0.6833 | 0.7500 | 0.8000 | 0.6500 | 0.6833 | 0.3700 | 0.4000 | 0.0180 |
| svm | SVM - Linear Kernel | 0.4833 | 0.000 | 0.5000 | 0.2500 | 0.3267 | 0.0000 | 0.0000 | 0.0210 |
| lightgbm | Light Gradient Boosting Machine | 0.4167 | 0.5000 | 0.7000 | 0.3167 | 0.4333 | 0.0000 | 0.0000 | 0.0170 |

FIG. 27

| | ACCURACY | AUC | RECALL | PREC. | F1 | KAPPA | MCC |
|---|---|---|---|---|---|---|---|
| 0 | 0.6667 | 0.5000 | 1.0000 | 0.6667 | 0.8000 | 0.0000 | 0.0000 |
| 1 | 1.0000 | 1.0000 | 1.0000 | 1.0000 | 1.0000 | 1.0000 | 1.0000 |
| 2 | 0.6667 | 1.0000 | 1.0000 | 1.0000 | 0.6667 | 0.4000 | 0.5000 |
| 3 | 1.0000 | 1.0000 | 0.0000 | 1.0000 | 1.0000 | 1.0000 | 1.0000 |
| 4 | 0.6667 | 1.0000 | 1.0000 | 0.0000 | 0.0000 | 0.0000 | 0.0000 |
| 5 | 1.0000 | 1.0000 | 1.0000 | 1.0000 | 1.0000 | 1.0000 | 1.0000 |
| 6 | 1.0000 | 1.0000 | 1.0000 | 1.0000 | 1.0000 | 1.0000 | 1.0000 |
| 7 | 1.0000 | 1.0000 | 1.0000 | 1.0000 | 1.0000 | 1.0000 | 1.0000 |
| 8 | 1.0000 | 1.0000 | 1.0000 | 1.0000 | 1.0000 | 1.0000 | 1.0000 |
| 9 | 0.5000 | 1.0000 | 0.5000 | 0.5000 | 0.6667 | 0.0000 | 0.0000 |
| Mean | 0.8500 | 0.9500 | 0.8167 | 0.8167 | 0.8133 | 0.6400 | 0.6500 |
| SD | 0.1893 | 0.1500 | 0.3202 | 0.3202 | 0.3023 | 0.4543 | 0.4500 |

FIG. 28

| | ACCURACY | AUC | RECALL | PREC. | F1 | KAPPA | MCC |
|---|---|---|---|---|---|---|---|
| 0 | 0.3333 | 0.2500 | 0.5000 | 0.5000 | 0.5000 | -0.5000 | -0.5000 |
| 1 | 0.0000 | 0.0000 | 0.0000 | 0.0000 | 0.0000 | -0.8000 | -1.0000 |
| 2 | 0.6667 | 0.7500 | 0.5000 | 1.0000 | 0.6667 | 0.4000 | 0.5000 |
| 3 | 0.6667 | 0.5000 | 0.0000 | 0.0000 | 0.0000 | 0.000 | 0.0000 |
| 4 | 0.3333 | 0.2500 | 0.0000 | 0.0000 | 0.0000 | -0.5000 | -0.5000 |
| 5 | 1.0000 | 1.0000 | 1.0000 | 1.0000 | 1.0000 | 1.0000 | 1.0000 |
| 6 | 1.0000 | 1.0000 | 1.0000 | 1.0000 | 1.0000 | 1.0000 | 1.0000 |
| 7 | 0.5000 | 1.0000 | 0.0000 | 0.0000 | 0.0000 | 0.0000 | 0.0000 |
| 8 | 0.5000 | 1.0000 | 0.0000 | 0.0000 | 0.0000 | 0.0000 | 0.0000 |
| 9 | 0.0000 | 0.0000 | 0.0000 | 0.0000 | 0.0000 | -1.0000 | -1.0000 |
| Mean | 0.5000 | 0.5750 | 0.3000 | 0.3500 | 0.3167 | -0.0400 | -0.0500 |
| SD | 0.3333 | 0.4039 | 0.4000 | 0.4500 | 0.4113 | 0.6545 | 0.6874 |

FIG. 29

| | ACCURACY | AUC | RECALL | PREC. | F1 | KAPPA | MCC |
|---|---|---|---|---|---|---|---|
| 0 | 0.6667 | 0.0000 | 1.0000 | 0.6667 | 0.8000 | 0.0000 | 0.0000 |
| 1 | 1.0000 | 1.0000 | 1.0000 | 1.0000 | 1.0000 | 1.0000 | 1.0000 |
| 2 | 0.6667 | 1.0000 | 0.5000 | 1.0000 | 0.6667 | 0.4000 | 0.5000 |
| 3 | 1.0000 | 1.0000 | 1.0000 | 1.0000 | 1.0000 | 1.0000 | 1.0000 |
| 4 | 0.6667 | 1.0000 | 0.0000 | 0.0000 | 0.0000 | 0.0000 | 0.0000 |
| 5 | 1.0000 | 1.0000 | 1.0000 | 1.0000 | 1.0000 | 1.0000 | 1.0000 |
| 6 | 1.0000 | 1.0000 | 1.0000 | 1.0000 | 1.0000 | 1.0000 | 1.0000 |
| 7 | 1.0000 | 1.0000 | 1.0000 | 1.0000 | 1.0000 | 1.0000 | 1.0000 |
| 8 | 0.5000 | 1.0000 | 1.0000 | 0.5000 | 0.6667 | 0.0000 | 0.0000 |
| 9 | 0.5000 | 1.0000 | 1.0000 | 0.5000 | 0.6667 | 0.0000 | 0.0000 |
| Mean | 0.8000 | 0.9000 | 0.8500 | 0.7667 | 0.7800 | 0.5400 | 0.5500 |
| SD | 0.2082 | 0.3000 | 0.3202 | 0.3266 | 0.2982 | 0.4737 | 0.4717 |

# FIG. 30

| | MODEL | ACCURACY | AUC | RECALL | PREC. | F1 | KAPPA | MCC |
|---|---|---|---|---|---|---|---|---|
| 0 | EXTRA TREES CLASSIFIER | 1.0000 | 1.0000 | 1.0000 | 1.0000 | 1.0000 | 1.0000 | 1.0000 |

EXTRA TREES CLASSIFIER CONFUSION MATRIX

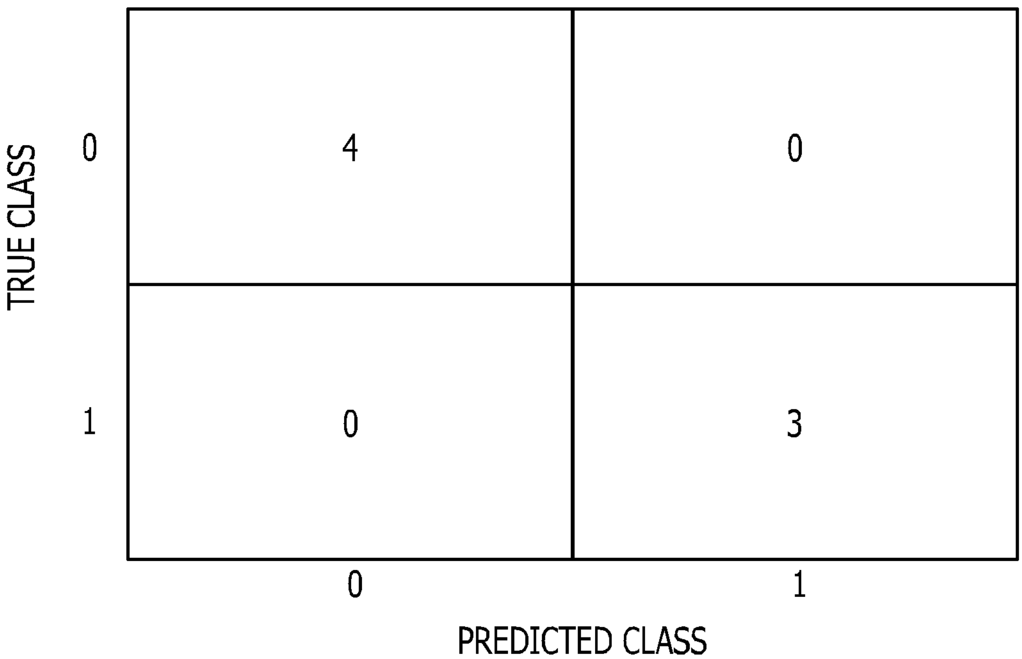

# FIG. 31

| | MODEL | ACCURACY | AUC | RECALL | PREC. | F1 | KAPPA | MCC |
|---|---|---|---|---|---|---|---|---|
| 0 | EXTRA TREES CLASSIFIER | 0.5714 | 0.7083 | 0.6667 | 0.5000 | 0.5714 | 0.1600 | 0.1667 |

EXTRA TREES CLASSIFIER CONFUSION MATRIX

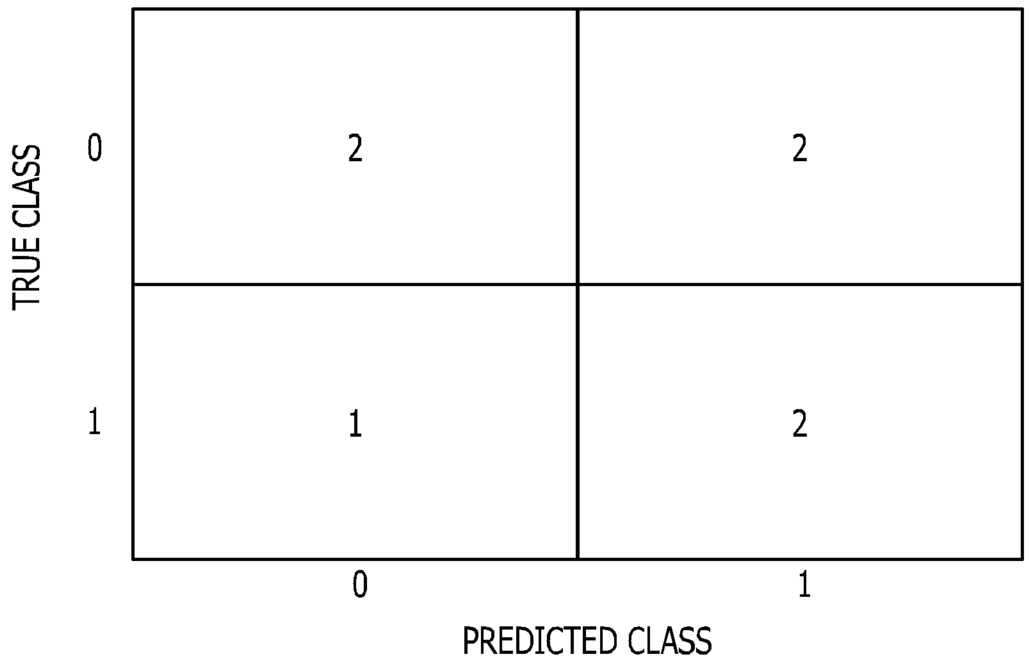

# FIG. 32

| PUBCHEM CID | $S_{new}$ | BIOLOGICAL ACTIVITY |
|---|---|---|
| 6035979 | 1 | ACTIVE |
| 856371 | 0.76258 | ACTIVE |
| 5952637 | 0.761892 | ACTIVE |
| 9620295 | 0.710231 | ACTIVE |
| 3944789 | 0.701941 | ACTIVE |
| 754762 | 0.665942 | ACTIVE |
| 658982 | 0.654074 | ACTIVE |
| 667386 | 0.631245 | ACTIVE |
| 917207 | 0.605423 | INACTIVE |
| 2459796 | 0.594177 | ACTIVE |
| 2614038 | 0.586167 | ACTIVE |

ACTIVE : 9/10

FIG. 33

| PUBCHEM CID | $S_{DA}$ | BIOLOGICAL ACTIVITY |
|---|---|---|
| 6035979 | 1 | ACTIVE |
| 856371 | 0.722222 | ACTIVE |
| 917207 | 0.708333 | INACTIVE |
| 754762 | 0.707071 | ACTIVE |
| 5952637 | 0.707071 | ACTIVE |
| 9620295 | 0703216 | ACTIVE |
| 4202261 | 0.684524 | INACTIVE |
| 3944789 | 0670635 | ACTIVE |
| 135512439 | 0.65812 | INACTIVE |
| 135401675 | 0.656566 | INACTIVE |
| 1213138 | 0.643939 | INACTIVE |

ACTIVE : 5/10

# FIG. 34

| PUBCHEM CID | BIOLOGICAL ACTIVITY | $1\text{-}E_{ave}$ |
|---|---|---|
| 6035979 | ACTIVE | 1 |
| 5952637 | ACTIVE | 0.816713 |
| 856371 | ACTIVE | 0.802938 |
| 658982 | ACTIVE | 0.780369 |
| 667386 | ACTIVE | 0.767321 |
| 3944789 | ACTIVE | 0.733247 |
| 9620295 | ACTIVE | 0.717246 |
| 2459796 | ACTIVE | 0.68637 |
| 2614038 | ACTIVE | 0.677164 |
| 853791 | ACTIVE | 0.674697 |
| 754762 | ACTIVE | 0.624813 |

ACTIVE : 10/10

FIG. 35

| | ACCURACY | AUC | RECALL | PREC. | F1 | KAPPA | MCC |
|---|---|---|---|---|---|---|---|
| 0 | 0.6667 | 0.5000 | 1.0000 | 0.6667 | 0.8000 | 0.0000 | 0.0000 |
| 1 | 1.0000 | 1.0000 | 1.0000 | 1.0000 | 1.0000 | 1.0000 | 1.0000 |
| 2 | 1.0000 | 1.0000 | 1.0000 | 1.0000 | 1.0000 | 1.0000 | 1.0000 |
| 3 | 1.0000 | 1.0000 | 1.0000 | 1.0000 | 1.0000 | 1.0000 | 1.0000 |
| 4 | 0.6667 | 1.0000 | 1.0000 | 0.5000 | 0.6667 | 0.4000 | 0.5000 |
| 5 | 1.0000 | 1.0000 | 1.0000 | 1.0000 | 1.0000 | 1.0000 | 1.0000 |
| 6 | 1.0000 | 1.0000 | 1.0000 | 1.0000 | 1.0000 | 1.0000 | 1.0000 |
| 7 | 1.0000 | 1.0000 | 1.0000 | 1.0000 | 1.0000 | 1.0000 | 1.0000 |
| 8 | 1.0000 | 1.0000 | 1.0000 | 1.0000 | 1.0000 | 1.0000 | 1.0000 |
| 9 | 0.0000 | 0.0000 | 0.0000 | 0.0000 | 0.0000 | -1.0000 | -1.0000 |
| Mean | 0.8333 | 0.8500 | 0.9000 | 0.8167 | 0.8467 | 0.6400 | 0.6500 |
| SD | 0.3073 | 0.3202 | 0.3000 | 0.3202 | 0.3027 | 0.6375 | 0.6344 |

FIG. 36

| | ACCURACY | AUC | RECALL | PREC. | F1 | KAPPA | MCC |
|---|---|---|---|---|---|---|---|
| 0 | 0.6667 | 0.0000 | 1.0000 | 0.6667 | 0.8000 | 0.0000 | 0.0000 |
| 1 | 1.0000 | 1.0000 | 1.0000 | 1.0000 | 1.0000 | 1.0000 | 1.0000 |
| 2 | 1.0000 | 1.0000 | 1.0000 | 1.0000 | 1.0000 | 1.0000 | 1.0000 |
| 3 | 1.0000 | 1.0000 | 1.0000 | 1.0000 | 1.0000 | 1.0000 | 1.0000 |
| 4 | 0.6667 | 1.0000 | 0.0000 | 0.0000 | 0.0000 | 0.0000 | 0.0000 |
| 5 | 1.0000 | 1.0000 | 1.0000 | 1.0000 | 1.0000 | 1.0000 | 1.0000 |
| 6 | 1.0000 | 1.0000 | 1.0000 | 1.0000 | 1.0000 | 1.0000 | 1.0000 |
| 7 | 1.0000 | 1.0000 | 1.0000 | 1.0000 | 1.0000 | 1.0000 | 1.0000 |
| 8 | 1.0000 | 1.0000 | 1.0000 | 1.0000 | 1.0000 | 1.0000 | 1.0000 |
| 9 | 0.5000 | 0.0000 | 0.0000 | 0.0000 | 0.0000 | 0.0000 | 0.0000 |
| Mean | 0.8333 | 0.8000 | 0.8000 | 0.7800 | 0.7800 | 0.7000 | 0.7000 |
| SD | 0.1833 | 0.4000 | 0.4000 | 0.3958 | 0.3945 | 0.4583 | 0.4583 |

# FIG. 37

| | MODEL | ACCURACY | AUC | RECALL | PREC. | F1 | KAPPA | MCC |
|---|---|---|---|---|---|---|---|---|
| 0 | EXTRA TREES CLASSIFIER | 1.0000 | 1.0000 | 1.0000 | 1.0000 | 1.0000 | 1.0000 | 1.0000 |

EXTRA TREES CLASSIFIER CONFUSION MATRIX

| TRUE CLASS \ PREDICTED CLASS | 0 | 1 |
|---|---|---|
| 0 | 4 | 0 |
| 1 | 0 | 3 |

FIG. 38

| | MODEL | MAE | MSE | RMSE | R2 | RMSLE | MAPE | TT (Sec) |
|---|---|---|---|---|---|---|---|---|
| catboost | CatBoost Regressor | 0.2474 | 0.1316 | 0.3308 | 0.4092 | 0.0753 | 0.0756 | 1.3090 |
| rf | Random Forest Regressor | 0.2616 | 0.1473 | 0.3562 | 0.3315 | 0.0806 | 0.0794 | 0.1050 |
| et | Extra Trees Regressor | 0.2621 | 0.1513 | 0.3597 | 0.2546 | 0.0806 | 0.0794 | 0.1050 |
| br | Bayesian Ridge | 0.2771 | 0.1216 | 0.3405 | 0.2191 | 0.0785 | 0.0854 | 0.0140 |
| ridge | Ridge Regression | 0.2668 | 0.1214 | 0.3357 | 0.1352 | 0.0775 | 0.0819 | 0.3320 |
| lr | Linear Regression | 0.2715 | 0.1180 | 0.3318 | 0.1178 | 0.0784 | 0.0847 | 0.6520 |
| ada | AdaBoost Regressor | 0.3018 | 0.1812 | 0.3998 | 0.0620 | 0.0901 | 0.0928 | 0.0190 |
| en | Elastic Net | 0.3412 | 0.2207 | 0.4407 | 0.0055 | 0.0988 | 0.1040 | 0.2940 |
| lasso | Lasso Regression | 03627 | 0.2643 | 0.4640 | 0.0022 | 0.1035 | 0.1093 | 0.3300 |
| gbr | Gradient Boosting Regressor | 0.3062 | 0.1799 | 0.4075 | -0.0673 | 0.0912 | 0.0940 | 0.0160 |
| knn | K Neighbors Regressor | 0.3844 | 0.2707 | 0.4757 | -0.1157 | 0.1068 | 0.1156 | 0.0230 |
| lightgbm | Light Gradient Boosting Machine | 0.3562 | 0.2564 | 0.4523 | -0.1783 | 0.1006 | 0.1059 | 0.0140 |
| llar | Lasso Least Angle Regression | 0.4476 | 0.4106 | 0.5694 | -03235 | 0.1284 | 0.1364 | 0.0100 |
| omp | Orthogonal Matching Putsuit | 0.3683 | 0.2232 | 0.4581 | -0.4315 | 0.1049 | 0.1150 | 0.0110 |
| dt | Decision Tree Regressor | 0.3468 | 0.2463 | 0.4752 | -0.5107 | 0.1094 | 0.1093 | 0.0110 |
| huber | Huber Rgressor | 0.3850 | 0.2462 | 0.4817 | -0.6855 | 0.1175 | 0.1211 | 0.0160 |
| par | Passive Aggressive Regressor | 0.7740 | 1.0187 | 0.9106 | -4.0382 | 0.2155 | 0.2451 | 0.0100 |
| lar | Least Angle Regression | 8.1473 | 528.3250 | 10.3567 | -5446.8280 | 0.5697 | 2.6479 | 0.0120 |

# FIG. 39

| | MAE | MSE | RMSE | R2 | RMSLE | MAPE |
|---|---|---|---|---|---|---|
| 0 | 0.1536 | 0.0352 | 0.1877 | 0.4929 | 0.0441 | 0.0459 |
| 1 | 0.4928 | 0.3638 | 0.6032 | 0.3936 | 0.1247 | 0.1272 |
| 2 | 0.2307 | 0.0904 | 0.3007 | 0.4024 | 0.0672 | 0.0668 |
| 3 | 0.3052 | 0.1293 | 0.3595 | 0.7209 | 0.0965 | 0.1193 |
| 4 | 0.1813 | 0.0694 | 0.2635 | -0.2084 | 0.0659 | 0.0628 |
| 5 | 0.1548 | 0.0472 | 0.2174 | 0.2690 | 0.0542 | 0.0536 |
| 6 | 0.0760 | 0.0065 | 0.0806 | 0.9314 | 0.0196 | 0.0248 |
| 7 | 0.2948 | 0.0971 | 0.3116 | 0.5011 | 0.0831 | 0.1092 |
| 8 | 0.2746 | 0.1402 | 0.3744 | 0.5630 | 0.0905 | 0.0829 |
| 9 | 0.4457 | 0.4713 | 0.6865 | 0.5777 | 0.1220 | 0.0999 |
| Mean | 0.2609 | 0.1450 | 0.3385 | 0.4644 | 0.0768 | 0.0792 |
| SD | 0.1247 | 0.1437 | 0.1745 | 0.2843 | 0.0316 | 0.0322 |

# FIG. 40

| | MAE | MSE | RMSE | R2 | RMSLE | MAPE |
|---|---|---|---|---|---|---|
| 0 | 0.1378 | 0.0286 | 0.1692 | 0.5879 | 0.0399 | 0.0428 |
| 1 | 0.4938 | 0.3810 | 0.6173 | 0.3650 | 0.1329 | 0.1346 |
| 2 | 0.3714 | 0.1863 | 0.4316 | -0.2309 | 0.1003 | 0.1093 |
| 3 | 0.5309 | 0.3947 | 0.6282 | 0.1480 | 0.1498 | 0.1829 |
| 4 | 0.1798 | 0.0490 | 0.2214 | 0.1464 | 0.0546 | 0.0600 |
| 5 | 0.3012 | 0.1560 | 0.3950 | -1.4136 | 0.0932 | 0.1012 |
| 6 | 0.1932 | 0.0519 | 0.2278 | 0.4518 | 0.0550 | 0.0627 |
| 7 | 0.3617 | 0.1770 | 0.4207 | 0.0901 | 0.1123 | 0.1401 |
| 8 | 0.3602 | 0.1671 | 0.4088 | 0.4792 | 0.0911 | 0.1036 |
| 9 | 0.5792 | 0.7046 | 0.8394 | 0.3687 | 0.1503 | 0.1318 |
| Mean | 0.3509 | 0.2296 | 0.4359 | 0.0993 | 0.0979 | 0.1069 |
| SD | 0.1441 | 0.1986 | 0.1989 | 0.5525 | 0.0374 | 0.0407 |

FIG. 41

| | MAE | MSE | RMSE | R2 | RMSLE | MAPE |
|---|---|---|---|---|---|---|
| 0 | 0.1450 | 0.0356 | 0.1886 | 0.4880 | 0.0444 | 0.0434 |
| 1 | 0.4505 | 0.3072 | 0.5543 | 0.4880 | 0.1135 | 0.1170 |
| 2 | 0.2467 | 0.0979 | 0.3130 | 0.3527 | 0.0695 | 0.0720 |
| 3 | 0.2685 | 0.1077 | 0.3282 | 0.7674 | 0.0888 | 0.1059 |
| 4 | 0.2033 | 0.0711 | 0.2667 | -0.2383 | 0.0668 | 0.0697 |
| 5 | 0.1823 | 0.0507 | 0.2253 | 0.2149 | 0.0560 | 0.0622 |
| 6 | 0.0890 | 0.0102 | 0.1009 | 0.8925 | 0.0238 | 0.0285 |
| 7 | 0.2564 | 0.0771 | 0.2777 | 0.6037 | 0.0749 | 0.0959 |
| 8 | 0.2522 | 0.1252 | 0.3539 | 0.5096 | 0.0848 | 0.0750 |
| 9 | 0.4514 | 0.4767 | 0.6904 | 0.5729 | 0.1215 | 0.1018 |
| Mean | 0.2545 | 0.1360 | 0.3299 | 0.4751 | 0.0744 | 0.0772 |
| SD | 0.1117 | 0.1373 | 0.1647 | 0.2993 | 0.0282 | 0.0268 |

# FIG. 42

| | MODEL | MAE | MSE | RMSE | R2 | RMSLE | MAPE |
|---|---|---|---|---|---|---|---|
| 0 | CatBoost Regressor | 0.2219 | 0.0730 | 0.2702 | 0.7165 | 0.0645 | 0.0734 |

Prediction Error for CatBoostRegressor

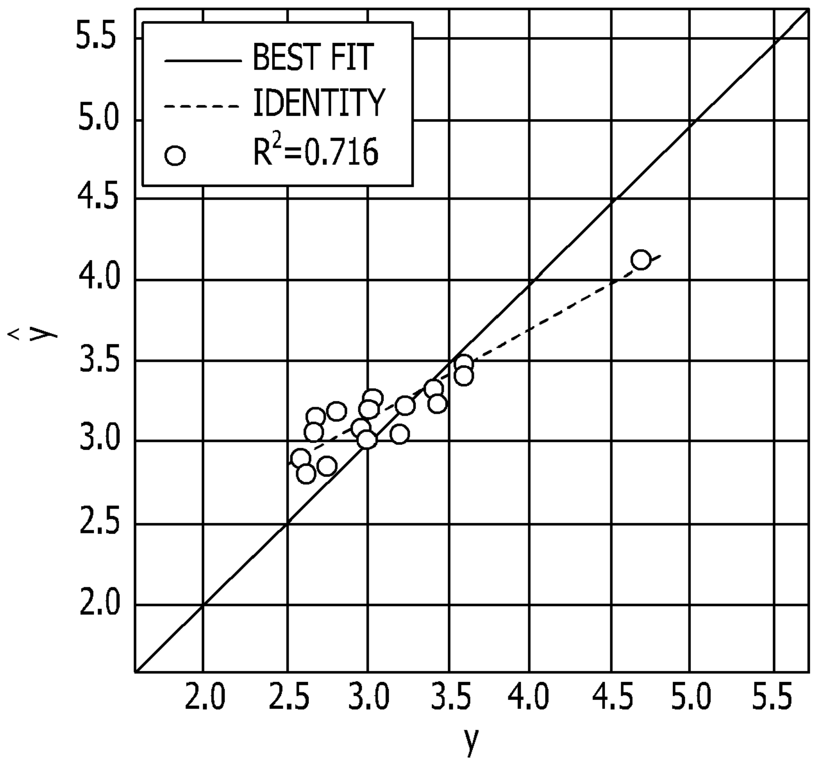

# FIG. 43

| | MODEL | MAE | MSE | RMSE | R2 | RMSLE | MAPE |
|---|---|---|---|---|---|---|---|
| 0 | CatBoost Regressor | 0.3709 | 0.2842 | 0.5331 | -0.1039 | 0.1170 | 0.1186 |

Prediction Error for CatBoostRegressor

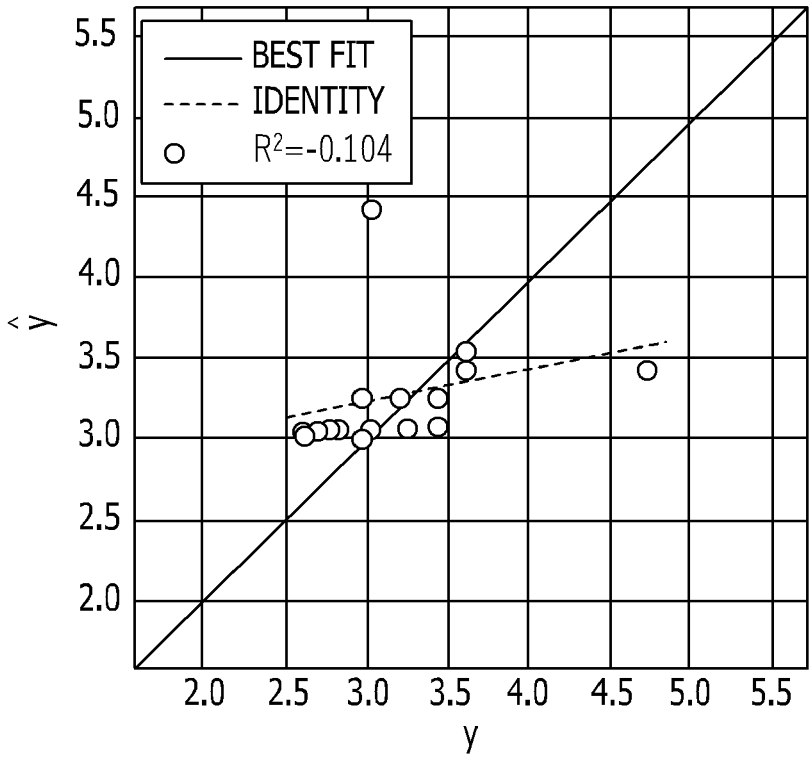

# FIG. 44

| | MODEL | MAE | MSE | RMSE | R2 | RMSLE | MAPE |
|---|---|---|---|---|---|---|---|
| 0 | CatBoost Regressor | 0.2132 | 0.0677 | 0.2603 | 0.7368 | 0.0620 | 0.0702 |

Prediction Error for CatBoostRegressor

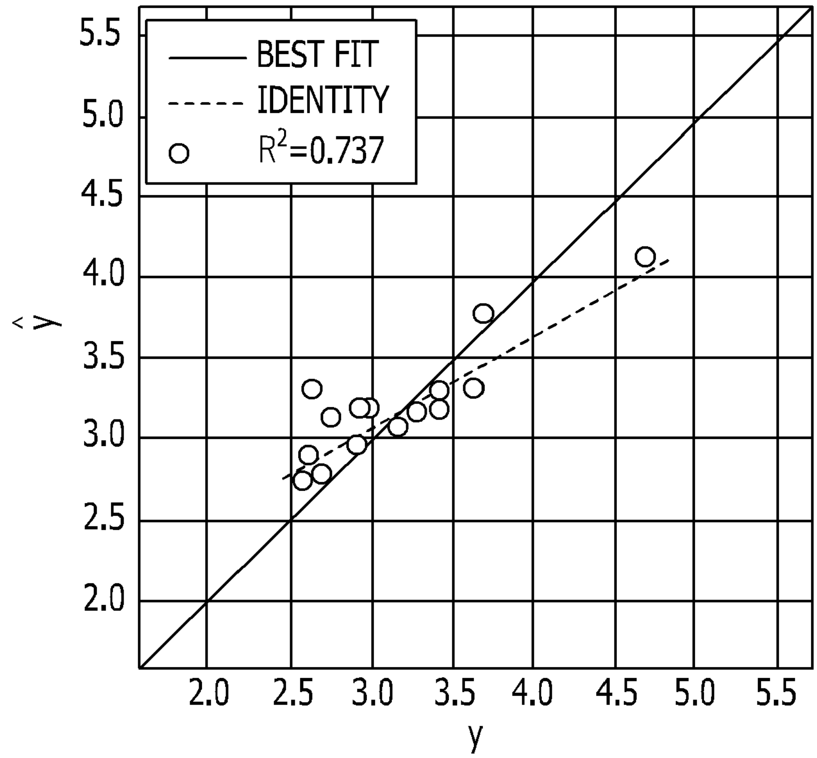

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020194488 A

### Non-patent literature cited in the description


- **MARITZA HERNANDEZ ; ARMAN ZARIBAFIYAN ; MALIHEH ARAMON ; MOHAMMAD NAGHIBI.** A Novel Graph-based Approach for Determining Molecular Similarity. *arXiv: 1601.06693, https://arxiv.org/pdf/1601.06693.pdf*
- **JUNMEI WANG ; ROMAIN M. WOLF ; JAMES W. CALDWELL ; PETER A. KOLLMAN ; DAVID A. CASE.** Development and Testing of a General Amber Force Field. *Journal of Computational Chemistry,* vol. 25 (9
- **KURSA MB ; RUDNICKI WR.** Feature Selection with the Boruta Package. *Journal of Statistical Software,* 2010, vol. 36 (11), 1-13, http://www.jstatsoft.org/v36/ill/.
- **TIBSHIRANI, R.** Regression shrinkage and selection via the lasso. *J. Roy. Statist. Soc. Ser. B,* 1996, vol. 58, 267-288
- **P. GEURTS ; D. ERNST. ; L. WEHENKEL.** Extremely randomized trees. *Machine Learning,* 2006, vol. 63 (1), 3-42